(19) Europäisches Patentamt
European Patent Office
Office européen des brevets

(11) EP 4 006 029 A1

(12) **EUROPEAN PATENT APPLICATION**
published in accordance with Art. 153(4) EPC

(43) Date of publication:
**01.06.2022 Bulletin 2022/22**

(21) Application number: **20844870.4**

(22) Date of filing: **23.07.2020**

(51) International Patent Classification (IPC):
***C07D 471/18*** *(2006.01)* ***C07D 487/18*** *(2006.01)*
***A61K 31/439*** *(2006.01)* ***A61P 9/10*** *(2006.01)*
***A61P 7/02*** *(2006.01)*

(52) Cooperative Patent Classification (CPC):
**A61K 31/439; A61P 7/02; A61P 9/10; C07D 471/18; C07D 487/18**

(86) International application number:
**PCT/CN2020/103692**

(87) International publication number:
**WO 2021/013209 (28.01.2021 Gazette 2021/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.07.2019 CN 201910668575**

(71) Applicant: **Medshine Discovery Inc.**
**Nanjing, Jiangsu 210032 (CN)**

(72) Inventors:
- **CAI, Yaxian**
  **Shanghai 200131 (CN)**
- **YAN, Xiaobing**
  **Shanghai 200131 (CN)**
- **CAI, Zhe**
  **Shanghai 200131 (CN)**
- **HU, Guoping**
  **Shanghai 200131 (CN)**
- **DING, Charles Z.**
  **Shanghai 200131 (CN)**
- **CHEN, Kevin X**
  **Shanghai 200131 (CN)**
- **LI, Jian**
  **Shanghai 200131 (CN)**
- **CHEN, Shuhui**
  **Shanghai 200131 (CN)**

(74) Representative: **Secerna LLP**
**The Old Fire Station**
**18 Clifford Street**
**York YO1 9RD (GB)**

(54) **MACROCYCLIC DERIVATIVES AS FACTOR XIA INHIBITORS**

(57) Macrocyclic derivatives, preparation methods therefor and pharmaceutical compositions comprising said derivatives, and uses thereof as therapeutic agents, especially as factor XIa inhibitors and in drugs for treating and preventing thromboembolisms and other diseases. Specifically disclosed is a compound represented by formula (I), and an isomer and pharmaceutically acceptable salt thereof.

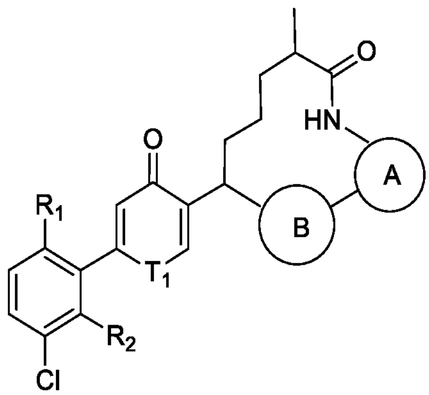

(I)

EP 4 006 029 A1

## Description

### This application claims the following priority:

Application number: CN201910668575.3, application date: July 23, 2019.

### Technical Field

The present disclosure relates to a novel class of macrocyclic derivatives, preparation method thereof and pharmaceutical composition comprising the derivatives, and uses thereof as therapeutic agents, especially as inhibitors of factor XIa and drugs for the treatment and prevention of diseases such as thromboembolism.

### Background

Antithrombotic drugs are mainly divided into antiplatelet drugs (such as clopidogrel, aspirin, ticagrelor, etc.), anticoagulant drugs (such as heparin, low molecular weight heparin, hirudin, warfarin, etc.) and thrombolytic drugs (such as urokinase, streptokinase, plasmin, etc.). In clinical applications, antiplatelet drugs and anticoagulant drugs are mainly used to prevent arterial and venous thrombosis, and thrombolytic drugs are used to dissolve thrombus. As the incidence of cardiovascular and cerebrovascular diseases in China continues to rise in recent years, the sales of antithrombotic drugs have also increased steadily, and the growth rate has been maintained between 15-20%. The sales in 2016 have been close to 20 billion yuan. With the aggravation of aging in the future, it is expected that the incidence of cardiovascular and cerebrovascular diseases will remain high and the market scale of antithrombotic drugs will continue to grow.

Anticoagulants can be widely used in the treatment and prevention of acute coronary syndrome, stroke, transient cerebral ischemia, deep vein embolism, pulmonary vein embolism, peripheral atherosclerosis obliterans and other arterial and venous thrombosis, and play an important role in various authoritative guidelines. In particular, the new oral anticoagulants that have been on the market in recent years have successively entered the authoritative guidelines, and they have replaced traditional anticoagulants such as warfarin and heparin as the first-choice drugs recommended by the guidelines with their good efficacy and safety in clinical trials.

Human coagulation process includes two processes: intrinsic pathway, extrinsic pathway and a common pathway. The extrinsic pathway means that under injury and various external stimuli, tissue factor and activated factor VII (FVIIa) combine to form a complex, and then factor X was reactivated by the complex (FX) to form activated FX (FXa). FXa then converts prothrombin into thrombin, which catalyzes the formation of fibrin from fibrinogen and plays a role in blood coagulation. The intrinsic pathway belongs to the body's inherent pathway, and all the factors involved in blood coagulation come from the blood. Factor XI (FXII) was activated through cascade reactions, factor XI (FXI) was activated by activated FXII (FXIIa), factor IX (FIX) was activated by activated FXI (FXIa), and FX was activated by activated FIX (FIXa). Thrombin is then produced through a common pathway, which in turn can activate FXI.

The risk of bleeding is a major problem of antithrombotic drugs. Therefore, coagulation factors that target intrinsic pathways but have no effect on extrinsic and common pathways are ideal antithrombotic drug targets. In view of the unique role of FXI/FXIa in the blood coagulation pathway and the process of coagulation, and the important feature of FXI gene defects that can prevent thrombosis without significantly increasing the risk of bleeding, FXI/FXIa has become an important target for the development of new anticoagulant drugs. FXI zymogen protein is a 160-kDa dimer with the same subunits connected by disulfide bonds, each subunit includes 4 "apple domains" and 1 C-terminal catalytic domain. After FXI is activated and becomes FXIa with enzymatic activity, the downstream zymogen protein FIX is cut by the catalytic domain to be activated.

Antithrombotic drugs targeting FXI/FXIa include antisense drugs, monoclonal antibodies, and small molecule inhibitors, some drugs have entered the clinical research phase. Among them, antisense drugs have made the fastest progress and have completed the key phase II clinical trial. A positive result was obtained, confirming the effectiveness and safety of antithrombotic drugs targeting FXI/FXIa in humans.

At present, many companies have patent reports of macrocyclic derivatives as FXIa inhibitors, such as BMS patents WO2011100401, WO2011100402, WO2013022814, WO2013022818, WO2014022766, WO2014022767, WO2015116882, WO2015116885, WO2015116886 and WO2016053455; Merck's patent WO2017074832 and WO2017074833; WO2018133793 of HEC Pharmaceutical Co., Ltd. The macrocyclic compounds reported in these patents generally have high activity, but due to their large molecular weight, the pharmacokinetic results in vivo are not ideal.

### Content of the present invention

The present disclosure provides a compound represented by formula (I), an isomer thereof or a pharmaceutically

acceptable salt thereof,

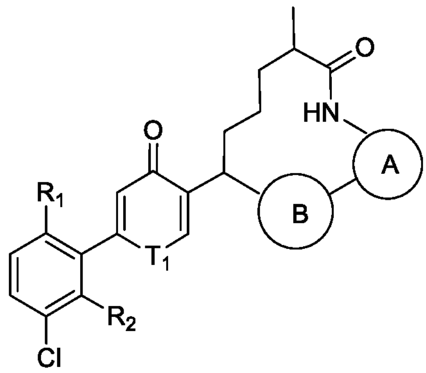

(I),

Wherein, $R_1$ is triazolyl or tetrazolyl, wherein the triazolyl and tetrazolyl are optionally substituted by Ra;

Ra is F, Cl, Br, I, CN, $C_{1-3}$ alkyl, $C_{1-3}$ alkoxy or $C_{3-4}$ cycloalkyl;

$R_2$ is H or F;

$T_1$ is -O- or -N(Rb)-;

$R_b$ is H, $-CH_3$, $-CH_2CH_3$, $-CH_2CH_2CH_3$ or $-CH(CH_3)_2$;

ring A is phenyl optionally substituted by 1, 2 or 3 $R_c$ or pyrazolyl optionally substituted by 1 or 2 $R_d$;

$R_c$ is H, F, Cl, Br, I, $C_{1-3}$ alkyl or $C_{1-3}$ alkoxy;

$R_d$ is H or $C_{1-3}$ alkyl, wherein the $C_{1-3}$ alkyl is optionally substituted by 1, 2 or 3 substituents independently selected from F, Cl, Br, I, D, $C_{1-3}$ alkoxy and $C_{3-4}$ cycloalkyl;

ring B is

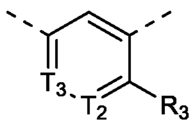

;

$T_2$ is N or $CR_4$;

$T_3$ is N or $CR_5$;

$R_3$ is H, F, Cl, Br, I, $C_{1-3}$ alkyl, $C_{1-3}$ haloalkyl or $C_{1-3}$ alkoxy;

$R_4$ and $R_5$ are each independently H, F, Cl, Br, I, $C_{1-3}$ alkyl, $C_{1-3}$ haloalkyl, or $C_{1-3}$ alkoxy.

The present disclosure also provides a compound represented by formula (I), an isomer thereof or a pharmaceutically acceptable salt thereof,

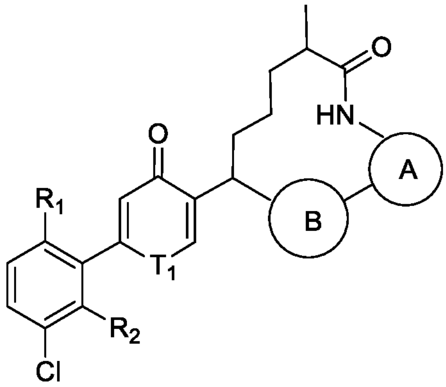

(I),

wherein, $R_1$ is triazolyl or tetrazolyl, wherein the triazolyl and tetrazolyl are optionally substituted by Ra;

Ra is F, Cl, Br, I, $C_{1-3}$ alkyl, $C_{1-3}$ alkoxy or $C_{3-4}$ cycloalkyl;

$R_2$ is H or F;

$T_1$ is -O- or -N(Rb)-;

$R_b$ is H, $-CH_3$, $-CH_2CH_3$, $-CH_2CH_2CH_3$ or $-CH(CH_3)_2$;

ring A is phenyl optionally substituted by 1, 2 or 3 $R_c$ or pyrazolyl optionally substituted by 1 or 2 $R_d$;

$R_c$ is H, F, Cl, Br, I, $C_{1-3}$ alkyl or $C_{1-3}$ alkoxy;

$R_d$ is H or $C_{1-3}$ alkyl, wherein the $C_{1-3}$ alkyl is optionally substituted by 1, 2 or 3 substituents independently selected from F, Cl, Br, I, $C_{1-3}$ alkoxy and $C_{3-4}$ cycloalkyl;

ring B is

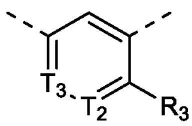

;

$T_2$ is N or $CR_4$;

$T_3$ is N or $CR_5$;

$R_3$ is H, F, Cl, Br, I, $C_{1-3}$ alkyl, $C_{1-3}$ haloalkyl or $C_{1-3}$ alkoxy;

$R_4$ and Rs are each independently H, F, Cl, Br, I, $C_{1-3}$ alkyl, $C_{1-3}$ haloalkyl, or $C_{1-3}$ alkoxy.

In some embodiments of the present disclosure, the $R_b$ is $-CH_3$, and other variables are as defined in the present disclosure.

In some embodiments of the present disclosure, the compound has the structure represented by formula (I-1):

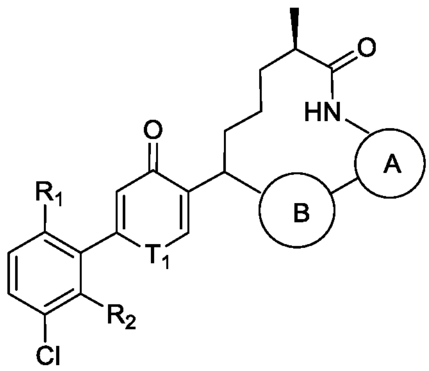

(I-1),

wherein, the carbon atom with "*" is a chiral carbon atom, existing in the form of (R) or (S) single enantiomer or enriched in one enantiomer; $R_1$, $R_2$, $T_1$, ring A and ring B are as defined in the present disclosure.

In some embodiments of the present disclosure, the ring B is

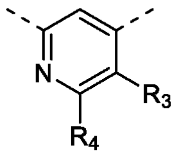

,

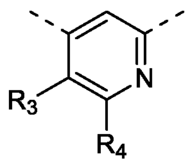

,

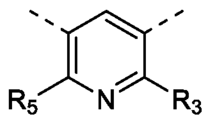

,

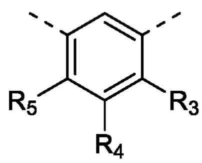

or

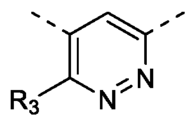

,

$R_3$, $R_4$ and $R_5$ and other variables are as defined in the present disclosure.

In some embodiments of the present disclosure, the B is

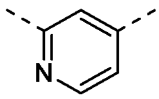

,

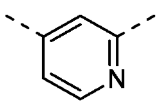

,

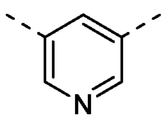

,

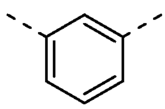

or

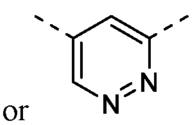

,

and other variables are as defined in the present disclosure.

In some embodiments of the present disclosure, the B is

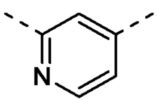

,

and other variables are as defined in the present disclosure.

In some embodiments of the present disclosure, the compound has the structure represented by formula (1-2), (1-3), (1-4) or (1-5) :

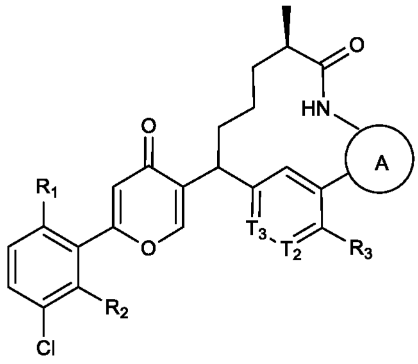
(I-2),
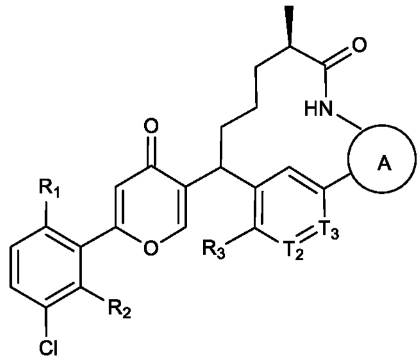
(I-3),

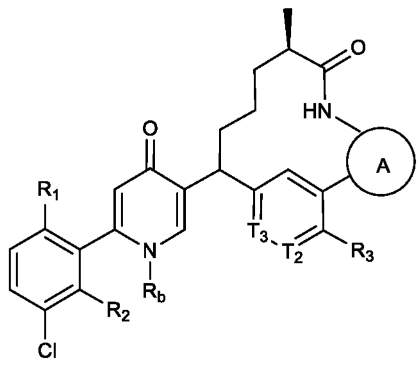
(I-4) or
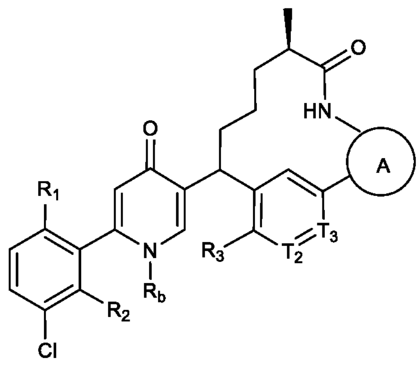
(I-5),

wherein, ring A, $R_1$, $R_2$, $R_3$, $T_2$, $T_3$ and $R_b$ are as defined in the present disclosure.

In some embodiments of the present disclosure, the compound has the structure represented by formula (1-6), (1-7), (1-8) or (1-9) :

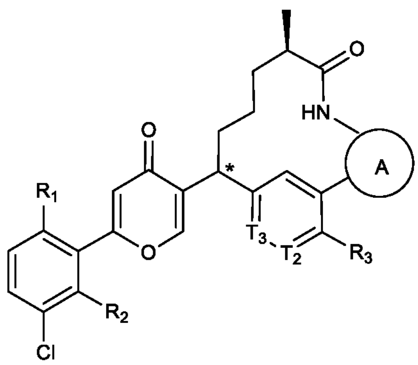
(I-6),
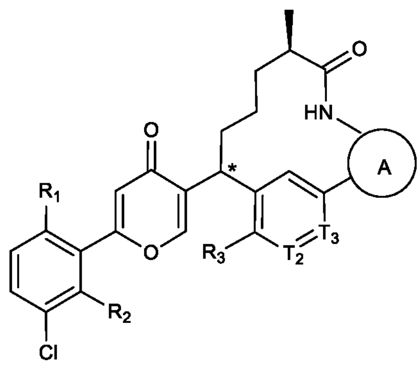
(I-7),

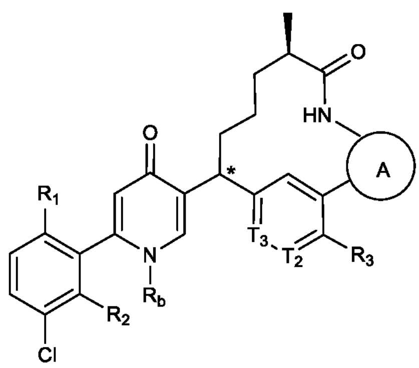
(I-8) or
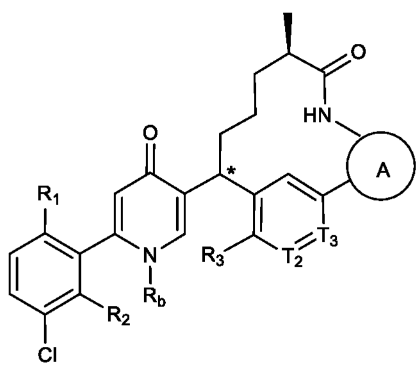
(I-9),

wherein, the carbon atom with "*" is a chiral carbon atom, existing in the form of (R) or (S) single enantiomer or enriched in one enantiomer; ring A, $R_1$, $R_2$, $R_3$, $R_4$ and $R_5$ are as defined in the present disclosure.

In some embodiments of the present disclosure, the compound has the structure represented by formula (1-10), (1-11), (1-12) or (1-13) :

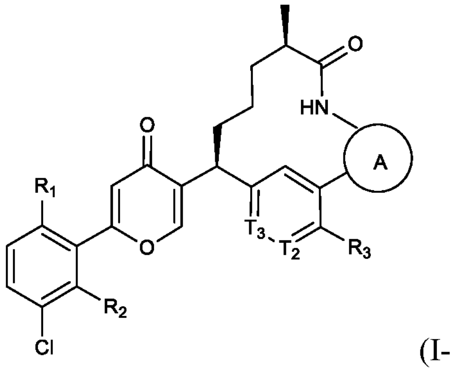

(I-10),

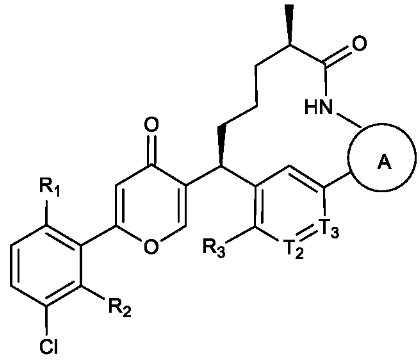

(I-11),

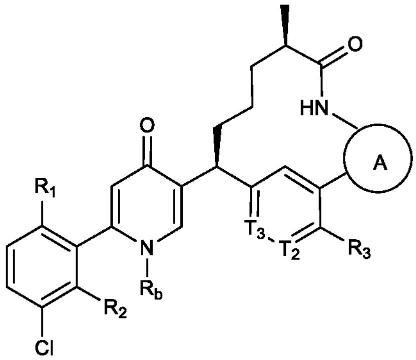

(I-12) or

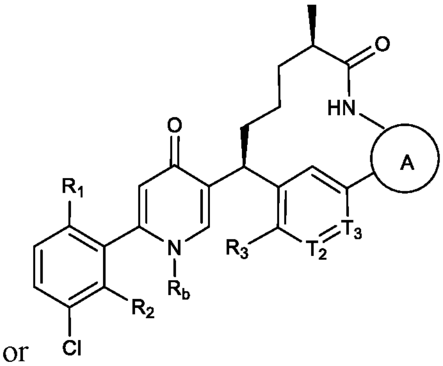

(I-13),

wherein, ring A, $R_1$, $R_2$, $R_3$, $T_2$ and $T_3$ are as defined in the present disclosure.

In some embodiments of the present disclosure, the $R_c$ is H, F, Cl or $-CH_3$, and other variables are as defined in the present disclosure.

In some embodiments of the present disclosure, the $R_d$ is

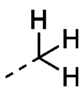,
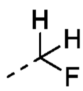,

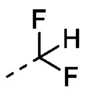 or
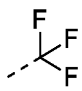,

and other variables are as defined in the present disclosure.

In some embodiments of the present disclosure, the $R_d$ is

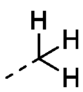,
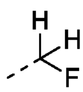,

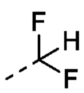,
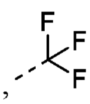 or
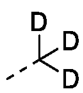,

and other variables are as defined in the present disclosure.

In some embodiments of the present disclosure, the $R_d$ is

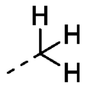, 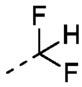

or

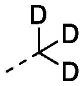,

and other variables are as defined in the present disclosure.

In some embodiments of the present disclosure, the ring A is

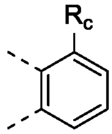,

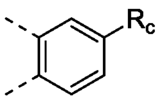, 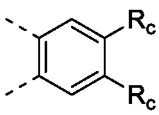 or 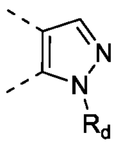,

$R_c$ and $R_d$ and other variables are as defined in the present disclosure.

In some embodiments of the present disclosure, the ring A is

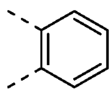,

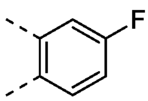, 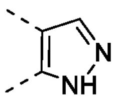 or 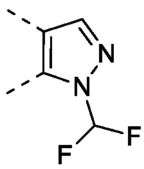,

and other variables are as defined in the present disclosure.

In some embodiments of the present disclosure, the ring A is

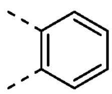,

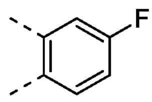, 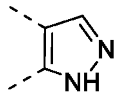, 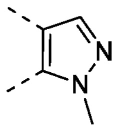, 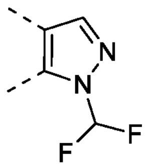 or 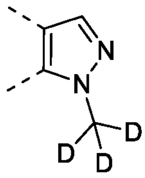,

and other variables are as defined in the present disclosure.

In some embodiments of the present disclosure, the ring A is

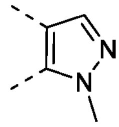,

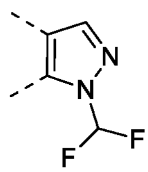 or 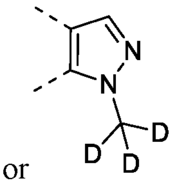,

and other variables are as defined in the present disclosure.

In some embodiments of the present disclosure, the compound has the structure represented by any one of formulas (I-14)-(I-21):

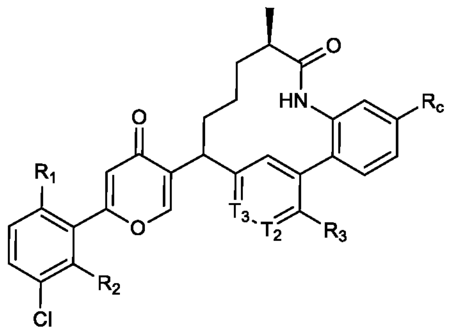

(I-14),

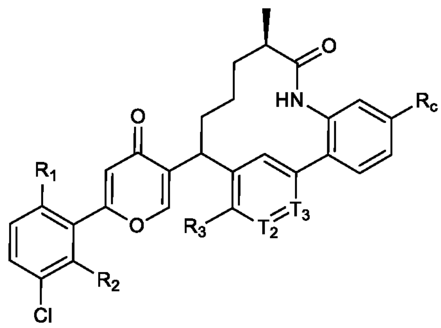

(I-15),

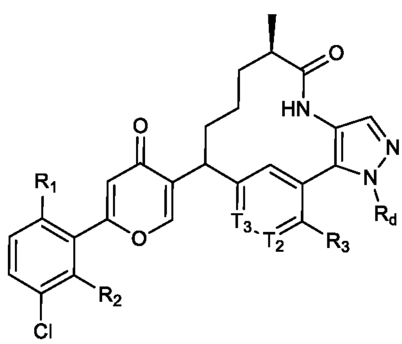

(I-16),

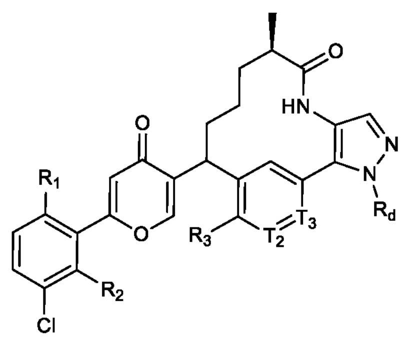

(I-17),

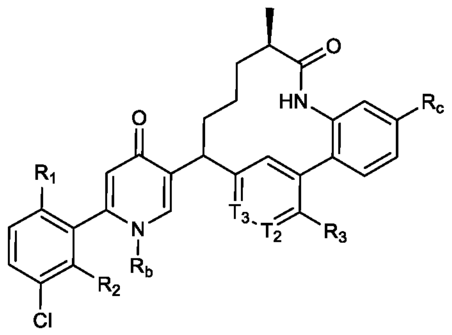

(I-18),

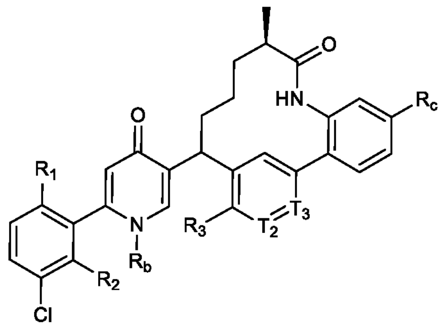

(I-19),

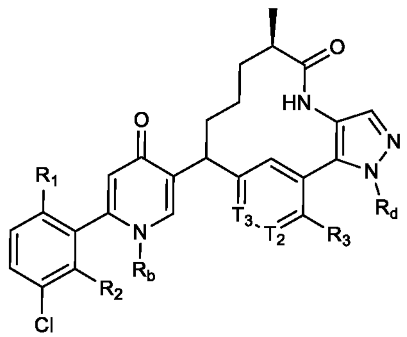

(I-20) or

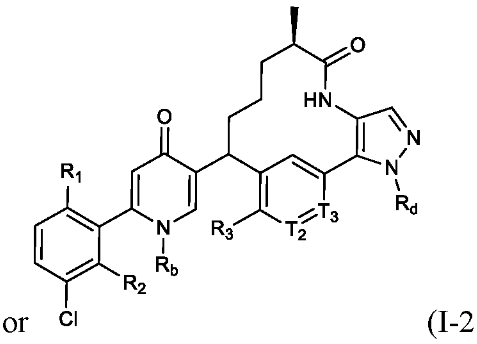

(I-21),

wherein, $R_1$, $R_2$, $R_3$, $T_2$, $T_3$, $R_b$, $R_c$ and $R_d$ are as defined in the present disclosure.

In some embodiments of the present disclosure, the compound has the structure represented by any one of formulas (I-22)-(I-29):

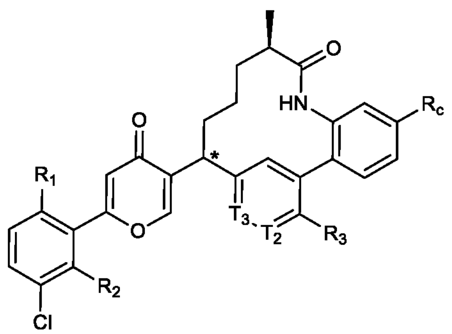

(I-22),

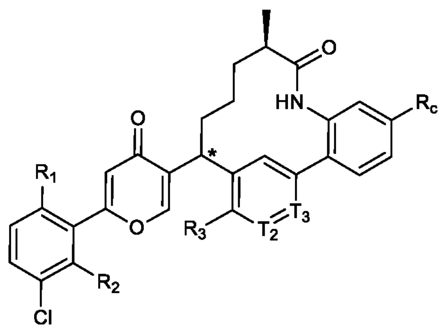

(I-23),

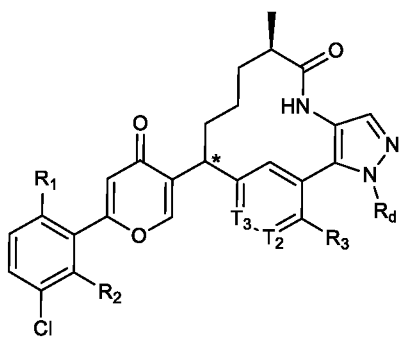

(I-24),

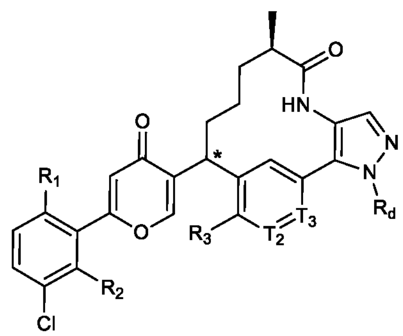

(I-25),

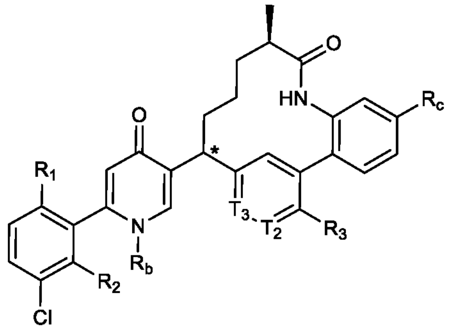 (I-26), 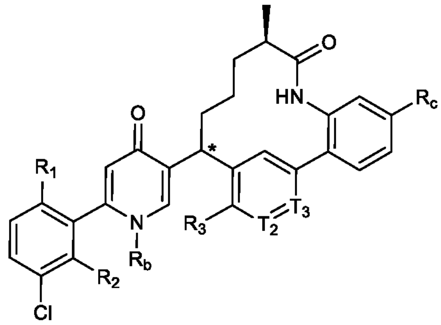 (I-27),

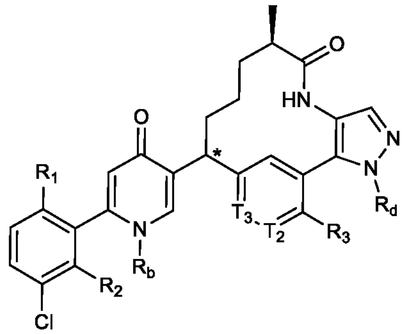 (I-28) or 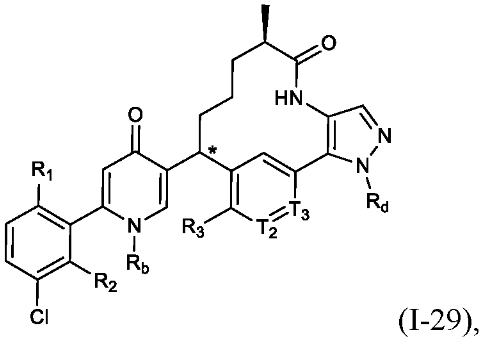 (I-29),

wherein, the carbon atom with "*" is a chiral carbon atom, existing in the form of (R) or (S) single enantiomer or enriched in one enantiomer; $R_1$, $R_2$, $R_3$, $T_2$, $T_3$, $R_b$, $R_c$ and $R_d$ are as defined in the present disclosure.

In some embodiments of the present disclosure, the compound has the structure represented by any one of formulas (I-30)-(I-37):

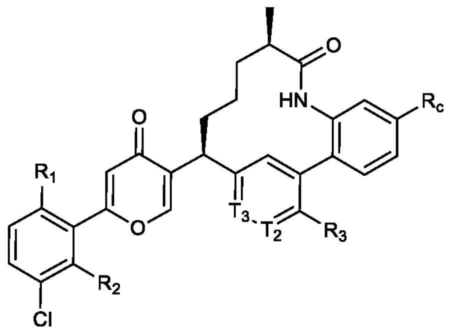 (I-30), 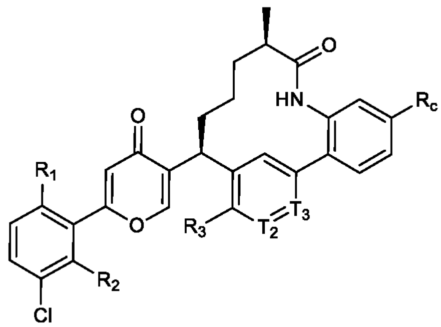 (I-31),

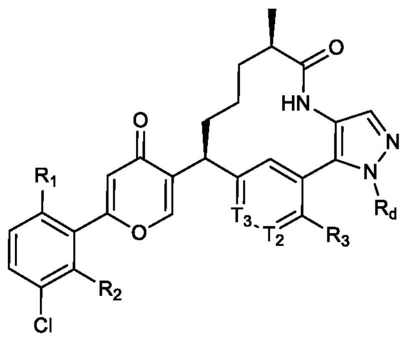 (I-32), 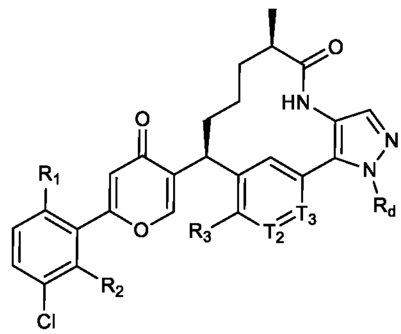 (I-33),

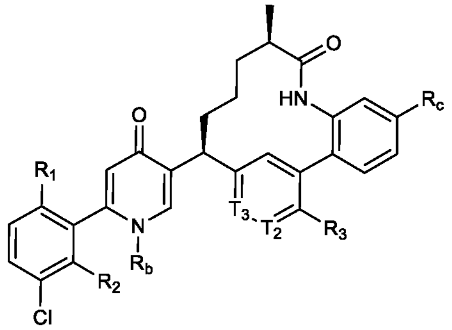

(I-34),

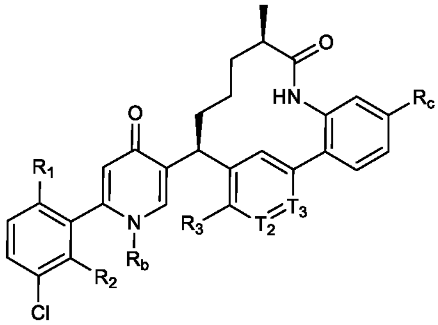

(I-35),

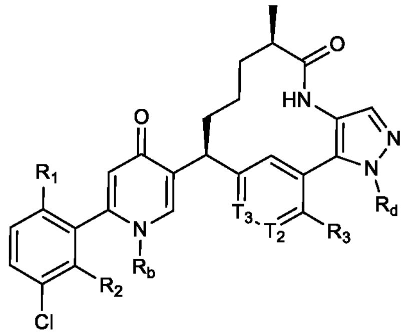

(I-36) or

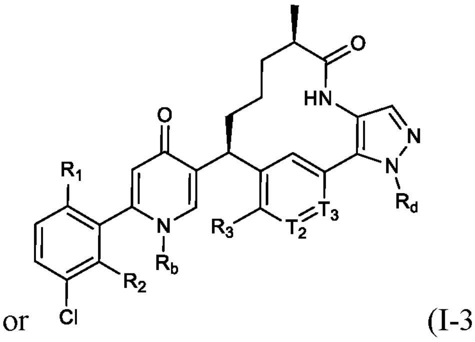

(I-37),

wherein, $R_1$, $R_2$, $R_3$, $T_2$, $T_3$, $R_b$, $R_c$ and $R_d$ are as defined in the present disclosure.

In some embodiments of the present disclosure, the compound has the structure represented by formula (1-38) or (1-39):

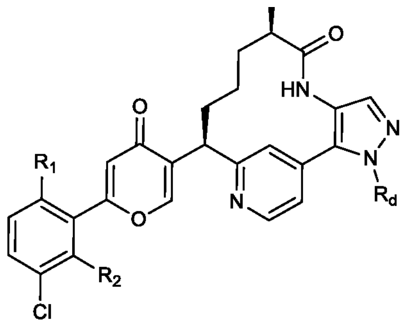

(I-38) or

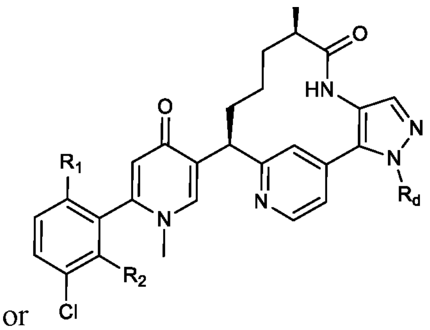

(I-39),

[0065] wherein, $R_1$, $R_2$ and $R_d$ are as defined in the present disclosure.

In some embodiments of the present disclosure, the Ra is F, Cl, $-CH_3$ or

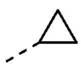

,

and other variables are as defined in the present disclosure.

In some embodiments of the present disclosure, the Ra is F, Cl, CN, $-CH_3$ or

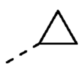

,

and other variables are as defined in the present disclosure.

In some embodiments of the present disclosure, the Ra is Cl or CN, and other variables are as defined in the present disclosure.

In some embodiments of the present disclosure, the $R_1$ is

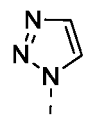 , 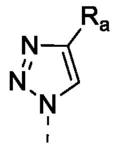 ,

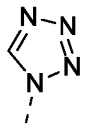 or 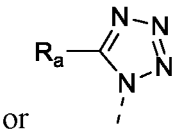 ,

Ra and other variables are as defined in the present disclosure.

In some embodiments of the present disclosure, the $R_1$ is

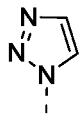 , 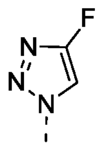 ,

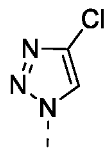 or 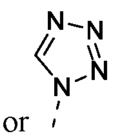 ,

and other variables are as defined in the present disclosure.

In some embodiments of the present disclosure, the $R_1$ is

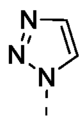 , 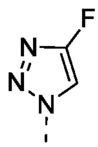 ,

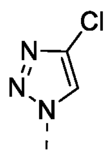 , 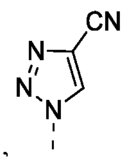 or 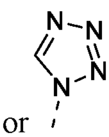 ,

and other variables are as defined in the present disclosure.

In some embodiments of the present disclosure, the $R_1$ is

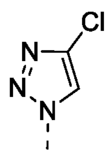 , 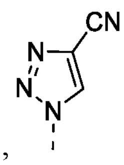

or

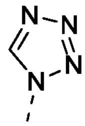,

and other variables are as defined in the present disclosure.

In some embodiments of the present disclosure, the $R_3$ is H, F, Cl, Br,

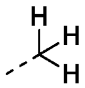,

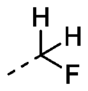, 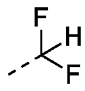, 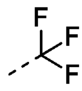 or 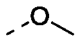,

and other variables are as defined in the present disclosure.

In some embodiments of the present disclosure, the $R_3$ is H, and other variables are as defined in the present disclosure.

In some embodiments of the present disclosure, the $R_4$ and $R_5$ are each independently H, F, Cl, Br,

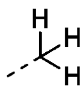, 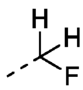, 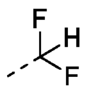, 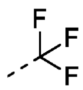 or 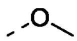,

and other variables are as defined in the present disclosure.

In some embodiments of the present disclosure, the $T_2$ is N, CH or CF, and other variables are as defined in the present disclosure.

In some embodiments of the present disclosure, the $T_2$ is CH, and other variables are as defined in the present disclosure.

In some embodiments of the present disclosure, the $T_3$ is N, CH or CF, and other variables are as defined in the present disclosure.

In some embodiments of the present disclosure, the $T_3$ is N, and other variables are as defined in the present disclosure.

There are also some embodiments of the present disclosure that come from any combination of the above variables.

In some embodiments of the present disclosure, the compound is a compound of the following formula, an isomer thereof, or a pharmaceutically acceptable salt thereof,

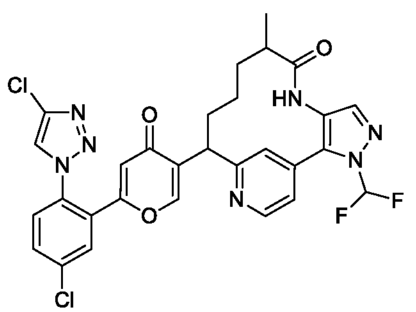,

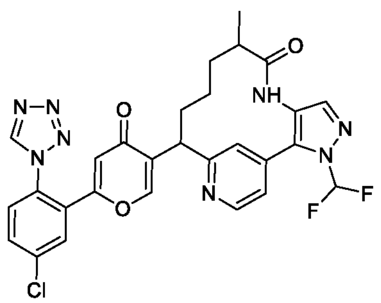,

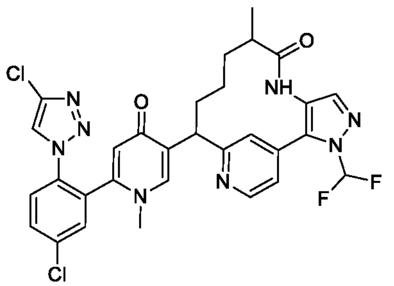
,
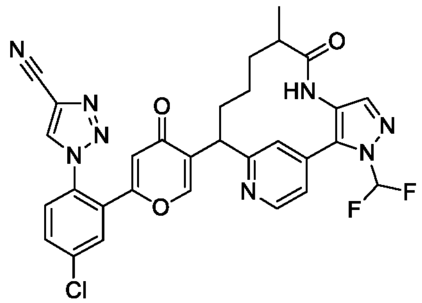
,

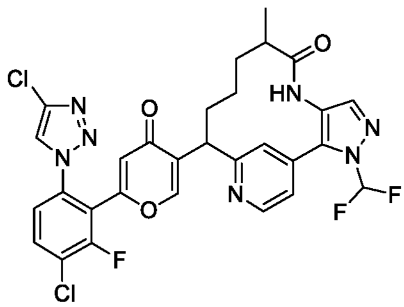
,
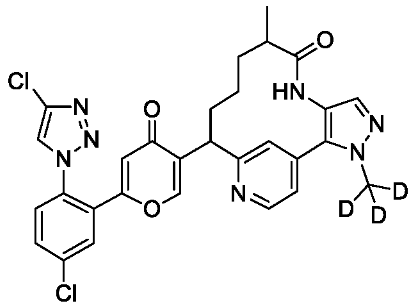
,

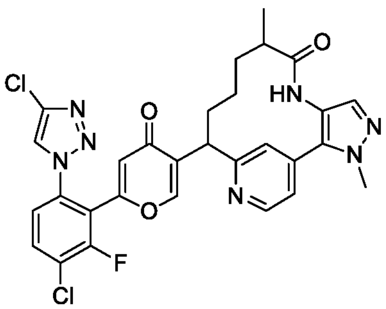
or
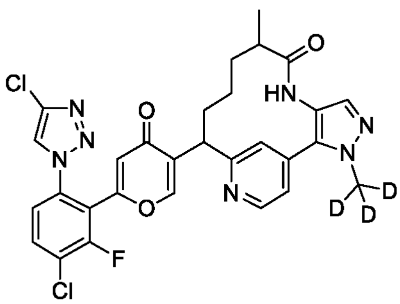
.

In some embodiments of the present disclosure, the compound is a compound of the following formula, an isomer thereof, or a pharmaceutically acceptable salt thereof,

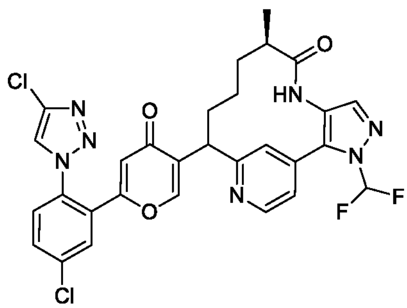
,
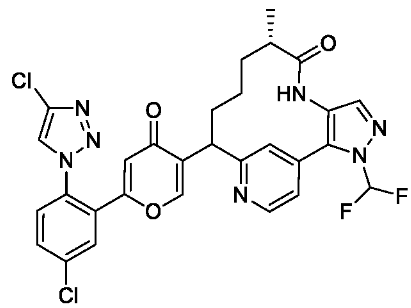
,

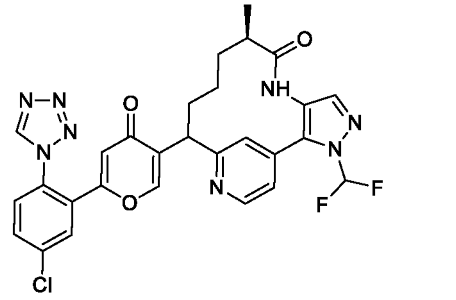,

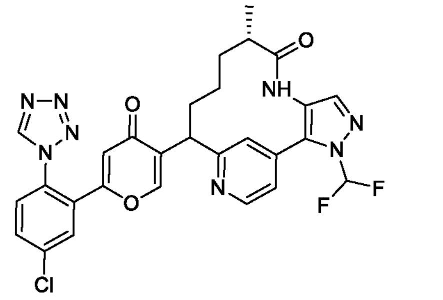,

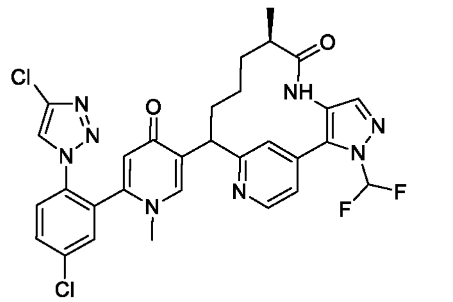,

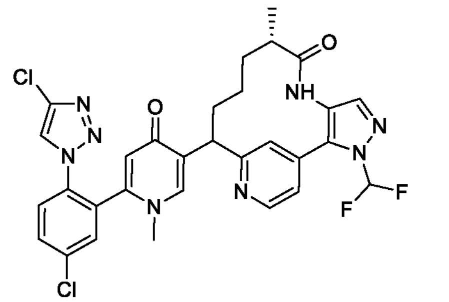,

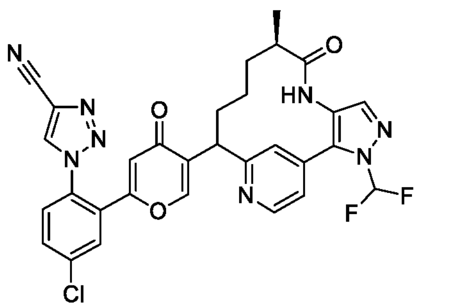,

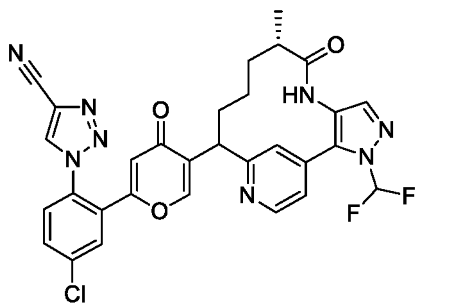,

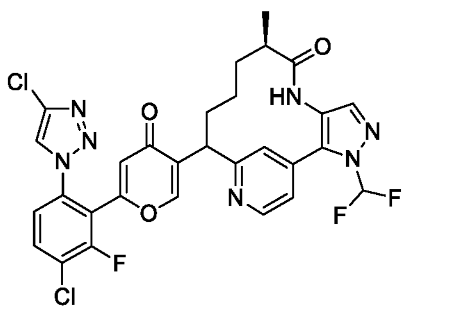,

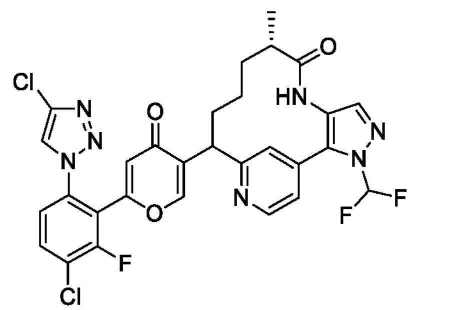,

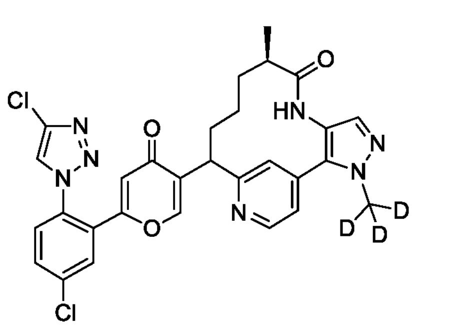,

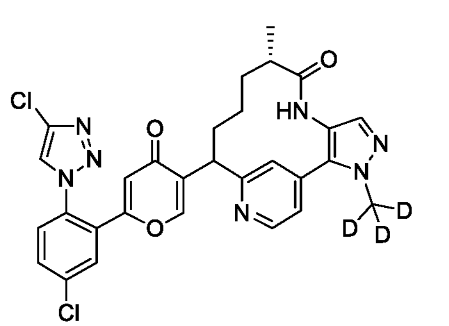,

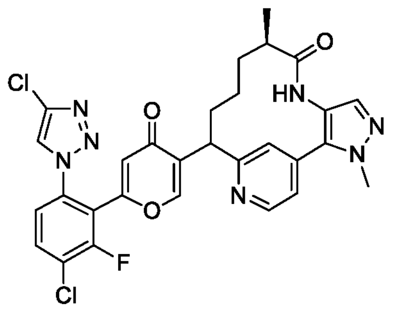

,

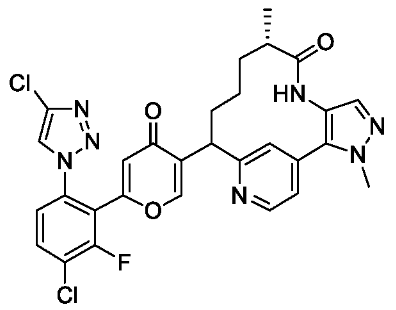

,

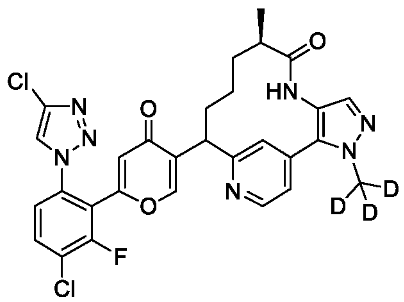

or

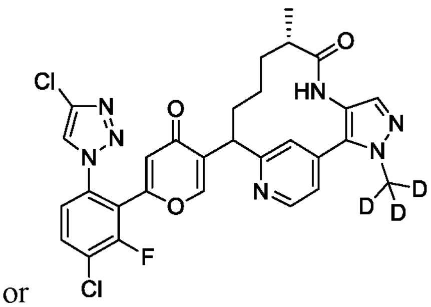

.

In some embodiments of the present disclosure, the compound is a compound of the following formula, an isomer thereof, or a pharmaceutically acceptable salt thereof,

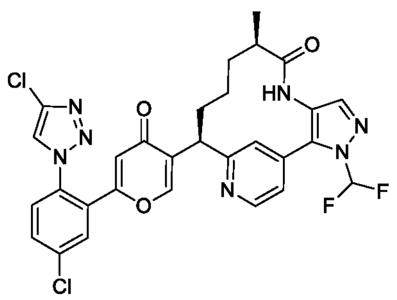

,

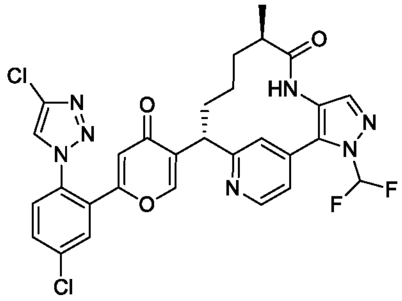

,

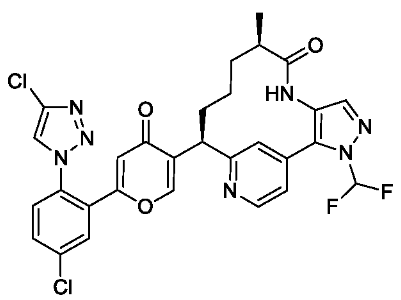

,

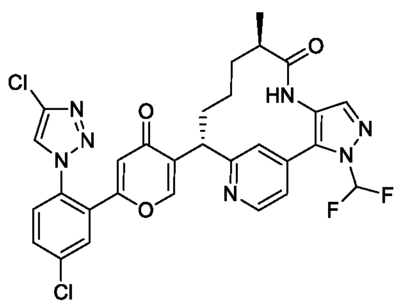

,

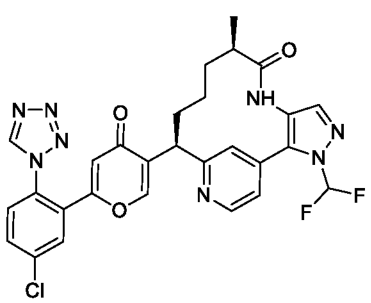

,

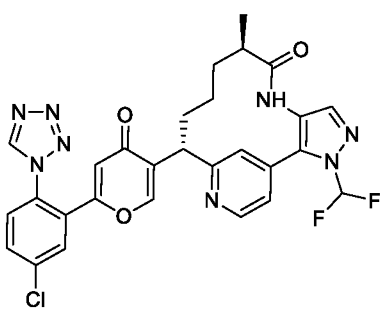

,

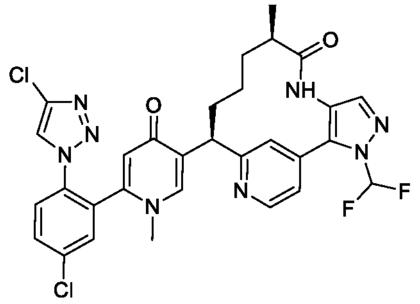
,
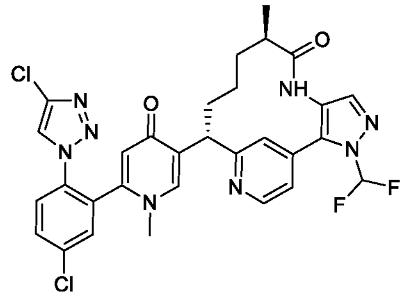
,
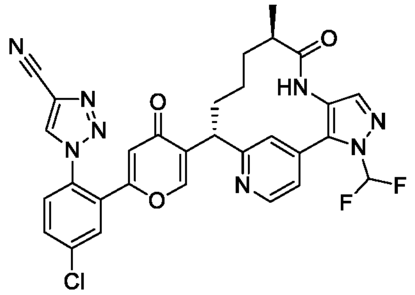
,
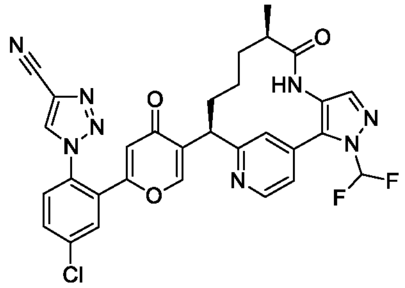
,
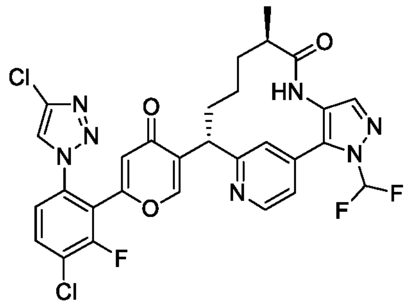
,
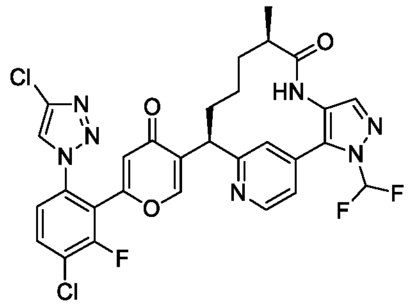
,
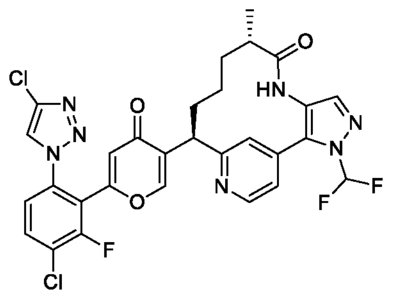
,
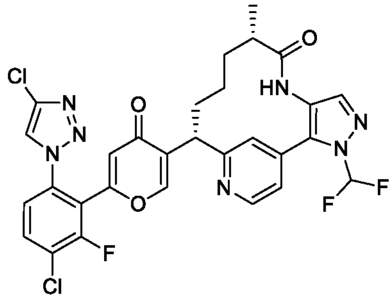
,
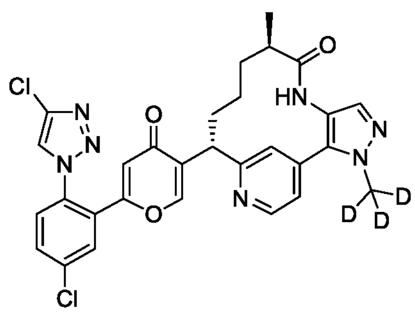
,
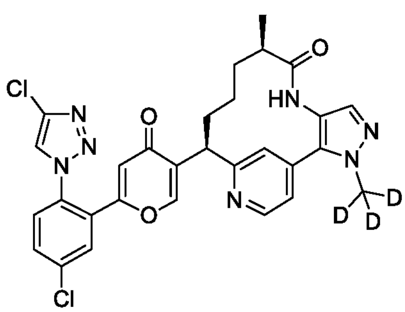
,

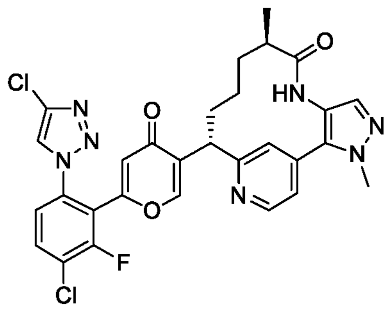

,

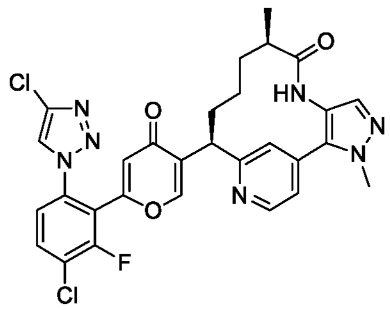

,

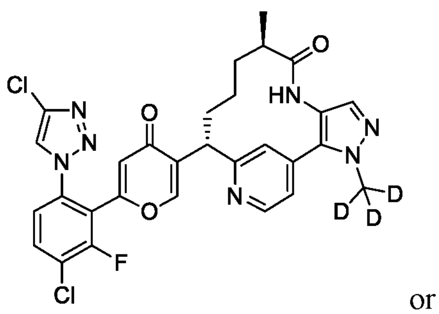

or

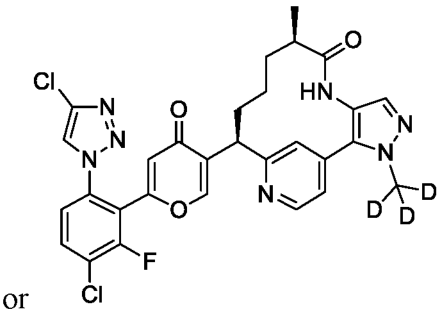

.

In another aspect, the present disclosure also provides a pharmaceutical composition comprising a therapeutically effective amount of the compound, an isomer thereof or a pharmaceutically acceptable salt thereof, and a pharmaceutically acceptable carrier.

The present disclosure also provides a use of the compound, an isomer thereof or a pharmaceutically acceptable salt and the pharmaceutical composition in the manufacture of a medicament of factor XIa inhibitor.

## Technical effect

The purpose of the present disclosure is to provide a macrocyclic compound suitable for FXIa enzyme inhibitors, a derivative thereof, and a pharmaceutical composition containing the macrocyclic compound, which can effectively treat and prevent thromboembolic diseases. The compound not only has high FXIa enzyme activity and anticoagulant effect of human blood in vitro, but also has good in vivo pharmacokinetic properties.

## Definition and description

Unless otherwise specified, the following terms and phrases when used herein have the following meanings. A specific term or phrase should not be considered indefinite or unclear in the absence of a particular definition, but should be understood in the ordinary sense. When a trade name appears herein, it is intended to refer to its corresponding commodity or active ingredient thereof.

With regard to drugs or pharmacologically active agents, the term "effective amount" or "therapeutically effective amount" refers to a sufficient amount of a drug or agent that is non-toxic but can achieve the desired effect. For the oral dosage form of the present disclosure, the "effective amount" of one active substance in the composition refers to the amount required to achieve the desired effect when combined with another active substance in the composition. The determination of the effective amount varies from person to person, and depends on the age and general conditions of the recipient, as well as the specific active substance. The appropriate effective amount in each case can be determined by those skilled in the art according to routine experiments.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine or magnesium, or similar salts. When the compound of the present disclosure contains a relatively basic functional group, an acid addition salt can be obtained by bringing the neutral form of the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the like; and salts of amino acid (such as arginine and the like), and a salt of an organic acid such as glucuronic acid and the like. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including cis and trans isomers, (-)-and (+)-enantiomers, (*R*)-and (*S*)-enantiomers, diastereomers isomers, (D)-isomers, (L)-isomers, and racemic and other mixtures thereof, such as enantiomers or diastereomeric enriched mixtures, all of which are within the scope of the present disclosure. Additional asymmetric carbon atoms may be present in substituents such as alkyl. All these isomers and their mixtures are included within the scope of the present disclosure.

Unless otherwise specified, the term "enantiomer" or "optical isomer" refers to stereoisomers that are mirror images of each other.

Unless otherwise specified, the term *"cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and or "(±)" refers to racemic.

Unless otherwise specified, the absolute configuration of a stereogenic center is represented by a wedged solid bond ( ) and a wedged dashed bond ( ), and the relative configuration of a stereogenic center is represented by a straight solid bond ( ) and a straight dashed bond ( ), a wave line ( ) is used to represent a wedged dashed bond ( ) or a wedged dashed bond ( ), or the wave line ( ) is used to represent a straight solid bond ( ) and a straight dashed bond ( ).

Unless otherwise specified, the terms "enriched in one isomer", "enriched in isomers", "enriched in one enantiomer" or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (R)- and (*S*)-isomer, *or D* and *L* isomer can be prepared using chiral synthesis or chiral reagents or other conventional techniques. If one kind of enantiomer of certain compound of the present disclosure is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to give the pure enantiomer. In addition, the enantiomer and the diastereoisomer are generally isolated through chromatography which uses a chiral stationary phase and optionally combines with a chemical derivative method (such as carbamate generated from amine). The compound of the present disclosure may contain an unnatural proportion of atomic isotope at one or more than one atom(s) that constitute the compound. For example, the compound can be radiolabeled with a radioactive isotope, such as tritium ($^{3}H$), iodine-125 ($^{125}I$) or C-14 ($^{14}C$). For another example, deuterated drugs can be formed by replacing hydrogen with

heavy hydrogen, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure. The term "optional" or "optionally" means that the subsequent event or condition may occur but not requisite, that the term includes the instance in which the event or condition occurs and the instance in which the event or condition does not occur.

The term "substituted" means one or more than one hydrogen atom(s) on a specific atom are substituted by the substituent, including deuterium and hydrogen variables, as long as the valence of the specific atom is normal and the substituted compound is stable. When the substituent is an oxygen (i.e., =O), it means two hydrogen atoms are substituted. Positions on an aromatic ring cannot be substituted by a ketone. The term "optionally substituted" means an atom can be substituted by a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted by 0-2 R, the group can be optionally substituted by up to two R, wherein the definition of R at each occurrence is independent. Moreover, a combination of the substituent and/or the variant thereof is allowed only when the combination results in a stable compound.

When the number of a linking group is 0, such as -(CRR)o-, it means that the linking group is a single bond.

When one of the variables is selected from a single bond, it means that the two groups linked by the single bond are connected directly. For example, when L in A-L-Z represents a single bond, the structure of A-L-Z is actually A-Z.

When a substituent is vacant, it means that the substituent does not exist, for example, when X is vacant in A-X, the structure of A-X is actually A. When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring.

When the enumerative linking group does not indicate the direction for linking, the direction for linking is arbitrary, for example, the linking group L contained in

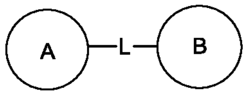

is -M-W-, then -M-W- can link ring A and ring B to form

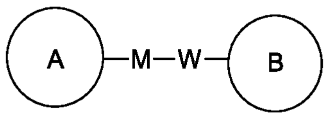

in the direction same as left-to-right reading order and form

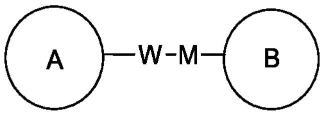

in the direction contrary to left-to-right reading order. A combination of the linking groups, substituents and/or variables thereof is allowed only when such combination can result in a stable compound.

Unless otherwise specified, when a group has one or more linkable sites, any one or more sites of the group can be linked to other groups through chemical bonds. The chemical bond between the site and other groups can be represented by a straight solid bond (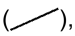), a straight dashed bond (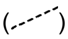) or a wavy line (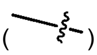) For example, the straight solid bond in $-OCH_3$ represents that it is connected to other groups through the oxygen atom in the group; the straight dashed bond in

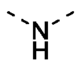

represents that it is connected to other groups through the two ends of the nitrogen atom in the group. The wavy line in

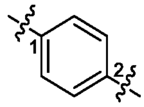

represents that the phenyl group is connected to other groups through the 1 and 2 carbon atoms.

Unless otherwise specified, the number of atoms in a ring is usually defined as the number of ring members. For example, "5-7 membered ring" refers to a "ring" in which 5-7 atoms are arranged around.

Unless otherwise specified, "5-membered ring" refers to a cycloalkyl, heterocycloalkyl, cycloalkenyl, heterocycloalkenyl, cycloalkynyl, heterocycloalkynyl, aryl, or heteroaryl composed of 5 ring atoms. The ring includes a single ring, as well as a bicyclic system such as a spiro ring, a fused ring, and a bridged ring. Unless otherwise specified, the ring optionally contains 1, 2, or 3 heteroatoms independently selected from O, S, and N. The term "ring" also includes a ring system containing at least one ring, where each "ring" independently meets the above definition.

Unless otherwise specified, "D" means deuterium (2H).

Unless otherwise specified, the term "$C_{1-3}$ alkyl" refers to a linear or branched saturated hydrocarbon group containing 1 to 3 carbon atoms. The $C_{1-3}$ alkyl group includes $C_{1-2}$ and $C_{2-3}$ alkyl groups and the like; it can be monovalent (such as methyl), divalent (such as methylene) or multivalent (such as methine). Examples of $C_{1-3}$ alkyl include but are not limited to methyl (Me), ethyl (Et), propyl (including *n*-propyl and isopropyl), etc.

Unless otherwise specified, the term "$C_{1-3}$ haloalkyl" represents monohaloalkyl and polyhaloalkyl containing 1 to 3 carbon atoms. The $C_{1-3}$ haloalkyl group includes $C_{1-2}$, $C_{2-3}$, $C_3$, $C_2$, and $C_1$ haloalkyl, etc. Examples of $C_{1-3}$ haloalkyl include, but are not limited to, trifluoromethyl, trichloromethyl, 2,2,2-trifluoroethyl, pentafluoroethyl, pentachloroethyl, 3-bromopropyl, etc.

Unless otherwise specified, the term "$C_{1-3}$ alkoxy" refers to an alkyl group containing 1 to 3 carbon atoms that are connected to the rest of the molecule through an oxygen atom. The $C_{1-3}$ alkoxy includes $C_{1-2}$, $C_{2-3}$, $C_3$ and $C_2$ alkoxy, etc. Examples of $C_{1-3}$ alkoxy include, but are not limited to, methoxy, ethoxy, propoxy (including n-propoxy and isopropoxy), etc.

Unless otherwise specified, "$C_{3-4}$ cycloalkyl" represents a saturated cyclic hydrocarbon group composed of 3 to 4 carbon atoms, which is a monocyclic ring system, which can be monovalent, divalent or multivalent. Examples of $C_{3-4}$ cycloalkyl include, but are not limited to, cyclopropyl, cyclobutyl, etc.

Unless otherwise specified, the terms "$C_{6-10}$ aromatic ring" and "$C_{6-10}$ aryl" can be used interchangeably, and the term "$C_{6-10}$ aromatic ring" or "$C_{6-10}$ aryl" represents a cyclic hydrocarbon group composed of 6 to 10 carbon atoms with a conjugated $\pi$-electron system, which can be a monocyclic, fused bicyclic or fused tricyclic system, in which each ring is aromatic. It can be monovalent, divalent or multivalent. $C_{6-10}$ aryl groups include $C_{6-9}$, $C_9$, $C_{10}$, and $C_6$ aryl groups. Examples of $C_{6-10}$ aryl groups include, but are not limited to, phenyl, naphthyl (including 1-naphthyl, 2-naphthyl, etc.).

Unless otherwise specified, Cn-n+m or $C_{n-Cn+m}$ includes any specific case of n to n+m carbons, for example, $C_{1-12}$ includes $C_1$, $C_2$, $C_3$, $C_4$, $C_5$, $C_6$, $C_7$, $C_8$, $C_9$, $C_{10}$, $C_{11}$, and $C_{12}$, and any range from n to n+m is also included, for example $C_{1-12}$ includes $C_{1-3}$, $C_{1-6}$, $C_{1-9}$, $C_{3-6}$, $C_{3-9}$, $C_{3-12}$, $C_{6-9}$, $C_{6-12}$, and $C_{9-12}$, etc.; similarly, n membered to n+m membered means that the number of atoms on the ring is from n to n+m, for example, 3-12 membered ring includes 3 membered ring, 4 membered ring, 5 membered ring, 6 membered ring, 7 membered ring, 8 membered ring, 9 membered ring, 10 membered ring, 11 membered ring, and 12 membered ring, and any range from n to n+m is also included, for example, 3-12 membered ring includes 3-6 membered ring, 3-9 membered ring, 5-6 membered ring, 5-7 membered ring, 6-7 membered ring, 6-8 membered ring, and 6-10 membered ring, etc.

The term "leaving group" refers to a functional group or atom which can be replaced by another functional group or atom through a substitution reaction (such as affinity substitution reaction). For example, representative leaving groups include triflate; chlorine, bromine, and iodine; sulfonate group, such as mesylate, tosylate, p-bromobenzenesulfonate, p-toluenesulfonates and the like; acyloxy, such as acetoxy, trifluoroacetoxy and the like.

The term "protecting group" includes, but is not limited to "amino protecting group", "hydroxy protecting group" or "thio protecting group". The term "amino protecting group" refers to a protecting group suitable for blocking the side reaction on the nitrogen of an amino. Representative amino protecting groups include, but are not limited to: formyl; acyl, such as alkanoyl (e.g., acetyl, trichloroacetyl or trifluoroacetyl); alkoxycarbonyl, such as tert-butoxycarbonyl (Boc); arylmethoxycarbonyl such as benzyloxycarbonyl (Cbz) and 9-fluorenylmethoxycarbonyl (Fmoc); arylmethyl, such as benzyl (Bn), trityl (Tr), 1,1-bis-(4'-methoxyphenyl)methyl; silyl, such as trimethylsilyl (TMS) and tert-butyldimethylsilyl (TBS) and the like. The term "hydroxy protecting group" refers to a protecting group suitable for blocking the side reaction on hydroxy. Representative hydroxy protecting groups include, but are not limited to: alkyl, such as methyl, ethyl, and tert-butyl; acyl, such as alkanoyl (e.g., acetyl); arylmethyl, such as benzyl (Bn), p-methoxybenzyl (PMB), 9-fluorenylmethyl (Fm), and diphenylmethyl (benzhydryl, DPM); silyl, such as trimethylsilyl (TMS) and tert-butyl dimethyl silyl (TBS) and

the like.

The structure of the compounds of the present disclosure can be confirmed by conventional methods known to those skilled in the art, and if the disclosure involves an absolute configuration of a compound, then the absolute configuration can be confirmed by means of conventional techniques in the art. For example, in the case of single crystal X-ray diffraction (SXRD), the absolute configuration can be confirmed by collecting diffraction intensity data from the cultured single crystal using a Bruker D8 venture diffractometer with CuKα radiation as the light source and scanning mode: φ/ ω scan, and after collecting the relevant data, the crystal structure can be further analyzed by direct method (Shelxs97).

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred implementations include but are not limited to the embodiments of the present disclosure.

The solvents used in the present disclosure are commercially available.

The present disclosure uses the following abbreviations: EtOH represents ethanol; PE represents petroleum ether; EA represents ethyl acetate; MeOH represents methanol; DCM represents dichloromethane; THF represents tetrahydrofuran; DMF represents N,N'-dimethylformamide; DMA represents N,N'-dimethylacetamide; DMAC represents N,N'-dimethylacetamide; TBSCl represents tert-butyldimethyl silicon chloride; LiCl represents lithium chloride; $K_2CO_3$ represents potassium carbonate; $Na_2CO3$ represents sodium carbonate; T3P represents propylphosphonic anhydride; DMSO represents dimethyl sulfoxide; DMAP represents 4-dimethylaminopyridine; $Pd(OAc)_2$ represents palladium acetate; $NH_4Cl$ represents ammonium chloride; HCl represents hydrochloric acid; IBX represents 2-Iodoxybenzoic acid; MeCN represents acetonitrile; KF represents potassium fluoride; XPhos represents 2-dicyclohexylphosphine-2,4,6-triisopropylbiphenyl; TLC represents thin layer chromatography; HPLC represents High pressure liquid chromatography; SFC represents supercritical fluid chromatography; NCS represents N-chlorosuccinimide; DBU represents 1,8-diazabicyclo[5.4.0]undec-7-ene; iPrOH represents isopropanol; TFAA represents trifluoroacetic anhydride; Tris represents trihydroxymethyl aminomethane.

**Detailed description of the preferred embodiment**

The present disclosure will be described in detail by the following examples, but it does not mean any unfavorable limitation of the present disclosure. The present disclosure has been described in detail herein, and specific embodiments thereof have also been disclosed. For those skilled in the art, various changes and modifications made to the specific embodiments of the present disclosure without departing from the spirit and scope of the invention are obvious.

**Synthesis of Intermediate Compound A1**

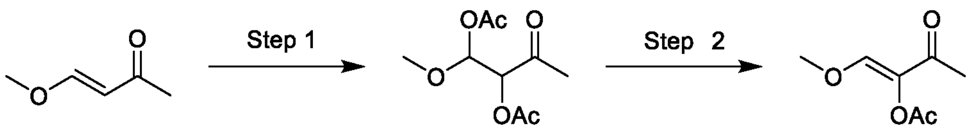


A1-1 A1-2 A1

Step 1

A1-1 (25 g, 249.71 mmol) was dissolved in toluene (250 mL) and lead tetraacetate (110.72 g, 249.71 mmol) was added. The reaction mixture was heated to 50 °C, stirred for 12 hours, and additional lead tetraacetate (60 g, 135.32 mmol) was added, and the reaction was continued at 50 °C for 12 hours. The reaction mixture was then filtered, and the filtrate was concentrated under reduced pressure to remove the solvent to obtain the crude product. The crude product was distilled under reduced pressure under high vacuum, and fractions at 90-100°C were collected to obtain product A1-2.

Step 2

A1-2 (20 g, 91.66 mmol) was added to pyridine (7.40 mL, 91.66 mmol), heated to 115 °C, stirred and the reaction was carried out for 4 hours, and then the reaction mixture was concentrated under reduced pressure. MeOH (7 mL) was added to the residue, cooled to 0 °C, filtered, and the cake was dried to obtain product A1. $^1H$ NMR (400

MHz, $CDCl_3$): δ 7.19 (s, 1H), 3.92 (s, 3H), 2.26 (s, 3H), 2.20 (s, 3H).

**Synthesis of Intermediate Compound A2**

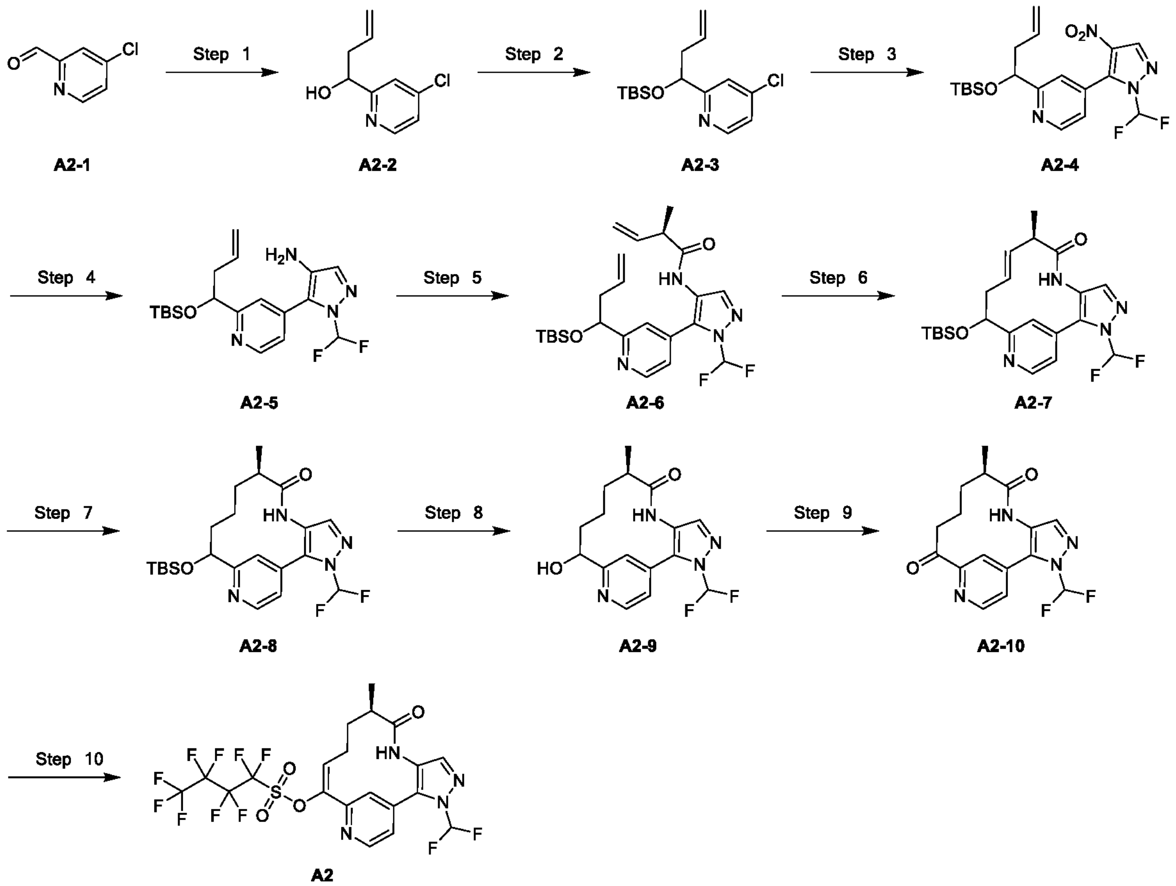


A2-1 A2-2 A2-3 A2-4

A2-5 A2-6 A2-7

A2-8 A2-9 A2-10

A2

Step 1

Compound A2-1 (60 g, 423.86 mmol) was dissolved in THF (600 ml) and water (300 ml), and allyl bromide (76.92 g, 635.80 mmol) and indium (73.00 g, 635.80 mmol) were added at 15 °C. The reaction mixture was stirred at 15°C for 12 hours and then filtered. Ethyl acetate (1 L) was added to the filtrate, the organic phase was separated, and the aqueous phase was extracted with ethyl acetate (1 L×2). The organic phases were combined, washed with saturated brine (1 L×3), dried over anhydrous sodium sulfate, and filtered. The filtrate was concentrated under reduced pressure to obtain compound A2-2. LCMS m/z (ESI): 184.2 (M+1). $^1H$ NMR (400 MHz, $CDCl_3$): δ 8.44 (d, *J*=5.2 Hz, 1H), 7.37 (s, 1H), 7.21 (d, *J=4.4* Hz, 1H), 5.86-5.76 (m, 1H), 5.15-5.11 (m, 2H), 4.80-4.78 (m, 1H), 2.68-2.62 (m, 1H), 2.50-2.45 (m, 1H).

Step 2

A2-2 (62 g, 337.63 mmol) was dissolved in DMF (500 mL), then imidazole (57.46 g, 844.07 mmol) and TBSCl (61.07 g, 405.15 mmol) were added. The mixture was stirred at 15°C for 12 hours, then water (1.5 L) was added and extracted with ethyl acetate (1 L). The organic phase was separated, washed with saturated brine (300 mL×4), dried over anhydrous sodium sulfate, and filtered. The filtrate was concentrated under reduced pressure to obtain compound A2-3. LCMS m/z (ESI): 298.1 (M+1).

Step 3

A2-3 (80 g, 268.55 mmol), 1-difluoromethyl-4-nitropyrazole (43.80 g, 268.55 mmol), $K_2CO_3$ (74.23 g, 537.10 mmol), n-butyl bis (1-adamantyl) phosphine (9.63 g, 26.86 mmol) and 2, 2-dimethylpropionic acid (8.23 g, 80.57 mmol)

were added to 1, 4-dioxane (800 ml), replaced with nitrogen three times, and then $Pd(OAc)_2$ (3.01 g, 13.43 mmol) was added. The reaction mixture was heated to 80°C, stirred for 12 hours, and filtered. Ethyl acetate (500 mL) was added to the filtrate, then the filtrate was washed with saturated brine (500 mL×2), dried over anhydrous sodium sulfate, and filtered. The filtrate was concentrated under reduced pressure, and the residue was purified by column chromatography (PE:EA = 3:1 (V:V)) to obtain compound A2-4. LCMS m/z (ESI):425.3 (M+1).

Step 4

A2-4 (36 g, 84.80 mmol) was dissolved in MeOH (400 mL), zinc powder (55.45 g, 848.02 mmol) and solid $NH_4Cl$ (45.36 g, 848.02 mmol) were added at 0°C, then stirred for 2 h, filtered. The filter cake was washed with MeOH (100 mL×3), the filtrate was collected, concentrated under reduced pressure to remove most of the organic solvent, and then extracted with ethyl acetate (500 mL×2). The organic phases were combined and washed with saturated brine (300 mL×3), dried over anhydrous sodium sulfate, and filtered. The filtrate was concentrated under reduced pressure to obtain compound A2-5. LCMS m/z (ESI):395.3 (M+1).

Step 5

A2-5 (46 g, 116.59 mmol), (2R)-2-methyl-3-butenoic acid (11.67 g, 116.59 mmol) and pyridine (8.45 g, 233.19 mmol) were dissolved in THF (500 mL), replaced with nitrogen three times, cooled to 0 °C, T3P (111.29 g, 174.89 mmol, 50% in ethyl acetate) was added. The reaction mixture was slowly warmed to 20°C and stirred for 12 hours. Ethyl acetate (200 mL) was added to the reaction mixture and washed with saturated salt water (200 mL × 3). The organic phase was separated, dried over anhydrous sodium sulfate, filtered, and the filtrate was concentrated under reduced pressure. The residue was purified by column chromatography (PE:EA=3:1 (V:V)) to obtain compound A2-6. LCMS m/z (ESI):477.3 (M+1).

Step 6

A2-6 (26.00 g, 54.55 mmol) was dissolved in ethyl acetate (4 L), Hoveyda-Grubbs second-generation catalyst (10.25 g, 16.36 mmol) was added, the mixture was replaced with nitrogen for 3 times, and then heated to 90 °C and stirred for 12 hours. The reaction mixture was quenched with saturated aqueous $Na_2CO3$ solution (500 mL). The organic phase was separated, washed with saturated brine (500 mL×2), dried over anhydrous sodium sulfate, and filtered. The filtrate was concentrated under reduced pressure and the residue was purified by column chromatography (PE:EA =1:1 (V:V)) to obtain compound A2-7. LCMS m/z (ESI):449.3 (M+1).

Step 7

A2-7 (21.00 g, 46.81 mmol) was dissolved in MeOH (1 L), palladium on carbon (10 g, 46.81 mmol, 10% content) was added, and the mixture was stirred under hydrogen (15 psi) at 20 °C for 24 hours. The reaction mixture was filtered, and the filtrate was concentrated under reduced pressure to obtain compound A2-8. LCMS m/z (ESI):451.3 (M+1).

Step 8

A2-8 (20.00 g, 44.39 mmol) was dissolved in 1,4-dioxane (250 mL) and HCl/1,4-dioxane (263.16 mL, 4 M) was added. The reaction mixture was stirred at 15°C for 12 hours and then concentrated under reduced pressure. Saturated aqueous $Na_2CO3$ solution (50 mL) was added to the residue and extracted with ethyl acetate (50 mL × 4). The organic phases were combined, dried over anhydrous sodium sulfate, and filtered. The filtrate was concentrated under reduced pressure to obtain compound A2-9. LCMS m/z (ESI):337.2 (M+1).

Step 9

A2-9 (8.5 g, 25.27 mmol) was dissolved in DMSO (50 mL), IBX (14.15 g, 50.54 mmol) was added at 20 °C, and stirred for 2 hours. The reaction mixture was poured into water (200 mL) and extracted with ethyl acetate (200 mL×3). The organic phases were combined, dried over anhydrous sodium sulfate, and filtered. The filtrate was concentrated under reduced pressure to obtain compound A2-10. LCMS m/z (ESI):335.3 (M+1).

Step 10

A2-10 (8.5 g, 25.42 mmol) was dissolved in DMF (100 mL) and toluene (100 ml), cooled to-78 °C under nitrogen,

and LiHMDS (63.56 mL, 1 M) was added. The reaction mixture was stirred at -78 °C for 0.5 h, then perfluorobutylsulfonyl fluoride (22.74 g, 76.27 mmol) was added. The temperature was slowly raised to 15 °C and stirred for 0.5 h, then quenched with saturated aqueous $NH_4Cl$ solution (100 mL), and extracted with ethyl acetate (100 mL×2). The organic phases were combined, dried over anhydrous sodium sulfate, and filtered. The filtrate was concentrated under reduced pressure and the residue was purified by column chromatography (PE:EA = 1:1 (V:V)) to obtain compound A2. LCMS m/z (ESI):617.0 (M+1).

**Synthesis of Intermediate Compound A3**

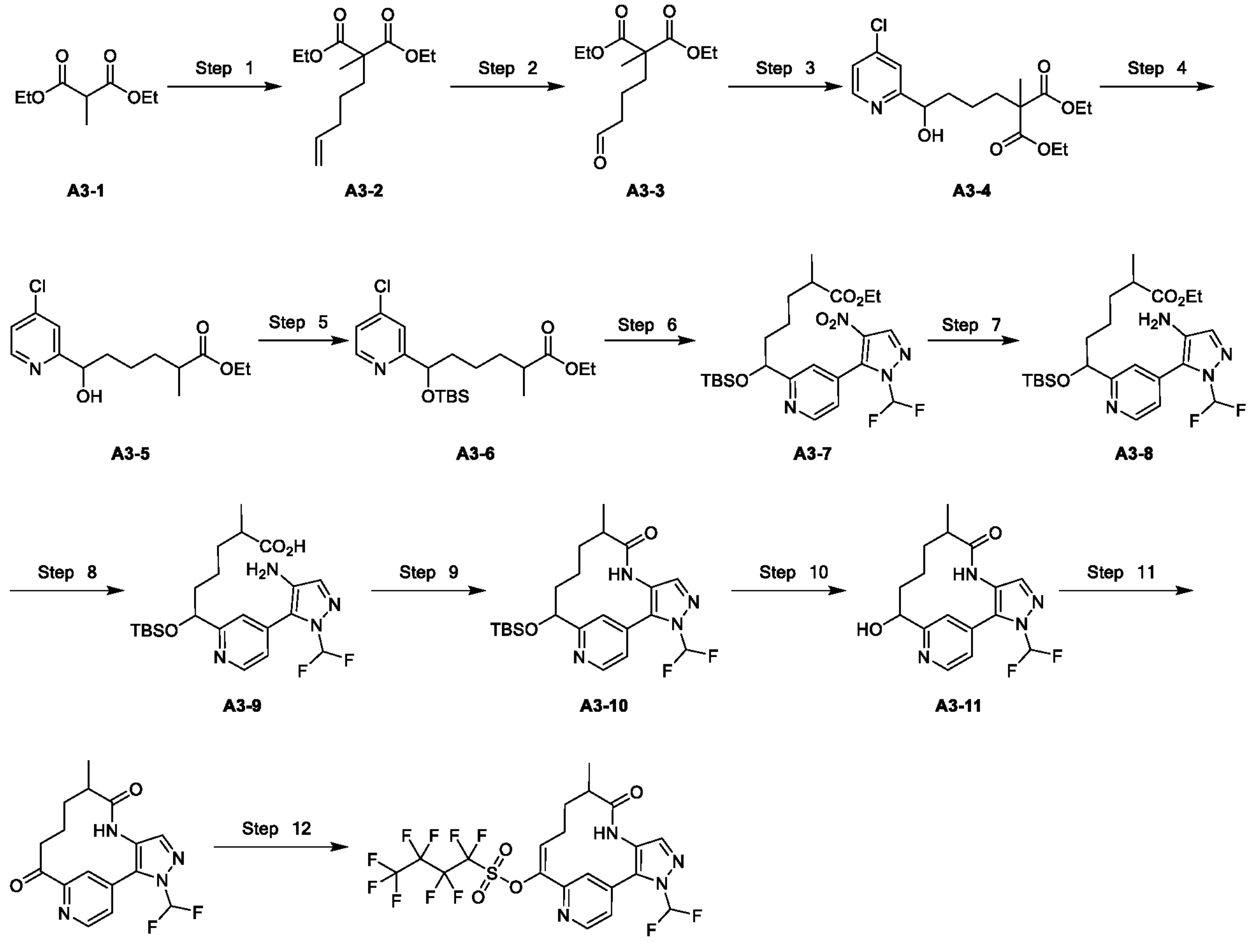


A3-1 A3-2 A3-3 A3-4 A3-5 A3-6 A3-7 A3-8 A3-9 A3-10 A3-11 A3-12 A3

Step 1

EtOH (120 ml) and metallic sodium (3.96 g, 172.22 mmol) were added to a pre-dried flask, stirred at 25°C for 0.5 hours, and raw materials A3-1 (30 g, 172.22 mmol) and 5-bromo-1-pentene (25.67 g, 172.22 mmol) were added to the mixture. After purging nitrogen three times, the mixture was stirred at 95°C for 5 hours, and the reaction mixture was cooled to room temperature. Saturated citric acid aqueous solution (200 mL) was poured into the reaction mixture, then ethyl acetate (100 mL) was added, the organic phase was separated, and then the aqueous phase was extracted with ethyl acetate (100 mL × 3). The organic phases were combined and washed with water (100 mL×2). After the organic phases were combined and concentrated under reduced pressure, the crude product was purified by column chromatography (PE:EA=300:1 to 100:1 (V:V)) to obtain compound A3-2. $^1H$ NMR (400MHz, $CDCl_3$): δ 5.79 (tdd, *J*=6.6, 10.2, 17.0 Hz, 1H), 5.06-4.93 (m, 2H), 4.18 (q, J=7.0 Hz, 4H), 2.11-2.03 (m, 2H), 1.91-1.83 (m, 2H), 1.40 (s, 3H), 1.39-1.30 (m, 2H), 1.25 (t, *J*=7.0 Hz, 6H).

Step 2

A3-2 (43 g, 177.46 mmol) was added to a 500 mL three-necked flask, followed by MeOH (120 mL) and DCM (240 mL), and ozone (8.52 g, 177.46 mmol) was introduced into the reaction mixture at -70°C until the color of the solution turned blue. After stirring for 0.5 hours at -70 °C, nitrogen was introduced into the system for 10 minutes, and $PPh_3$ (51.20 g, 195.20 mmol) was added, and the temperature was raised to 25 °C and stirred for 2 hours. The reaction mixture was concentrated under reduced pressure, and the crude product was purified by column chromatography (PE:EA=100:1 to 20:1 (V:V)) to obtain compound A3-3.

Step 3

2-Bromo-4-chloropyridine (28.60 g, 148.60 mmol) and toluene (500 mL) were added to a pre-dried flask, and the temperature was lowered to -78°C, to which was added n-BuLi (59.44 mL, 2.5 M). A3-3 (33 g, 135.09 mmol) and toluene (500 mL) were added to another flask, and the previous lithium reagent solution was slowly added dropwise to this solution at -78 °C, and stirred at this temperature for 0.5 h. The reaction mixture was poured into saturated ammonium chloride solution (200 mL), ethyl acetate (200 mL) was added thereto, and the layers were allowed to separate. After the organic phase was separated, the aqueous phase was extracted with ethyl acetate (100 mL×3), and the organic phases were combined and concentrated under reduced pressure. The crude product was purified by column chromatography (PE:EA=100:1 to 3:1 (V:V)) to obtain compound A3-4. LCMS m/z (ESI): 358.1 (M+1). $^1H$ NMR (400MHz, $CDCl_3$): δ 8.44 (d, *J*=5.6 Hz, 1H), 7.32 (d, *J*=1.6 Hz, 1H), 7.22 (dd, *J*=2.0, 5.5 Hz, 1H), 4.73 (td, *J*=4.6, 8.8 Hz, 1H), 4.23-4.11 (m, 4H), 3.78 (d, *J*=5.6 Hz, 1H), 2.07-1.80 (m, 3H), 1.68 (dt, *J*=8.0, 14.3 Hz, 1H), 1.46-1.36 (m, 4H), 1.28-1.19 (m, 6H).

Step 4

DMSO (120 mL), A3-4 (38 g, 106.20 mmol), LiCl (9.00 g, 212.39 mmol) and water (1.91 g, 106.20 mmol) were added into a pre-dried flask, and nitrogen was purged three times, then the mixture was heated to 180 °C and stirred for 24 hours. The reaction mixture was poured into water (200 ml) and ethyl acetate (200 ml) was added. The organic phase was separated, washed with saturated brine (200 mL × 3), and concentrated under reduced pressure to obtain compound A3-5. LCMS m/z (ESI): 286.1 (M+1).

Step 5

A3-5 (27 g, 94.48 mmol) and DCM (200 ml) were added to a pre-dried flask, TBSCl (28.48 g, 188.97 mmol), DMAP (8.66 g, 70.86 mmol) and imidazole (16.08 g, 236.21 mmol) were added, and stirred at 20 °C for 2 hours. The reaction mixture was filtered, and the filtrate was concentrated under reduced pressure. The residue was purified by column chromatography (PE:EA=300:1 to 10:1 (V:V)) to obtain compound A3-6. LCMS m/z (ESI): 400.2 (M+1). $^1H$ NMR (400MHz, $CDCl_3$): δ 8.38 (d, *J*=5.4 Hz, 1H), 7.51-7.47 (m, 1H), 7.16 (dd, *J*=2.0, 5.4 Hz, 1H), 4.78 (t, *J*=6.0 Hz, 1H), 4.18-4.05 (m, 2H), 2.39 (qd, *J*=6.8, 13.7 Hz, 1H), 1.78-1.58 (m, 2H), 1.49-1.30 (m, 2H), 1.26-1.20 (m, 5H), 1.11 (d, *J*=7.6 Hz, 3H), 0.99-0.90 (m, 9H), 0.07 (s, 3H), -0.07 (s, 3H).

Step 6

A3-6 (11 g, 27.50 mmol), 1-difluoromethyl-4-nitropyrazole (4.48 g, 27.50 mmol), n-butyl bis (1-adamantyl) phosphine (2.96 g, 8.25 mmol), $K_2CO_3$ (9.50 g, 68.75 mmol), 2, 2-dimethylpropionic acid (842.53 mg, 8.25 mmol) and 1, 4-dioxane (220 mL) were added in a pre-dried flask, and Pd $(OAc)_2$ (1.23 g, 5.50 mmol) was added after nitrogen purging for three times. The reaction mixture was heated to 100°C and stirred for 13 hours. The reaction mixture was filtered, and ethyl acetate (50 mL) and water (50 mL) were directly added to the filtrate. The organic phase was separated, the aqueous phase was extracted three times with ethyl acetate (50 mL×3), and the organic phases were combined and concentrated under reduced pressure. The crude product was purified by column chromatography (PE:EA=300:1 to 10:1 (V:V)) to obtain compound A3-7. $^1H$ NMR (400MHz, $CDCl_3$): δ 8.71 (d, *J*=5.0 Hz, 1H), 8.34 (s, 1H), 7.57 (s, 1H), 7.25 (s, 1H), 7.10 (t, *J*=57.2Hz, 1H), 4.98-4.86 (m, 1H), 4.21-4.03 (m, 2H), 2.45-2.33 (m, 1H), 1.87-1.74 (m, 2H), 1.47-1.31 (m, 2H), 1.29-1.20 (m, 5H), 1.10 (d, *J*=7.0 Hz, 3H), 0.88 (s, 9H), 0.08 (s, 3H), -0.07 (s, 3H).

Step 7

A3-7 (11 g, 20.89 mmol), EtOH (110 mL) and water (30 mL) were added into a pre-dried flask, $NH_4Cl$ (5.59 g, 104.43 mmol) and iron powder (5.83 g, 104.43 mmol) were added after nitrogen purging for three times, and the reaction

was carried out at 80 °C for 1 hour. The reaction mixture was filtered, and the filter cake was washed with ethyl acetate (30 mL×3). The filtrates were combined and concentrated under reduced pressure to obtain compound A3-8. $^1$H NMR (400MHz, $CDCl_3$): δ 8.62 (d, *J*=5.4 Hz, 1H), 7.60 (s, 1H), 7.43 (s, 1H), 7.26-6.95 (m, 2H), 4.94-4.83 (m, 1H), 4.18-4.03 (m, 2H), 3.18 (br s, 2H), 2.45-2.32 (m, 1H), 1.87-1.73 (m, 2H), 1.71-1.54 (m, 1H), 1.47-1.32 (m, 3H), 1.24-1.17 (m, 3H), 1.10 (dd, *J*=1.0, 6.8 Hz, 3H), 0.91 (s, 9H), 0.09 (s, 3H), -0.07 (s, 3H).

Step 8

A3-8 (8 g, 16.11 mmol), THF (100 ml) and water (50 ml) were added to a pre-dried flask, and KOH (1.81 g, 32.21 mmol) was added to the mixture, which was then stirred at 30°C for 12 hours. Most of the organic solvent was removed from the reaction mixture under reduced pressure. Saturated sodium hydroxide solution was added to the remaining aqueous phase until pH = 13, and then the mixture was extracted with ethyl acetate (50 mL × 3). The combined organic phases were concentrated under reduced pressure to obtain compound A3-9. LCMS m/z (ESI): 469.3 (M+1). $^1$H NMR (400MHz, $CDCl_3$): δ 8.52-8.45 (m, 1H), 7.57 (br d, *J*=2.4 Hz, 1H), 7.37-7.31 (m, 1H), 7.26-6.89 (m, 2H), 4.79 (br d, *J*=2.9 Hz, 1H), 2.14-2.05 (m, 1H), 1.76-1.61 (m, 2H), 1.56-1.41 (m, 1H), 1.32-1.12 (m, 3H), 0.95-0.80 (m, 12H), 0.06--0.02 (m, 3H), -0.10-0.18 (m, 3H).

Step 9

A3-9 (2.5 g, 5.33 mmol) and DMA (2.5 L) were added into a pre-dried flask, and HATU (4.06 g, 10.67 mmol) and DIEA (1.38 g, 10.67 mmol) were added to the flask at 20 °C. The reaction mixture was stirred at 100 °C for 24 hours, and directly concentrated under reduced pressure. The crude product was purified by column chromatography (PE:EA=100:1 to 1:1 (V:V)) to obtain compound A3-10. $^1$H NMR (400MHz, $CD_3OD$): δ 9.22 (s, 1H), 8.66-8.60 (m, 1H), 7.82-7.61 (m, 2H), 7.54-7.42 (m, 1H), 5.13 (td, *J*=4.6, 11.4 Hz, 1H), 2.59-2.47 (m, 0.5H), 2.36-2.23 (m, 0.5H), 2.14-1.66 (m, 4H), 1.43-1.14 (m, 2H), 1.13-0.95 (m, 3H), 0.92 (d, *J*=2.4 Hz, 9H), 0.12 (d, *J*=6.0 Hz, 3H), 0.01 (d, *J*=13.6 Hz, 3H).

Step 10

MeOH (35 mL), A3-10 (3.8 g, 8.43 mmol) and a MeOH solution of HCl (6.32 mL, 4 M) were added to a pre-dried flask, stirred at 30 °C for 10 hours, and then concentrated under reduced pressure. The obtained crude product was dissolved in water (20 mL), ethyl acetate (20 mL) was added thereto, and stirred for 5 minutes, and the organic phase was separated to remove impurities. Saturated sodium carbonate solution was added to the aqueous phase until pH=8, ethyl acetate (20 mL) was added thereto, and the organic phase was separated. The aqueous phase was extracted with ethyl acetate (20 mL×5), and the organic phases were combined and concentrated under reduced pressure to obtain compound A3-11. LCMS m/z (ESI): 337.2 (M+1). $^1$H NMR (400MHz, $CD_3OD$): δ 8.66 (t, *J*=5.4 Hz, 1H), 7.81-7.48 (m, 3H), 7.46 (d, *J*=4.8 Hz, 1H), 5.04-4.93 (m, 1H), 2.53-2.36 (m, 1H), 1.98-1.71 (m, 3H), 1.61-1.45 (m, 1H), 1.34-1.21 (m, 1H), 1.10-1.01 (m, 3H), 0.94-0.72 (m, 1H).

Step 11

A3-11 (1.9 g, 5.65 mmol), DCM (20 ml) and Dess-Martin reagent (2.88 g, 6.78 mmol) were added to a pre-dried flask, the mixture was heated to 40 °C and stirred for 12 hours. Saturated sodium thiosulfate solution (50 mL) was added to the reaction mixture and stirred for 10 minutes. The organic phase was separated and the aqueous phase was extracted with DCM (20 mL × 3). After the organic phases were combined, concentrated under reduced pressure. The crude product was purified by column chromatography (DCM:MeOH=100:1 to 0:1 (V:V)) to obtain compound A3-12. LCMS m/z (ESI): 335.1 (M+1).

Step 12

A3-12 (7.00 g, 20.94 mmol) was dissolved in DMF (140 mL), DBU (9.56 g, 62.81 mmol) and perfluorobutylsulfonyl fluoride (15.81 g, 52.35 mmol) were added at 0°C, then the reaction was carried out at 0°C for 1 hour. The reaction mixture was quenched with water (600 mL) and extracted with ethyl acetate (500 mL×3). The organic phases were combined, washed with saturated brine (400 mL×3), dried over anhydrous sodium sulfate, and filtered. The filtrate was concentrated under reduced pressure and the residue was purified by column chromatography (PE:EA = 1:1) to obtain compound A3. LCMS m/z (ESI): 617.4 (M+1). $^1$H NMR (400MHz, DMSO- $d_6$): δ 9.63 (s, 1H), 8.89 (d, *J*=5.2 Hz, 1H), 8.01 (t, J=56 Hz, 1H), 7.94 (s, 1H), 7.62-7.60 (m, 1H), 7.50 (br s, 1H), 6.44-6.40 (m, 1H), 2.54 (br s, 1H), 2.18 (br s, 2H), 1.93-1.84 (m, 1H), 1.84-1.63 (m, 1H), 1.05 (d, *J*=8.0 Hz, 3H).

**Synthesis of Intermediate Compound A4**

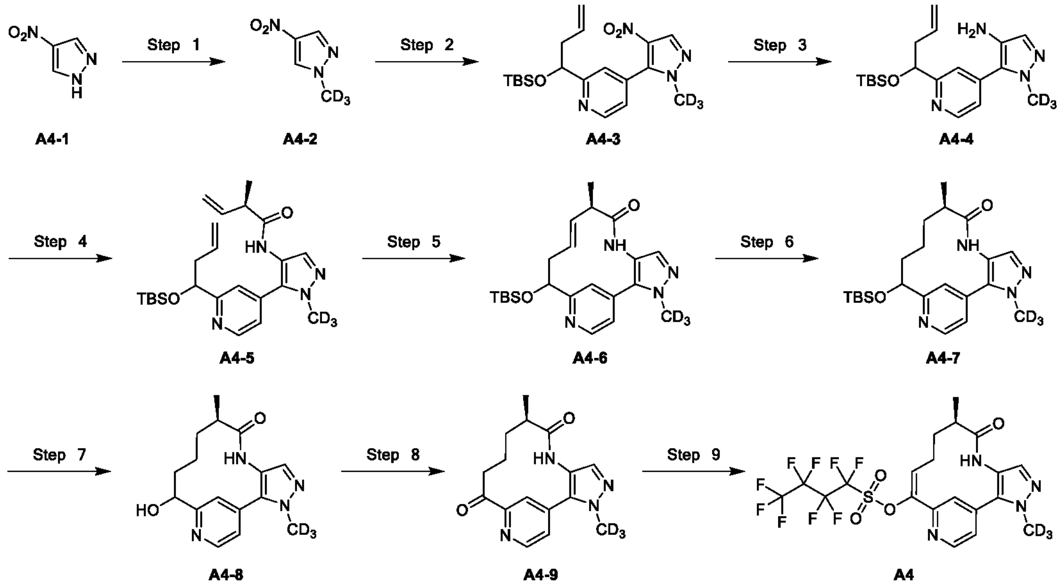


A4-1 A4-2 A4-3 A4-4 A4-5 A4-6 A4-7 A4-8 A4-9 A4

Step 1

Compound A4-1 (11 g, 97.28 mmol) was dissolved in DMF (110 mL), potassium carbonate (14.79 g, 107.01 mmol) and deuterated iodomethane (15.51 g, 107.01 mmol) were added at 5 °C, and then the temperature was raised to 25°C naturally and the reaction was stirred for 1 hour. The reaction mixture was quenched with water (300 mL) and extracted with ethyl acetate (100 mL×3). The organic phases were combined, washed with saturated brine (100 mL), dried over anhydrous sodium sulfate, and filtered. The filtrate was concentrated under reduced pressure to obtain compound A4-2. $^1$H NMR (400MHz, $CDCl_3$): δ 8.13 (s, 1H), 8.05 (s, 1H).

Step 2

A2-3 (24 g, 80.57 mmol), A4-2 (8.39 g, 64.45 mmol), $K_2CO_3$ (27.84 g, 201.41 mmol), n-butyl bis (1-adamantyl) phosphine (2.89 g, 8.06 mmol), $Pd(OAc)_2$ (1.81 g, 8.06 mmol) and 2, 2-dimethylpropionic acid (1.65 g, 16.11 mmol) were added to 1, 4-dioxane (300 mL), the reaction was heated to 90°C under nitrogen protection, stirred for 12 hours, and filtered. The filtrate was concentrated under reduced pressure, and the residue was purified by column chromatography (PE:EA= 6:1) to obtain compound A4-3.

Step 3

A4-3 (8 g, 20.43 mmol) and solid ammonium chloride (8.96 g, 167.54 mmol) were added to MeOH (400 mL), and zinc powder (10.96 g, 167.54 mmol) was added in three portions at 0 °C, then the mixture was stirred for 1 hour at 20°C and filtered. The filtrate was concentrated under reduced pressure, and ethyl acetate (100 mL) was added to the residue for extraction, followed by filtration. The filtrate was concentrated under reduced pressure to obtain compound A4-4.

Step 4

A4-4 (10 g, 27.66 mmol), (2R)-2-methyl-3-butenoic acid (3.05 g, 30.42 mmol) and pyridine (6.56 g, 82.97 mmol) were dissolved in ethyl acetate (100 mL), T3P (35.20 g, 55.31 mmol, 50% in ethyl acetate) was added. The reaction mixture was stirred at 25°C for 2 hours. The reaction mixture was washed with saturated brine (50 mL), dried over anhydrous sodium sulfate, and filtered. The filtrate was concentrated under reduced pressure, and the residue was purified by column chromatography (PE:EA=2:1) to obtain compound A4-5.

Step 5

A4-5 (3.4 g, 7.66 mmol) was dissolved in toluene (1.5 L), Hoveyda-Grubbs second-generation catalyst (1.44 g, 2.30 mmol) was added, and the reaction was heated to 130 °C under nitrogen protection and stirred for 3 hours. The reaction mixture was concentrated under reduced pressure, and the residue was purified by column chromatography (EA) to obtain compound A4-6.

Step 6

A4-6 (2.4 g, 5.77 mmol) was dissolved in MeOH (50 mL), palladium on carbon (1 g, 10% content) was added under nitrogen protection, then replaced with hydrogen several times, and finally the reaction was stirred at 20°C for 48 hours under hydrogen (15 psi). The reaction mixture was filtered, and the filtrate was concentrated under reduced pressure to obtain compound A4-7. LCMS m/z (ESI):418.3 (M+1).

Step 7

A4-7 (1.7 g, 4.07 mmol) was dissolved in 1, 4-dioxane (20 mL) and HCl in 1, 4-dioxane solution (2.04 mL, 4 M) was added. The reaction mixture was stirred at 20°C for 2 hours and then concentrated under reduced pressure. The residue was dissolved in MeOH (6 mL), then extracted with ethyl acetate (30 mL), and filtered. The filter cake was dried under reduced pressure to obtain compound A4-8.

Step 8

Sodium bicarbonate (830.72 mg, 9.89 mmol) was added to dichloromethane (5 mL), Dess-Martin reagent (2.10 g, 4.94 mmol) and A4-8 (1 g, 3.30 mmol) were added at 0 °C. The reaction mixture was stirred at 20 °C for 1 h, then quenched with saturated aqueous sodium bicarbonate solution (20 mL) and extracted with dichloromethane (20 mL × 3). The organic phases were combined, washed with saturated brine (15 mL), dried over anhydrous sodium sulfate, and filtered. The filtrate was concentrated under reduced pressure, and the residue was purified by column chromatography (EA:MeOH=10:1) to obtain compound A4-9.

Step 9

A4-9 (0.65 g, 2.16 mmol) was dissolved in DMF (15 mL), DBU (1.05 g, 6.90 mmol) was added at 0 °C, and then perfluorobutyl sulfonyl fluoride (1.82 g, 6.04 mmol) was added. The reaction mixture was stirred at 0 °C for 2 hours, then quenched with water (15 mL) and extracted with ethyl acetate (10 mL × 3). The organic phases were combined, washed with saturated brine (10 mL), dried over anhydrous sodium sulfate, and filtered. The filtrate was concentrated under reduced pressure, and the residue was purified by column chromatography (PE:EA=1:3) to obtain compound A4. LCMS m/z (ESI): 616.9 (M+1). $^1H$ NMR (400MHz, DMSO-$d_6$): δ 9.61 (s, 1H), 8.89 (d, *J*=5.2 Hz, 1H), 8.01 (t, *J*=57.6 Hz, 1H), 7.94 (s, 1H), 7.62-7.60 (m, 1H), 7.50 (br s, 1H), 6.44-6.40 (m, 1H), 2.42-2.39 (m, 1H), 2.18 (br s, 2H), 1.86 (br s, 1H), 1.70-1.64 (m, 1H), 1.05 (d, *J*=6.8 Hz, 3H).

**Synthesis of Intermediate Compound A5**

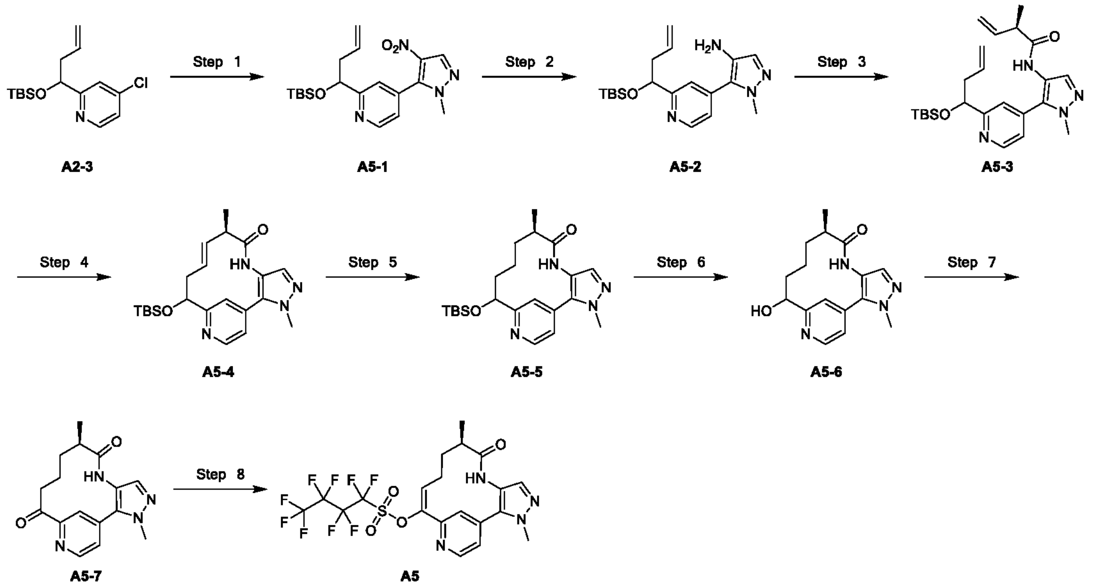


A2-3, A5-1, A5-2, A5-3, A5-4, A5-5, A5-6, A5-7, A5

Step 1

A2-3 (21 g, 70.49 mmol), 1-methyl-4-nitropyrazole (8.96 g, 70.49 mmol), $K_2CO_3$ (24.36 g, 176.24 mmol), n-butylbis(1-adamantyl)phosphine (8.96 g, 70.49 mmol), $Pd(OAc)_2$ (3.17 g, 14.10 mmol) and 2,2-dimethylpropionic acid (2.16 g, 21.15 mmol) were added to 1,4-dioxane (250 mL), heated to 80 °C under nitrogen protection, stirred for 24 hours, and filtered. The filtrate was concentrated under reduced pressure, and the residue was purified by column chromatography (PE:EA = 8:1) to obtain compound A5-1. LCMS m/z (ESI):389.2 (M+1).

Step 2

A5-1 (14 g, 36.03 mmol) was dissolved in MeOH (140 ml), zinc powder (23.56 g, 360.33 mmol) and solid ammonium chloride (19.27 g, 360.33 mmol) were added at 0 °C, then stirred at 20 °C for 2 hours and filtered. The filter cake was washed with MeOH (300 mL×3), and the combined filtrates were concentrated under reduced pressure. The residue was extracted with ethyl acetate (500 mL) and washed with saturated brine (300 mL × 3). The organic phase was separated, dried over anhydrous sodium sulfate, and filtered. The filtrate was concentrated under reduced pressure to obtain compound A5-2. LCMS m/z (ESI):359.3 (M+1).

Step 3

A5-2 (11.4 g, 31.79 mmol), (2R)-2-methyl-3-butenoic acid (3.18 g, 31.79 mmol) and pyridine (5.03 g, 63.59 mmol) were dissolved in THF (120 ml), replaced with nitrogen three times, cooled to 0 °C under nitrogen protection, and then T3P (30.35 g, 47.69 mmol, 50% ethyl acetate solution) was added. The reaction mixture was stirred at 20°C for 12 hours. Ethyl acetate (500 mL) was added to the reaction mixture, followed by washing with saturated brine (200 mL×3). The organic phase was separated, dried over anhydrous sodium sulfate, and filtered. The filtrate was concentrated under reduced pressure, and the residue was purified by column chromatography (PE:EA=2:1) to obtain compound A5-3. LCMS m/z (ESI):441.2 (M+1).

Step 4

A5-3 (7.3 g, 16.57 mmol) was dissolved in toluene (1.5 L), Hoveyda-Grubbs second-generation catalyst (3.11 g, 4.97 mmol) was added, after being replaced with nitrogen for three times, the reaction was heated to 130 °C under nitrogen protection and stirred for 3 hours. Saturated aqueous $Na_2CO_3$ solution (800 mL) was added to the reaction mixture, stirred for 1 hour, and then extracted with ethyl acetate (1000 mL×3). The organic phases were combined and washed with saturated brine (1000 mL×3), then the organic phases were dried over anhydrous sodium sulfate and filtered. The filtrate was concentrated under reduced pressure, and the residue was purified by column chromatography (EA) to obtain compound A5-4.

Step 5

A5-4 (3.6 g, 8.73 mmol) was dissolved in MeOH (150 mL), palladium on carbon (3 g, 10% content) was added under nitrogen protection, then replaced with hydrogen several times, and finally the reaction was stirred at 20°C for 12 hours under hydrogen (15 psi). The reaction mixture was filtered, the filter cake was washed with MeOH (50 mL×3), and the filtrate was concentrated under reduced pressure to obtain compound A5-5. LCMS m/z (ESI):415.1 (M+1).

Step 6

A5-5 (3.6 g, 8.68 mmol) was dissolved in 1, 4-dioxane (30 mL) and HCl in 1, 4-dioxane solution (30 mL, 4 M) was added. The reaction mixture was stirred at 20°C for 2 hours and then concentrated under reduced pressure. The residue was added with water (20 mL), then the pH value was adjusted to 8 with saturated sodium carbonate solution, and the mixture was extracted with a mixed solvent of ethyl acetate and isopropanol (EA:iPrOH=10:1, 50 mL×4). The organic phases were combined, dried over anhydrous sodium sulfate, and filtered. The filtrate was concentrated under reduced pressure to obtain compound A5-6. LCMS m/z (ESI): 301.1 (M+1).

Step 7

A5-6 (2.2 g, 7.32 mmol) was dissolved in DCM (100 mL), then sodium bicarbonate (3.08 g, 36.62 mmol) and Dess-Martin reagent (4.66 g, 10.99 mmol) were added at 0 °C. The reaction mixture was stirred at 0°C for 1 hour, then at 20°C for 12 hours. Water (50 mL), dichloromethane (100 mL) and saturated aqueous sodium sulfite solution (50 mL) were added to the reaction mixture, and stirred for 0.5 h. After the organic phase was separated, the aqueous phase was extracted with dichloromethane (100 mL×3), the organic phases were combined, washed with saturated brine (100 mL×2), dried over anhydrous sodium sulfate, and filtered. The filtrate was concentrated under reduced pressure to obtain compound A5-7. LCMS m/z (ESI): 299.2 (M+1).

Step 8

A5-7 (1 g, 3.35 mmol) was dissolved in DMF (20 mL), DBU (1.53 g, 10.06 mmol) was added at 0 °C, then perfluorobutylsulfonyl fluoride (2.53 g, 8.38 mmol) was added. The reaction mixture was stirred at 0 °C for 1 hours, then quenched with water (30 mL) and extracted with ethyl acetate (30 mL × 3). The organic phases were combined, washed with saturated brine (30 mL×3), dried over anhydrous sodium sulfate, and filtered. The filtrate was concentrated under reduced pressure, and the residue was purified by column chromatography (EA) to obtain compound A5.

**Example 1: Compound 1-1 and Compound 1-2**

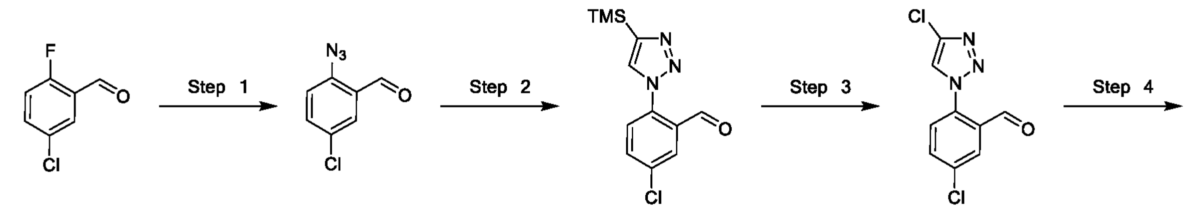


1a 1b 1c 1d

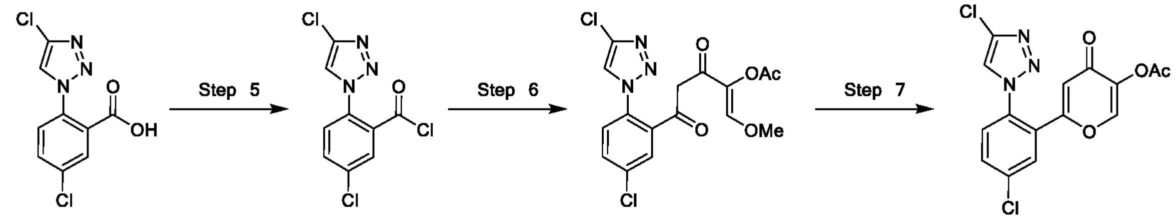


1e 1f 1g 1h

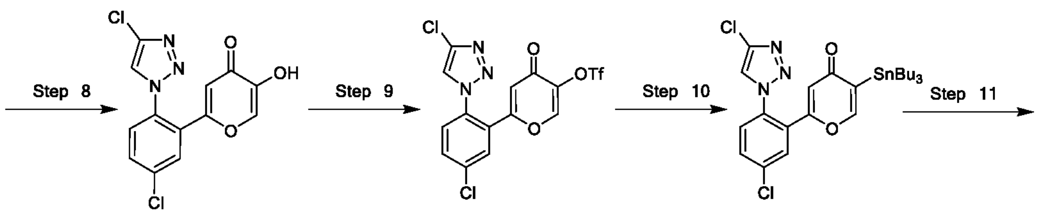


1i 1j 1k

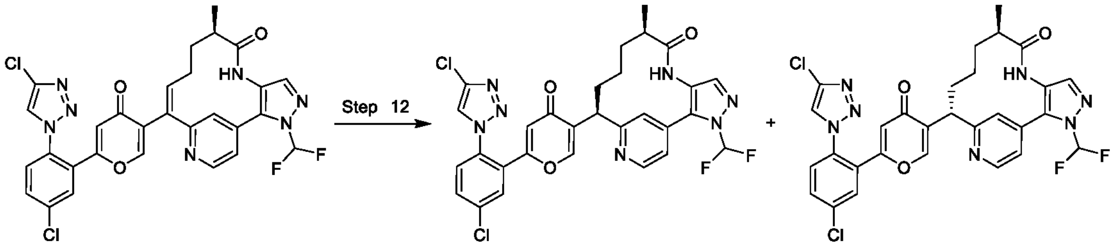


1l 1-1 or 1-2 + 1-2 or 1-1

Step 1

1a (100 g, 630.69 mmol), DMF (400 mL) and $NaN_3$ (41.82 g, 643.30 mmol) were added to a pre-dried 1 L three-necked flask, heated to 55 °C and stirred for 24 hours. The reaction mixture was poured into water (1 L), ethyl acetate (1 L) was added, and the organic phase was separated. The aqueous phase was then extracted with ethyl acetate (1 L×2), and the organic phases were combined and concentrated under reduced pressure to obtain compound 1b. $^1H$ NMR (400MHz, $CDCl_3$): δ 10.29 (s, 1H), 7.85 (d, *J*=2.5 Hz, 1H), 7.57 (dd, *J*=2.5, 8.5 Hz, 1H), 7.24 (d, *J*=8.5 Hz, 1H).

Step 2

1b (110 g, 605.80 mmol), trimethylsilylacetylene (178.50 g, 1.82 mol) and toluene (1.1 L) were added to a pre-dried 2 L flask, the mixture was stirred at 110 °C for 21 hours, and the reaction mixture was concentrated under reduced pressure. The crude product was purified by column chromatography (PE:EA=20:1 to 2:1) to obtain compound 1c. LCMS m/z (ESI): 280.0 (M+1). $^1H$ NMR (400MHz, $CDCl_3$):δ 9.86 (s, 1H), 8.08 (d, *J*=2.4 Hz, 1H), 7.90 (s, 1H), 7.72 (dd, *J*=2.2, 8.4 Hz, 1H), 7.48 (d, *J*=8.4 Hz, 1H), 0.41 (s, 9H).

Step 3

1c (130 g, 464.62 mmol) and MeCN (3.5 L) were added to a pre-dried 5 L flask, and NCS (744.49 g, 5.58 mol) and KF (161.97 g, 2.79 mol) were added at 25 °C. Heated to 90°C and the reaction was carried out for 40 hours. The reaction mixture was directly filtered, and the filtrate was evaporated to dryness to obtain a crude product. Aqueous NaOH solution was added to the crude product until the pH of the mixture was greater than 13, then ethyl acetate (1 L) was added to the mixture and stirred at 25°C for half an hour. After the organic phase was separated, the aqueous phase was extracted with ethyl acetate (1L×3). The organic phases were combined and concentrated under reduced pressure, and the residue was purified by column chromatography (DCM) to obtain compound 1d. LCMS m/z (ESI): 241.9 (M+1). $^1H$ NMR (400MHz, $CDCl_3$): δ 9.88 (s, 1H), 8.09 (d, *J*=2.5 Hz, 1H), 7.94 (s, 1H), 7.76 (dd, *J*=2.5, 8.5 Hz, 1H), 7.49 (d, *J*=8.5 Hz, 1H).

Step 4

Compound 1d (10 g, 41.31 mmol) and $NaH_2PO_4$ (14.87 g, 123.94 mmol) were dissolved in a mixed solvent of tert-butanol (100 mL) and water (100 mL), then 2-methyl-2-butene (57.94 g, 826.24 mmol) and sodium chlorite (11.21 g, 123.94 mmol) were added. The mixture was stirred at 20 °C for 2 h, then washed with aqueous NaOH (40 mL × 2, 1 M) solution. The aqueous phases were combined, acidified with dilute hydrochloric acid (2M) to pH = 3-4, and then extracted with ethyl acetate (60 mL×2). The organic phases were combined, washed with saturated brine (200 mL), dried over anhydrous sodium sulfate, and filtered. The filtrate was concentrated under reduced pressure to obtain compound 1e. LCMS m/z (ESI): 258.0 (M+1).

Step 5

Compound 1e (10.84 g, 42.01 mmol) was dissolved in DCM, oxalyl chloride (10.66 g, 84.01 mmol) and a catalytic amount of DMF (307.03 mg, 4.20 mmol) were added at 0 °C. The reaction mixture was heated to 20°C and stirred for 2 hours, and then concentrated under reduced pressure to obtain compound 1f.

Step 6

Intermediate **A1** (5.66 g, 35.80 mmol) was dissolved in THF (60 mL), cooled to -70 °C and LiHMDS (43.76 mL, 1 M) was added. After the reaction was stirred at -70 °C for 0.5 h, a solution of compound 1f (11 g, 39.78 mmol) in THF (120 mL) was added dropwise. The reaction mixture was slowly heated to 20 °C and stirred for 15 hours, poured into aqueous hydrochloric acid solution (300 mL, 2M), ethyl acetate (100 mL) was added, and extracted with ethyl acetate (100 mL×3). The organic phases were combined and washed successively with water (500 mL) and saturated brine (500 mL), dried over anhydrous sodium sulfate, and filtered. The filtrate was concentrated under reduced pressure to obtain compound 1g.

Step 7

Compound 1g (16 g, 40.18 mmol) was dissolved in toluene (350 mL), p-toluenesulfonic acid pyridinium salt (1.01 g, 4.02 mmol) was added, the reaction was heated to 120 °C and stirred for 5 hours, and then concentrated under reduced pressure. The residue was dissolved in ethyl acetate (200 mL), washed with water (300 mL) and saturated brine (300 mL), dried over anhydrous sodium sulfate, and filtered. The filtrate was concentrated under reduced pressure to obtain compound 1h.

Step 8

Compound 1h (14.1 g, 38.51 mmol) was dissolved in MeOH (150 mL), $K_2CO_3$ (10.64 g, 77.02 mmol) was added, the reaction was stirred at 20 °C for 1 hour, and filtered. The filter cake was washed successively with MeOH (10 mL×5) and ethyl acetate (10 mL×5. The washing solution was collected and concentrated under reduced pressure. The residue was dissolved in ethyl acetate (150 mL), washed with dilute hydrochloric acid (200 mL, 1M), water (200 mL×2) and saturated brine (200 mL×2) successively, dried over anhydrous sodium sulfate, filtered. The filtrate was concentrated under reduced pressure, MeOH (20 mL) was added to the obtained residue, the mixture was well stirred, filtered, and dried to obtain compound 1i. LCMS m/z (ESI): 323.9 (M+1).

Step 9

Compound 1i (2 g, 6.17 mmol) was dissolved in DMF (30 mL), triethylamine (936.60 mg, 9.26 mmol) and trifluoromethanesulfonic anhydride (2.20 g, 6.17 mmol) were added. The reaction mixture was stirred at 15°C for 2 hours, water (120 mL) was added, and the mixture was stirred at 20°C for 0.5 hours. After filtration, the filter cake was dried to obtain compound 1j.

Step 10

Compound 1j (2.2 g, 4.82 mmol), hexabutylditin (3.62 g, 6.24 mmol), $Pd(OAc)_2$ (108.27 mg, 482.27 μmol), XPhos (229.90 mg, 482.27 μmol) and LiCl (1.02 g, 24.11 mmol) were dissolved in DMF (40 mL), and after being replaced by nitrogen for three times, the reaction mixture was heated to 60 °C under nitrogen protection and the reaction was carried out for 2 hours. The reaction mixture was quenched with water (100 mL), extracted with ethyl acetate (100 mL×3). The organic phases were combined, washed with saturated brine (100 mL×3), dried over anhydrous sodium sulfate, and filtered. The filtrate was concentrated under reduced pressure, and the residue was purified by column chromatography (PE:EA=5:1 to 3:1) to obtain compound 1k.

Step 11

Compound 1k (0.8 g, 1.34 mmol), compound A2 (825.78 mg, 1.34 mmol), $Pd(PPh_3)_4$ (77.40 mg, 66.98 μmol), LiCl (283.94 mg, 6.70 mmol) and CuCl (663.14 mg, 6.70 mmol)) was dissolved in DMSO (30 mL), after being replaced with nitrogen three times, the reaction mixture was heated to 80 °C under nitrogen protection and the reaction was carried out for 2 hours. The reaction mixture was quenched with water (100 mL), ethyl acetate (100 mL) was added, and filtered. The organic phase was separated from the filtrate, and the aqueous phase was extracted with ethyl acetate

(100 mL×2). The organic phases were combined, washed successively with saturated $NaHCO_3$ solution (100 mL×2) and saturated brine (100 mL×2), dried over anhydrous sodium sulfate, and filtered. The filtrate was concentrated under reduced pressure, and the residue was purified by column chromatography (DCM:EA=1:0 to 1:1) to obtain compound 11. LCMS m/z (ESI): 624.0 (M+1).

Step 12

Compound 11 (0.22 g, 352.32 μmol) was dissolved in THF (20 mL), chloroform (4.21 mg, 35.23 μmol) was added, and then Raney nickel (30.18 mg, 352.32 μmol) was added under nitrogen protection. The reaction mixture was replaced with hydrogen several times, then the reaction was stirred and carried out under hydrogen (15 psi) at 20 °C for 0.5 h. The reaction mixture was filtered, the filtrate was concentrated under reduced pressure, and the residue was separated by preparative TLC (PE:EA:MeOH = 4:4:1) to obtain Isomer 1 (Rf = 0.51) and Isomer 2 (Rf = 0.47).

Isomer 1 was purified by preparative HPLC to obtain compound 1-1. LCMS m/z (ESI): 626.1 (M+1). $^1H$ NMR (400MHz, $CDCl_3$): δ 8.62 (d, *J*=5.2 Hz, 1H), 8.48 (s, 1H), 8.19 (s, 1H), 7.96 (s, 1H), 7.81-7.83 (m, 1H), 7.69-7.74 (m, 2H), 7.64 (t, J=56.0 Hz, 1H), 7.62 (s, 1H), 7.42 (d, *J*=0.8 Hz, 1H), 6.32 (s, 1H), 4.29-4.34 (m, 1H), 2.72 (br s, 1H), 2.07 (br s, 1H), 1.75-1.95 (m, 2H), 1.55 (br s, 1H), 1.35 (br s, 1H), 0.98 (d, *J*=6.8 Hz, 3H), 0.44 (br s, 1H).

Isomer 2 was purified by preparative HPLC to obtain compound 1-2. LCMS m/z (ESI): 626.1 (M+1). $^1H$ NMR (400MHz, $CDCl_3$): δ 8.60 (d, *J*=4.8 Hz, 1H), 8.49 (s, 1H), 8.23 (s, 1H), 7.97 (s, 1H), 7.81-7.84 (m, 1H), 7.76 (s, 1H), 7.70-7.72 (m, 1H), 7.65 (t, *J*=56.0 Hz, 1H), 7.58 (s, 1H), 7.40 (d, *J*=0.8 Hz, 1H), 6.33 (s, 1H), 4.30 (t, *J*=8.4 Hz, 1H), 2.29 (br s, 1H), 1.76-1.95 (m, 3H), 1.50 (br s, 1H), 1.34 (br s, 1H), 1.24 (d, *J*=7.2 Hz, 3H), 0.69 (br s, 1H).

**Example 2: Compound 2-1 and Compound 2-2**

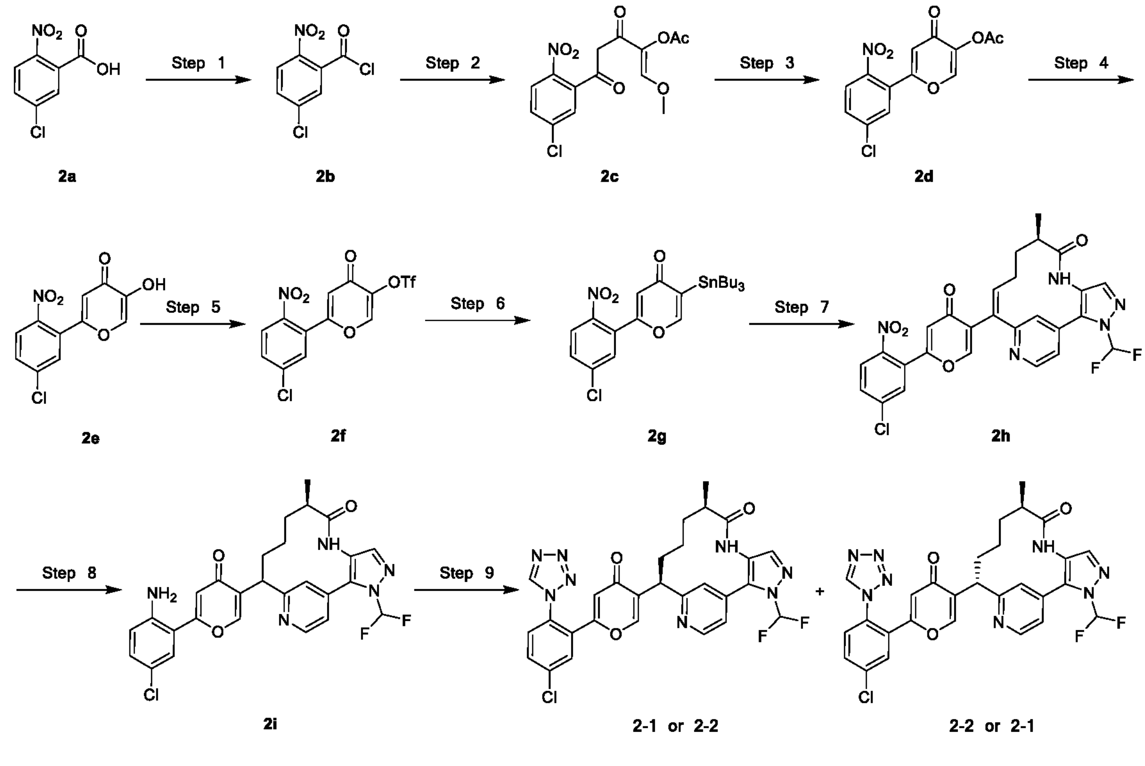


2a, 2b, 2c, 2d, 2e, 2f, 2g, 2h, 2i, 2-1 or 2-2, 2-2 or 2-1

Step 1

Compound 2a (15 g, 74.42 mmol) was dissolved in DCM (150 mL), oxalyl chloride (18.89 g, 148.84 mmol) and a catalytic amount of DMF (543.95 mg, 7.44 mmol) were added at 0 °C. The reaction mixture was heated to 15°C and stirred for 1 hours, and then concentrated under reduced pressure to obtain compound 2b.

Step 2

Intermediate A1 (10.35 g, 65.45 mmol) was dissolved in THF (160 mL), cooled to -78 °C and LiHMDS (72.72 mL, 1 M) was added. After the reaction was stirred at -78 °C for 0.5 h, a solution of compound 2b (16 g, 72.72) in THF (50 mL) was added dropwise. The reaction mixture was slowly heated to 15°C and stirred for 12 hours, poured into aqueous hydrochloric acid solution (500 mL, 2M), and extracted with ethyl acetate (500 mL×2). The organic phases were combined and washed with saturated brine (500 mL×3), dried over anhydrous sodium sulfate, and filtered. The filtrate was concentrated under reduced pressure to obtain compound 2c.

Step 3

Compound 2c (11 g, 32.19 mmol) was dissolved in toluene (500 mL), p-toluenesulfonic acid pyridinium salt (808.99 mg, 3.22 mmol) was added, the reaction was heated to 120 °C and stirred for 1 hours, and then concentrated under reduced pressure. The residue was dissolved in ethyl acetate (500 mL), washed with saturated brine (200 mL×2), dried over anhydrous sodium sulfate, and filtered. The filtrate was concentrated under reduced pressure, and the residue was purified by column chromatography (PE:EA=2:1 to 1:1) to obtain compound 2d. LCMS m/z (ESI): 310.0 (M+1).

Step 4

Compound 2d (14 g, 45.21 mmol) was dissolved in MeOH (140 mL), $K_2CO_3$ (12.50 g, 90.42 mmol) was added, the reaction was stirred at 15 °C for 1 hour, and filtered. Water (50 mL) was added to the filtrate, the pH value was adjusted to 7 with dilute hydrochloric acid (2M) and extracted with ethyl acetate (50 mL × 2). The extracts were combined, washed with saturated brine (40 mL×3), dried over anhydrous sodium sulfate, and filtered. The filtrate was concentrated under reduced pressure, and the resulting residue was well stirred with MeOH (5 mL) at -30°C and filtered. The filter cake was dried to give compound 2e. LCMS m/z (ESI): 268.0 (M+1).

Step 5

Compound 2e (2 g, 7.47 mmol) was dissolved in DMF (20 mL), triethylamine (1.13 g, 11.21 mmol) and trifluoromethanesulfonic anhydride (2.67 g, 7.47 mmol) were added. The reaction mixture was stirred at 15°C for 12 hours, water (200 mL) was added, and the mixture was stirred at 15°C for 0.5 hours. After filtration, the filter cake was dried to obtain compound 2f. LCMS m/z (ESI): 400.0 (M+1).

Step 6

Compound 2f (2.5 g, 6.25 mmol), hexabutylditin (7.26 g, 12.51 mmol), $Pd(PPh_3)_4$ (722.80 mg, 625.49 μmol) and LiCl (1.33 g, 31.27 mmol) were dissolved in toluene (160 mL), and after being replaced by nitrogen for three times, the reaction was heated to 120 °C and carried out for 8 hours under nitrogen protection. The reaction mixture was quenched with water (100 mL), extracted with ethyl acetate (100 mL×2). The organic phases were combined, washed with saturated brine (100 mL×3), dried over anhydrous sodium sulfate, and filtered. The filtrate was concentrated under reduced pressure, and the residue was purified by column chromatography (PE:EA=5:1 to 3:1) to obtain compound 2g.

Step 7

Compound 2g (0.75 g, 1.39 mmol), compound A2 (877.26 mg, 1.39 mmol), $Pd(PPh_3)_4$ (160.30 mg, 138.72 μmol), LiCl (588.08 mg, 13.87 mmol) and CuCl (1.37 g, 13.87 mmol) were dissolved in DMSO (15 mL), after being replaced with nitrogen three times, the reaction mixture was heated to 50°C and the reaction was carried out for 2 h under nitrogen protection. The reaction mixture was quenched with water (40 mL), ethyl acetate (40 mL) was added, and filtered. The organic phase was separated from the filtrate, and the aqueous phase was extracted with ethyl acetate (40 mL×2). The organic phases were combined, washed with saturated brine (40 mL×2), dried over anhydrous sodium sulfate, and filtered. The filtrate was concentrated under reduced pressure, and the residue was purified by column chromatography (DCM:EA=1:1) to obtain compound 2h. LCMS m/z (ESI): 568.0 (M+1).

Step 8

Compound 2h (0.28 g, 493.02 μmol) was dissolved in MeOH (20 mL), then Raney nickel (211.19 mg, 2.47 mmol) was added under nitrogen protection. The reaction mixture was replaced with hydrogen several times, then the reaction was stirred and carried out under hydrogen (15 psi) at 15 °C for 0.5 h. The reaction mixture was filtered, the

filtrate was concentrated under reduced pressure, and the residue was purified by preparative TLC (PE:EA:MeOH=4:4:1) to obtain compound 2i. LCMS m/z (ESI): 540.0 (M+1).

Step 9

Compound 2i (0.11 g, 203.72 μmol) was dissolved in acetic acid (10 mL) and toluene (10 mL), methyl orthoformate (216.19 mg, 2.04 mmol) and trimethylsilyl azide (234.70 mg, 2.04 mmol) were added. The reaction mixture was heated to 90°C and carried out for 5 hours, and then concentrated under reduced pressure. The residue was separated by SFC to obtain isomer 1 ($t_R$ (retention time) = 1.95min) and isomer 2 ($t_R$ (retention time) = 2.15min), which were purified by preparative HPLC to obtain compounds 2-1 and 2-2.

Compound 2-1: LCMS m/z (ESI): 593.1 (M+1). $^1$H NMR (400MHz, $CD_3OD$): δ 9.57 (s, 1H), 8.61 (d, *J*=5.2 Hz, 1H), 8.11 (s, 1H), 8.00 (d, *J*=2.4 Hz, 1H), 7.89-7.83 (m, 1H), 7.79-7.49 (m, 1H), 7.77-7.72 (m, 2H), 7.62 (s, 1H), 7.41 (d, *J*=5.2 Hz, 1H), 6.40 (s, 1H), 4.29 (dd, *J*=4.8, 12.4 Hz, 1H), 2.74-2.69 (m, 1H), 2.12-2.00 (m, 1H), 1.95-1.75 (m, 2H), 1.61-1.47 (m, 1H), 1.29-1.27 (m, 1H), 0.97 (d, *J*=6.8 Hz, 3H), 0.48-0.42 (m, 1H).

Compound 2-2: LCMS m/z (ESI): 593.1 (M+1). $^1$H NMR (400MHz, $CD_3OD$): δ 9.58 (s, 1H), 8.59 (d, *J*=5.2 Hz, 1H), 8.14 (s, 1H), 8.01 (d, *J*=2.4 Hz, 1H), 7.86 (dd, *J*=2.4, 8.4 Hz, 1H), 7.80-7.51 (m, 2H), 7.78-7.74 (m, 2H), 7.57 (s, 1H), 7.39 (d, *J*=5.2 Hz, 1H), 4.32-4.25 (m, 1H), 2.34-2.24 (m, 1H), 1.95-1.83 (m, 1H), 1.82-1.74 (m, 2H), 1.54-1.43 (m, 1H), 1.34-1.29 (m, 1H), 1.24 (d, *J*=6.8 Hz, 3H), 0.74-0.61 (m, 1H).

**Example 3: Compound 3-1 and Compound 3-2**

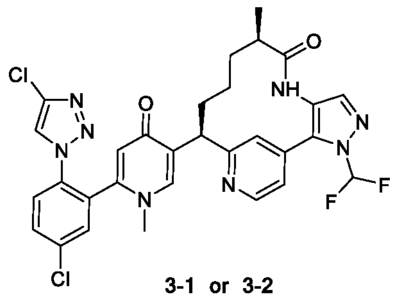

3-1 or 3-2 ,

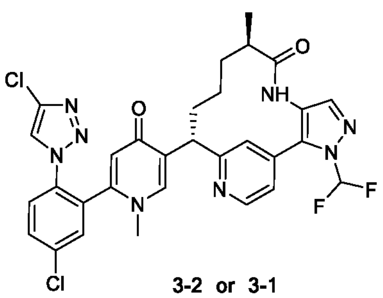

3-2 or 3-1

Compound 1-1 (50 mg, 79.82 μmol) and methylamine (61.97 mg, 798.16 μmol) were mixed, heated to 100 °C and the reaction was carried out for 1 hour. The reaction mixture was poured into water (20 mL) and extracted with EtOAc (30 mL×2). The organic phases were combined, dried over anhydrous sodium sulfate, and filtered. The filtrate was concentrated under reduced pressure, and the residue was purified by preparative thin layer chromatography (PE:EtOAc:MeOH=4:4:1) to obtain compound 3-1. LCMS m/z (ESI): 639.2 (M+1). $^1$H NMR (400 MHz, $CD_3OD$): δ 8.72-8.57 (m, 1H), 8.51-8.32 (m, 1H), 8.16-8.06 (m, 1H), 7.88-7.74 (m, 4H), 7.71-7.64 (m, 1H), 7.42 (br d, *J* = 4.8 Hz, 1H), 6.21-6.07 (m, 1H), 4.60 - 4.42 (m, 1H), 3.64 (d, *J* = 17.3 Hz, 3H), 2.81-2.73 (m, 1H), 2.20-1.98 (m, 2H), 1.90-1.77 (m, 1H), 1.66-1.50 (m, 1H), 1.40-1.31 (m, 1H), 1.00 (d, *J* = 6.9 Hz, 3H), 0.57-0.30 (m, 1H).

Compound 3-2 was obtained by using compound 1-2 as raw material by the same method mentioned above. LCMS m/z (ESI): 639.2 (M+1). $^1$H NMR (400 MHz, $CD_3OD$): δ 8.68-8.57 (m, 1H), 8.50-8.34 (m, 1H), 8.22-8.09 (m, 1H), 7.88-7.76 (m, 4H), 7.68-7.63 (m, 1H), 7.44-7.38 (m, 1H), 6.19-6.09 (m, 1H), 4.53-4.42 (m, 1H), 3.69-3.62 (m, 3H), 2.37-2.25 (m, 1H), 2.05-1.94 (m, 2H), 1.90-1.78 (m, 1H), 1.61-1.48 (m, 1H), 1.44-1.40 (m, 2H), 1.34-1.31 (m, 1H), 1.30 (s, 3H), 0.75-0.58 (m, 1H).

**Example 4: Compound 4**

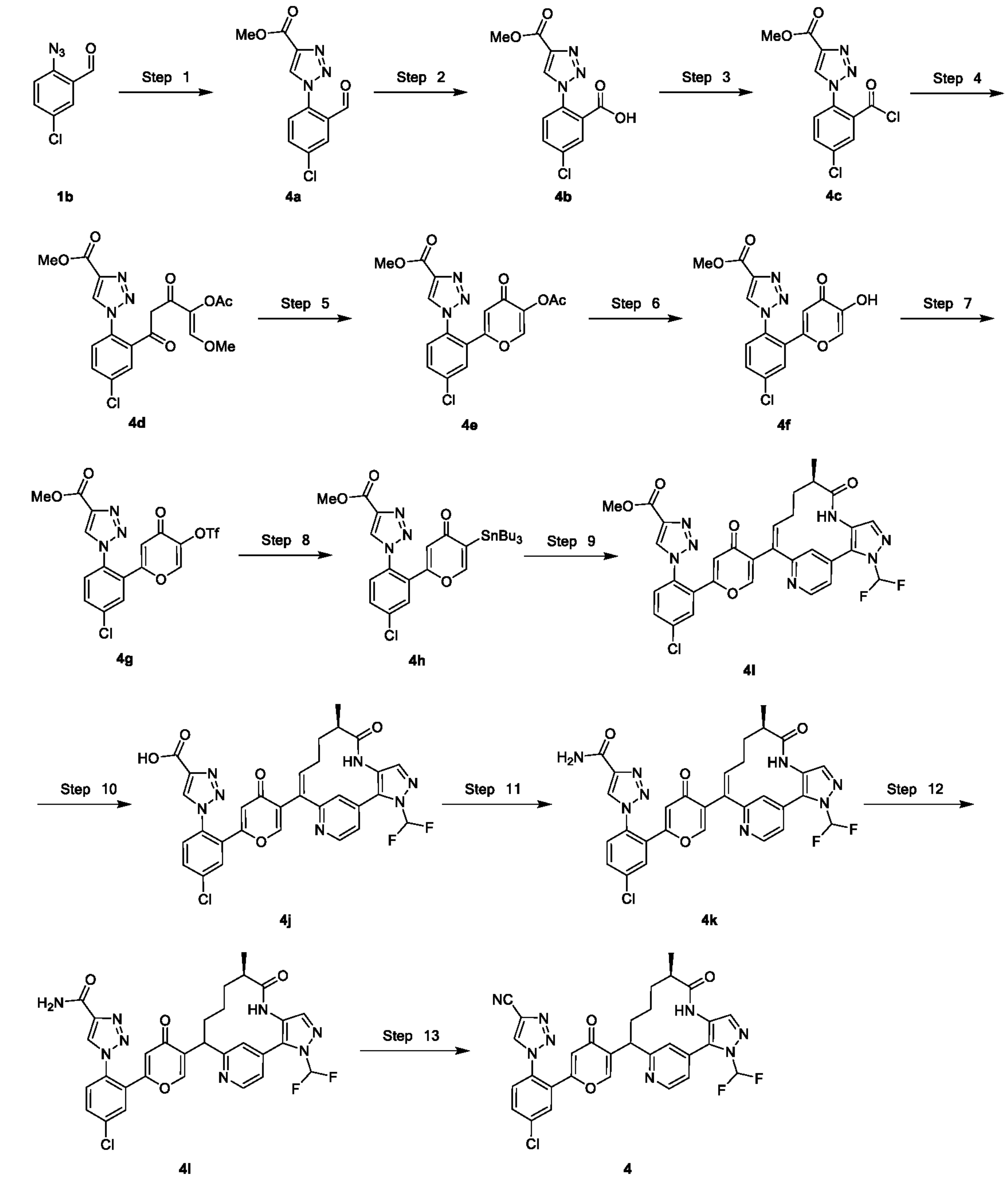


1b 4a 4b 4c 4d 4e 4f 4g 4h 4i 4j 4k 4l 4

Step 1

Compound 1b (10 g, 55.07 mmol), methyl propiolate (6.95 g, 82.61 mmol), triethylamine (111.46 mg, 1.10 mmol) and cuprous iodide (209.77 mg, 1.10 mmol) were dissolved in MeCN (100 mL), after being replaced with nitrogen three times, and then under nitrogen protection, the reaction mixture was stirred at 25 °C and carried out for 12 hours. The reaction mixture was concentrated under reduced pressure, then slurried with petroleum ether (150 mL) for 1 hour, filtered and dried to obtain compound 4a. LCMS m/z (ESI): 266.1 (M+1).

Step 2

Compound 4a (6 g, 22.59 mmol) and sodium dihydrogen phosphate (8.13 g, 67.76 mmol) were dissolved in tert-butanol (50 mL) and water (50 mL), then 2-methyl-2-butene (31.68 g, 451.72 mmol) and sodium chlorite (6.13 g, 67.76 mmol) were added. The reaction mixture was stirred at 25°C and carried out for 1 hour and filtered. The filter cake was washed with water (10 mL × 5), the filtrate was acidified with dilute hydrochloric acid (1 M) to pH 3-4, and then extracted with ethyl acetate (40 mL × 3). The extracts were combined, washed with saturated brine (150 mL), dried

over anhydrous sodium sulfate, and filtered. The filtrate was concentrated under reduced pressure to obtain compound 4b. LCMS m/z (ESI): 282.5 (M+1).

Step 3

Compound 4b (6.2 g, 20.63 mmol) was dissolved in dichloromethane (65 mL), DMF (150.76 mg, 2.06 mmol) was added, then cooled to 0°C and oxalyl chloride (5.24 g, 41.25 mmol) was added. The reaction mixture was slowly heated to 25°C and the reaction was carried out for 3 hours, and then concentrated under reduced pressure to obtain compound 4c.

Step 4

Intermediate A1 (2.61 g, 16.50 mmol) was dissolved in THF (30 mL), cooled to -70 °C and LiHMDS (24.75 mL, 1 M) was added. After the reaction was stirred at -70 °C for 0.5 h, a solution of compound 4c (6.19 g, 20.63 mmol) in THF (60 mL) was added dropwise. The reaction mixture was slowly heated to 25 °C and stirred for 15 hours, poured into aqueous hydrochloric acid solution (150 mL, 2M), ethyl acetate (50 mL) was added, and extracted with ethyl acetate (40 mL×3). The organic phases were combined and washed successively with water (150 mL) and saturated brine (150 mL), dried over anhydrous sodium sulfate, and filtered. The filtrate was concentrated under reduced pressure to obtain compound 4d. LCMS m/z (ESI):422.1 (M+1).

Step 5

Compound 4d (7.9 g, 18.73 mmol) was dissolved in toluene (150 mL), p-toluenesulfonic acid pyridinium salt (470.68 mg, 1.87 mmol) was added, the reaction was heated to 120 °C and stirred for 4 hours, and then concentrated under reduced pressure. The residue was dissolved in ethyl acetate (60 mL), washed with water (100 mL) and saturated brine (100 mL), dried over anhydrous sodium sulfate, and filtered. The filtrate was concentrated under reduced pressure to obtain compound 4e. LCMS m/z (ESI):390.0 (M+1).

Step 6

Compound 4e (7.07 g, 18.14 mmol) was dissolved in MeOH (70 mL), $K_2CO_3$ (2.51 g, 18.14 mmol) was added, the reaction was stirred at 25 °C and carried out for 0.5 hour, and filtered. The filter cake was washed successively with ethyl acetate (10 mL×3) and MeOH (10 mL×3). The washing solution was collected and concentrated under reduced pressure. The residue was dissolved in ethyl acetate (80 mL) and MeOH (10 mL), washed successively with dilute hydrochloric acid (100 mL, 1M), water (100 mL×2) and saturated brine (100 mL×2), dried over anhydrous sodium sulfate, filtered. The filtrate was concentrated under reduced pressure, MeOH (20 mL) was added to the obtained residue, and the mixture was stirred at 25°C for 15 minutes, filtered, and dried to obtain compound 4f. LCMS m/z (ESI): 348.4 (M+1).

Step 7

Compound 4f (2.4 g, 6.90 mmol) was dissolved in DMF (25 mL), triethylamine (838.13 mg, 8.28 mmol) and N-phenylbis(trifluoromethanesulfonyl)imide (2.47 g, 6.90 mmol) were added. The reaction mixture was slowly heated to 25 °C and stirred for 1 hours, poured into aqueous hydrochloric acid solution (100 mL), ethyl acetate (40 mL) was added, and extracted with ethyl acetate (30 mL×2). The organic phases were combined, washed with water (100 mL) and saturated brine (100 mL), and dried over anhydrous sodium sulfate. After filtration, the filtrate was concentrated under reduced pressure to obtain compound 4g.

Step 8

Compound 4g (3.5 g, 7.30 mmol), hexabutylditin (6.35 g, 10.94 mmol), $Pd(OAc)_2$ (163.78 mg, 729.51 μmol), XPhos (521.66 mg, 1.09 mmol) and LiCl (618.54 mg), 14.59 mmol) were dissolved in DMF (35 mL), and after being replaced with nitrogen three times, the reaction mixture was heated to 60 °C and the reaction was carried out for 1 hour under nitrogen protection. The reaction mixture was quenched with water (100 mL), ethyl acetate (40 mL) was added, the aqueous phase was separated and then extracted with ethyl acetate (20 mL×3). The organic phases were combined, washed with saturated brine (100 mL), dried over anhydrous sodium sulfate, and filtered. The filtrate was concentrated under reduced pressure, and the residue was purified by column chromatography (PE:EA=10:1 to 5:1) to obtain compound 4h. LCMS m/z (ESI): 564.1 (M+1).

Step 9

Compound 4h (1.31 g, 2.11 mmol), compound A2 (1.11 g, 1.79 mmol), $Pd(PPh_3)_4$ (243.86 mg, 211.03 μmol), LiCl (357.86 mg, 8.44 mmol) and CuCl (835.68 mg, 8.44 mmol)) were dissolved in DMSO (15 mL), after being replaced by nitrogen for 3 times, and the reaction mixture was heated to 80 °C and the reaction was carried out for 1 hour under nitrogen protection. The reaction mixture was quenched with saturated ammonium chloride solution (60 mL), ethyl acetate (30 mL) was added, and filtered. The organic phase was separated from the filtrate, and the aqueous phase was extracted with ethyl acetate (20 mL×3). The organic phases were combined, washed with saturated brine (80 mL), dried over anhydrous sodium sulfate, and filtered. The filtrate was concentrated under reduced pressure, and the residue was purified by column chromatography (PE:EA=1:1 to DCM:EA=1:1) to obtain compound 4i. LCMS m/z (ESI): 648.3 (M+1).

Step 10

Compound 4i (700 mg, 1.08 mmol) was dissolved in water (2.5 mL), lithium hydroxide monohydrate (135.99 mg, 3.24 mmol) was added, and then the reaction was carried out at 0°C for 1 hour. Ethyl acetate (15 mL) and water (15 mL) were added to the reaction mixture, the aqueous phase was separated, the pH value was adjusted to 4 with dilute hydrochloric acid (1 M), and then extracted with ethyl acetate (15 mL×3). The organic phases were combined, washed with saturated brine (60 mL), dried over anhydrous sodium sulfate, and filtered. The filtrate was concentrated under reduced pressure to obtain compound 4j. LCMS m/z (ESI): 634.1 (M+1).

Step 11

Compound 4j (550 mg, 867.52 μmol) was dissolved in DMF (6 mL), triethylamine and HATU (494.79 mg, 1.30 mmol) were added at 25°C, and after being stirred for 10 minutes, solid ammonium chloride (185.62 mg, 3.47 mmol) was added. The reaction was continuously carried out at 25°C for 10 hours, then quenched by the addition of water (30 mL), then ethyl acetate (15 mL) was added. The aqueous phase was separated and then extracted with ethyl acetate (15 mL×3). The organic phases were combined, washed with saturated brine (40 mL), dried over anhydrous sodium sulfate, filtered, and the filtrate was concentrated under reduced pressure to obtain compound 4k. LCMS m/z (ESI): 633.3 (M+1).

Step 12

Compound 4k (650 mg, 1.03 mmol) was dissolved in THF (20 mL), chloroform (0.1 mL) was added, then Raney nickel (87.98 mg, 1.03 mmol) was added under nitrogen protection. The reaction mixture was replaced with hydrogen several times, then the reaction was stirred and carried out under hydrogen (15 psi) at 25 °C for 0.5 h. The reaction mixture was filtered, and the filtrate was concentrated under reduced pressure to obtain compound 41. LCMS m/z (ESI): 635.1 (M+1).

Step 13

Compound 41 (330 mg, 519.67 μmol) and triethylamine (210.34 mg, 2.08 mmol) were dissolved in THF (8 mL), cooled to 0°C, and trifluoroacetic anhydride (327.44 mg, 1.56 mmol) was added. The reaction mixture was carried out at 0°C for 1 hour and concentrated under reduced pressure. The residue was purified by column chromatography (DCM:EA=1:1), then purified by preparative HPLC (separation column: UniSil 3-100 C18 Ultra (150×25mm×3μm); mobile Phase: [water (0.225% formic acid)-acetonitrile]; acetonitrile%: 35%-65%, 10 min) to give compound 4. LCMS m/z (ESI): 617.0 (M+1). $^1H$ NMR (400MHz, $CD_3OD$): δ 9.11-9.08 (m, 1H), 8.68-8.57 (m, 1H), 8.18-8.13 (m, 1H), 8.01-7.98 (m, 1H), 7.87-7.82 (m, 2H), 7.79 (s, 1H), 7.76 (d, *J*=3.2Hz, 1H), 7.74-7.72 (m, 1H), 7.65-7.62 (m, 1H), 7.57 (s, 1H), 7.50 (s, 1H), 7.41 (d, *J*=4.8Hz, 1H), 6.39-6.35 (m, 1H), 4.34-4.27 (m, 1H), 2.72 (br s, 1H), 2.11-2.03 (m, 1H), 1.94-1.78 (m, 3H), 1.53 (s, 2H), 1.34 (br s, 3H), 1.24 (d, *J*=7.2Hz, 1H), 0.98 (d, *J*=7.0Hz, 3H).

**Example 5: Compound 5-1, Compound 5-2, Compound 5-3 and Compound 5-4**

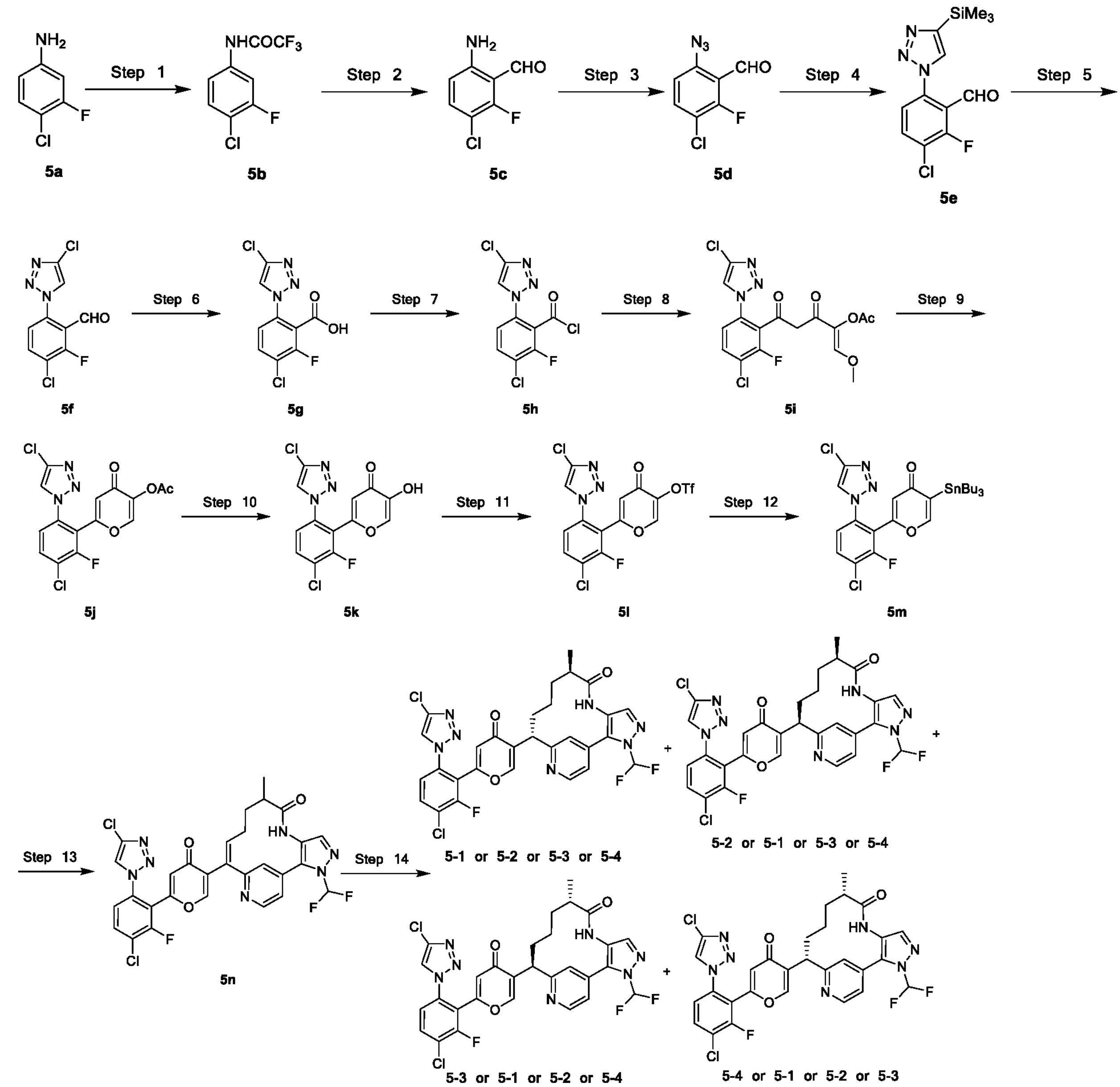


Step 1

Compound 5a (10.0 g, 68.70 mmol) was added to TFAA (30 mL) at 0 °C, then naturally heated to 25 °C, and the reaction was stirred and carried out for 12 hours. The reaction mixture was poured into ice water (200 mL), stirred for 0.5 h, and then filtered. The filter cake was washed with water (30 mL×2), then dissolved in ethyl acetate (150 mL), washed with saturated aqueous $NaHCO_3$ solution (80 mL×2) and saturated brine (80 mL) successively, and dried over anhydrous sodium sulfate, and filtered. The filtrate was concentrated under reduced pressure to obtain compound 5b.

Step 2

Compound 5b (10.0 g, 41.40 mmol) was dissolved in THF (100 mL), cooled to -78 °C, and n-BuLi (34.77 mL, 2.5 M) was added dropwise. After the reaction was stirred and carried out at -78°C for 10 minutes, DMF (9.08 g, 124.19 mmol) was added dropwise, and the reaction was continuously carried out at -78°C for 20 minutes. The reaction mixture was quenched with water (150 mL) and extracted with ethyl acetate (100 mL×2). The organic phases were combined, washed with saturated brine (150 mL), dried over anhydrous sodium sulfate, and filtered. The filtrate was concentrated under reduced pressure to obtain compound 5c.

Step 3

Compound 5c (7.0 g, 40.33 mmol) was dissolved in acetonitrile (100 mL), and isoamyl nitrite (7.09 g, 60.49 mmol) was added at 0 °C. After the reaction was carried out at 0°C for 0.5 hours, trimethylsilyl azide (6.97 g, 60.49 mmol) was added, then slowly heated to 25°C, and the reaction was continuously carried out for 11.5 hours. The reaction mixture was poured into saturated brine (200 mL), and extracted with ethyl acetate (200 mL). After the organic phase was separated, washed with saturated brine (100 mL), dried over anhydrous sodium sulfate, and filtered. The filtrate was concentrated under reduced pressure to obtain compound 5d.

Step 4

Compound 5d (8.0 g, 40.09 mmol) was dissolved in toluene (30 mL) and trimethylsilylacetylene (19.69 g, 200.43 mmol) was added at 25 °C. Then the reaction was heated to 120 °C and carried out for 1.5 hours, concentrated under reduced pressure, and the residue was purified by silica gel column chromatography (PE:EA=20:1 to 3:1) to obtain compound 5e. MS (ESI) m/z: 298.0 (M+1).

Step 5

Compound 5e (4.0 g, 13.43 mmol) was dissolved in acetonitrile (100 mL), NCS (10.76 g, 80.59 mmol) and p-toluenesulfonic acid monohydrate (511.01 mg, 2.69 mmol) were added at 25 °C. The reaction mixture was heated to 50°C for 12 hours and concentrated under reduced pressure. The residue was dissolved in ethyl acetate (100 mL) and water (100 mL), the organic phase was separated, and the aqueous phase was extracted once with ethyl acetate (50 mL). The organic phases were combined, washed successively with water (80 mL×2) and saturated brine (80 mL), and dried over anhydrous sodium sulfate. The filtrate was filtered under reduced pressure and the residue was purified by silica gel column chromatography (PE: EA = 20: 1 to 3: 1) to obtain compound 5f. MS (ESI) m/z: 259.9 (M+1).

Step 6

Compound 5f (50 g, 192.27 mmol) was dissolved in tert-butanol (500 mL) and water (500 mL), then 2-methyl-2-butene (269.69 g, 3.85 mol), sodium dihydrogen phosphate (69.20 g, 576.81 mmol) and sodium chlorite (52.17 g, 576.81 mmol) were added. The reaction mixture was stirred at 20 °C for 2 h, then washed with NaOH solution (500 mL × 2, 1 M). The aqueous phases were combined and acidified to pH 3-4 with hydrochloric acid (2 M), then extracted with ethyl acetate (1000 mL×3). The extracts were combined, washed with saturated brine (1000 mL), dried over anhydrous sodium sulfate, and filtered. The filtrate was concentrated under reduced pressure to obtain compound 5g. LCMS m/z (ESI): 276.0 (M+1).

Step 7

Compound 5g (55 g, 199.24 mmol) was dissolved in dichloromethane (550 mL), DMF (1.46 g, 19.92 mmol) was added, then cooled to 0°C and oxalyl chloride (50.58 g, 398.48 mmol) was added. The reaction mixture was carried out at 15°C for 1 hour, and concentrated under reduced pressure to obtain compound 5h.

Step 8

Intermediate A1 (28.03 g, 177.25 mmol) was dissolved in THF (600 mL), cooled to -78 °C and LiHMDS (196.95 mL, 1 M) was added. After the reaction was stirred at -78 °C for 0.5 h, a solution of compound 5h (58 g, 196.95 mmol) in THF (300 mL) was added dropwise. The reaction mixture was slowly heated to 20 °C and stirred for 12 hours, poured into aqueous hydrochloric acid (500 mL, 2M), and extracted with ethyl acetate (1000 mL×2). The organic phases were combined and washed with saturated brine (1000 mL×3), dried over anhydrous sodium sulfate, and filtered. The filtrate was concentrated under reduced pressure to obtain compound 5i. LCMS m/z (ESI):415.8 (M+1).

Step 9

Compound 5i (80 g, 192.22 mmol) was dissolved in toluene (2000 mL), pyridinium p-toluenesulfonate (4.83 g, 19.22 mmol) was added, the reaction was heated to 120 °C and stirred for 2 hours, and then concentrated under reduced pressure. The residue was dissolved in ethyl acetate (500 mL), washed with saturated brine (200 mL×2), dried over anhydrous sodium sulfate, and filtered. The filtrate was concentrated under reduced pressure to obtain compound 5j. LCMS m/z (ESI):384.1 (M+1).

Step 10

Compound 5j (64 g, 166.60 mmol) was dissolved in MeOH (500 mL), $K_2CO_3$ (27.63 g, 199.92 mmol) was added, the reaction was stirred and carried out at 20 °C for 2 hours, filtered, and the filtrate was concentrated under reduced pressure. Water (50 mL) was added to the residue, then the pH was adjusted to 7 with hydrochloric acid solution (2 M), and extracted with ethyl acetate (100 mL×2). The organic phases were combined, washed with saturated brine (50 mL×3), dried over anhydrous sodium sulfate, and filtered. The filtrate was concentrated under reduced pressure, and the residue was purified by column chromatography (PE:EA=3:1) to obtain compound 5k. LCMS m/z (ESI): 342.0 (M+1).

Step 11

Compound 5k (8.1 g, 23.68 mmol) was dissolved in DMF (80 mL), triethylamine (3.59 g, 35.51 mmol) and N-phenylbis(trifluoromethanesulfonyl)imide (8.46 g, 23.68 mmol) were added. The reaction mixture was stirred at 15°C for 2 hours, water (100 mL) was added, and the mixture was stirred at 20°C for 0.5 hours. After filtration, the filter cake was dried under reduced pressure to obtain compound 51.

Step 12

Compounds 51 (0.5 g, 1.05 mmol), hexabutyldistin (734.04 mg, 1.27 mmol), $Pd(OAc)_2$ (23.67 mg, 105.45 μ mol), XPhos (50.27 mg, 105.45 μ mol) and LiCl (223.50 mg, 5.27 mmol) were dissolved in DMF (10 ml). After being replaced by nitrogen for 3 times, the reaction was heated to 60 °C and carried out for 2 hours under nitrogen protection. The reaction mixture was quenched with water (50 mL), extracted with ethyl acetate (50 mL×3). The organic phases were combined, washed with saturated brine (50 mL×3), dried over anhydrous sodium sulfate, and filtered. The filtrate was concentrated under reduced pressure, and the residue was purified by column chromatography (PE:EA=5:1 to 3:1) to obtain compound 5m. LCMS m/z (ESI): 616.1 (M+1).

Step 13

Compound 5m (0.36 g, 585.22 μmol), compound A3 (360.73 mg, 585.22 μmol), $Pd(PPh_3)_4$ (33.81 mg, 29.26 μmol) and CuI (11.15 mg, 58.52 μmol) were dissolved in DMF (8 mL), after being replaced with nitrogen for 3 times, the reaction was heated to 80°C and carried out for 3 hours under nitrogen protection. Water (50 mL) and ethyl acetate (50 mL) were added to the reaction mixture, the aqueous phase was separated, and extracted with ethyl acetate (50 mL×2). The organic phases were combined, dried over anhydrous sodium sulfate, and filtered. The filtrate was concentrated under reduced pressure, and the residue was purified by column chromatography (DCM:EA=1::0 to 1:1) to obtain compound 5n. LCMS m/z (ESI): 642.1 (M+1).

Step 14

Compound 5n (250.00 mg, 389.16 μmol) was dissolved in MeOH (25 mL), chloroform (3.14 μL) was added, and then Raney nickel (33.34 mg) was added under nitrogen protection. The reaction mixture was replaced with hydrogen several times, then the reaction was stirred and carried out under hydrogen (15 psi) at 20 °C for 5 h. The reaction mixture was filtered, and the filtrate was concentrated under reduced pressure to obtain a crude product mixture. The mixture was separated by SFC (separation column: DAICEL CHIRALPAK AD-H (250mm×30mm, 5μm); mobile phase: ethanol (0.1% ammonia water); ethanol%: 40%-40%, 9min) to obtain the crude product of compound 5-1 ($t_{R=}$1.763 min), the crude product of compound 5-2 ($t_R$ = 1.775 min), a mixture of compounds 5-3 and 5-4.

The crude product of 5-1 was purified by preparative HPLC (separation column: Unisil 3-100 C18 Ultra 150×50mm×3μm; mobile Phase: [water (0.225% formic acid)-acetonitrile]; acetonitrile %: 35%-65%, 10 min) to obtain compound 5-1. LCMS m/z (ESI): 644.1 (M+1). $^1$H NMR (400MHz, $CD_3OD$): δ 8.62 (d, *J*=5.2Hz, 1H), 8.49 (s, 1H), 8.31 (s, 1H), 7.96 (dd, *J*=7.6, 8.4Hz, 1H), 7.76 (s, 1H), 7.80-7.51 (m, 1H), 7.61 (dd, *J*=1.6, 8.8Hz, 1H), 7.59 (s, 1H), 7.40 (d, *J*=4.8Hz, 1H), 6.45 (s, 1H), 4.36-4.26 (m, 1H), 2.37-2.23 (m, 1H), 1.97-1.76 (m, 3H), 1.59-1.43 (m, 1H), 1.39-1.29 (m, 1H), 1.24 (d, *J*=6.8Hz, 3H), 0.75-0.61 (m, 1H).

The crude product of 5-2 was prepared by preparative HPLC (separation column: Unisil 3-100 C18 Ultra 150×50mm×3μm; mobile phase: [water (0.225% formic acid)-acetonitrile]; acetonitrile %: 35%-65%, 10 min) to obtain compound 5-2. LCMS m/z (ESI): 644.1 (M+1). $^1$H NMR (400MHz, $CD_3OD$): δ 8.63 (d, *J*=5.2Hz, 1H), 8.48 (s, 1H), 8.27 (s, 1H), 7.96 (dd, *J*=7.6, 8.4Hz, 1H), 7.74 (s, 1H), 7.80-7.51 (m, 1H), 7.63 (s, 1H), 7.61 (dd, *J*=1.6, 8.8Hz, 1H), 7.41 (d, *J*=4.8Hz, 1H), 6.44 (s, 1H), 4.32 (dd, *J*=4.4, 12.8Hz, 1H), 2.74-2.66 (m, 1H), 2.13-2.02 (m, 1H), 1.97-1.77 (m, 2H), 1.62-1.49 (m, 1H), 1.42-1.27 (m, 1H), 0.97 (d, *J*=7.2Hz, 3H), 0.50-0.35 (m, 1H).

The mixture of 5-3 and 5-4 was separated by SFC (separation column: DAICEL CHIRALPAK AD-H (250mm×30mm, 5μm); mobile phase: ethanol (0.1% ammonia water); ethanol%: 50%-50%, 3.75min) to obtain compound 5-3 ($t_R$=1.855 min) and compound 5-4 ($t_R$=1.937 min).

Compound 5-3: LCMS m/z (ESI): 644.2 (M+1). $^1$H NMR (400MHz, $CD_3OD$): δ 8.62 (d, *J*=5.2Hz, 1H), 8.49 (s, 1H), 8.31 (s, 1H), 7.96 (dd, *J*=7.6, 8.4Hz, 1H), 7.76 (s, 1H), 7.80-7.51 (m, 1H), 7.61 (dd, *J*=1.6, 8.8Hz, 1H), 7.59 (s, 1H), 7.40 (d, *J*=4.8 Hz, 1H), 6.45 (s, 1H), 4.36-4.26 (m, 1H), 2.37-2.23 (m, 1H), 1.97-1.76 (m, 3H), 1.59-1.43 (m, 1H), 1.39-1.29 (m, 1H), 1.24 (d, *J*=6.8Hz, 3H), 0.75-0.61 (m, 1H).

Compound 5-4: LCMS m/z (ESI): 644.2 (M+1). $^1$H NMR (400MHz, $CD_3OD$): δ 8.63 (d, *J*=5.2Hz, 1H), 8.48 (s, 1H), 8.27 (s, 1H), 7.96 (dd, *J*=7.6, 8.4Hz, 1H), 7.74 (s, 1H), 7.80-7.51 (m, 1H), 7.63 (s, 1H), 7.61 (dd, *J*=1.6, 8.8 Hz, 1H), 7.41 (d, *J*=4.8Hz, 1H), 6.44 (s, 1H), 4.32 (dd, *J*=4.4, 12.8Hz, 1H), 2.74-2.66 (m, 1H), 2.13-2.02 (m, 1H), 1.97-1.77 (m, 2H), 1.62-1.49 (m, 1H), 1.42-1.27 (m, 1H), 0.97 (d, *J*=7.2Hz, 3H), 0.50-0.35 (m, 1H).

**Example 6: Compound 6-1 and Compound 6-2**

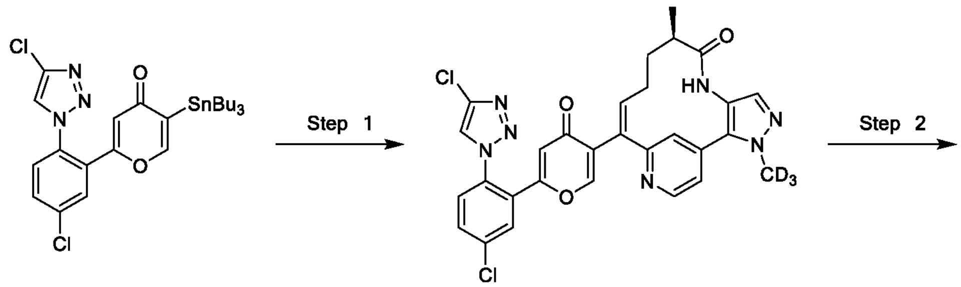


1K 6a

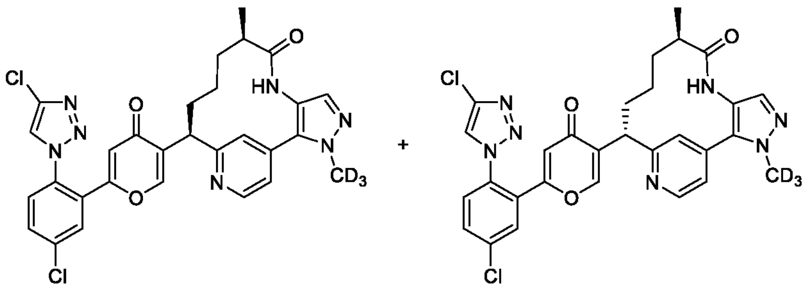

6-1 or 6-2 + 6-2 or 6-1

Step 1

Compound 1K (358.23 mg, 599.89 μmol), compound A4 (0.35 g, 599.89 μmol), $Pd(PPh_3)_4$ (34.66 mg, 29.99 μmol) and CuI (11.42 mg, 59.99 μmol) were dissolved in DMF (6 mL). The reaction was heated to 80 °C and carried out under nitrogen protection for 2 h, quenched with water (10 mL), and extracted with ethyl acetate (5 mL×3). The organic phases were combined, washed with saturated brine (5 mL), dried over anhydrous sodium sulfate, and filtered. The filtrate was concentrated under reduced pressure, and the residue was purified by preparative thin layer chromatography (EA:MeOH=10:1) to obtain compound 6a. LCMS m/z (ESI): 591.1 (M+1).

Step 2

Compound 6a (0.17 g, 287.42 μmol) was dissolved in MeOH (25 mL), chloroform (23.18 μL) was added, and then Raney nickel (0.05 g) was added under nitrogen. The reaction mixture was replaced with hydrogen several times, then the reaction was stirred and carried out under hydrogen (15 psi) at 20 °C for 5 h. The reaction mixture was filtered, the filtrate was concentrated under reduced pressure, and the residue was separated by SFC (separation column: DAICEL CHIRALPAK AD (250 mm×30 mm, 10 μm); mobile phase: isopropanol (0.1% ammonia water); isopropanol %: 40% - 40%, 5.3 min) to obtain the crude product of compound 6-1 ($t_R$= 0.964) and the crude product of compound 6-2 ($t_R$=1.506).

The crude product of compound 6-1 was purified by thin-layer preparative chromatography (EA:MeOH = 10:1)

to obtain compound 6-1. LCMS m/z (ESI): 593.3 (M+1). $^1$HNMR (400MHz, $CD_3OD$): δ 8.60 (d, *J*=5.1Hz, 1H), 8.48 (s, 1H), 8.15 (s, 1H), 7.96 (d, J=2.2Hz, 1H), 7.82 (dd, J=2.3, 8.6Hz, 1H), 7.70 (d, J=8.6Hz, 1H), 7.61 (s, 1H), 7.48 (s, 1H), 7.40 (dd, *J*=1.6, 5.1Hz, 1H), 6.31 (s, 1H), 4.29 (dd, *J*=4.7, 12.2Hz, 1H), 2.78-2.65 (m, 1H), 2.19-2.05 (m, 1H), 1.94-1.76 (m, 2H), 1.63-1.47 (m, 1H), 1.41-1.32 (m, 1H), 0.99 (d, *J*=7.0 Hz, 3H), 0.59-0.37 (m, 1H).

Compound 6-1 was added to acetonitrile (5 mL), a solution of hydrogen chloride in ethyl acetate (4 M, 96.89 μL) was added, then the mixture was stirred at 15°C for 5 minutes, and concentrated under reduced pressure to obtain hydrochloride salt of compound 6-1. LCMS m/z (ESI): 593.4 (M+1). $^1$HNMR (400MHz, $CD_3OD$): δ 8.79 (d, *J*=6.4Hz, 1H), 8.54 (s, 1H), 8.17 (s, 1H), 8.12 (s, 1H), 8.01-8.05 (m, 1H), 7.96 (d, *J*=2.0Hz, 1H), 7.86-7.83 (m, 1H), 7.72 (d, *J*=8.4Hz, 1H), 7.55 (s, 1H), 6.48 (s, 1H), 4.45-4.41 m, 1H), 2.74-2.71 (m, 1H), 2.17-2.16 (m, 1H), 2.03-1.98 (m, 2H), 1.62 (br s, 1H), 1.42 (br s, 1H), 1.02 (d, *J*=7.2 Hz, 3H), 0.69 (br s, 1H).

The crude product of compound 6-2 was purified by thin-layer preparative chromatography (EA:MeOH = 10:1) to obtain compound 6-2. LCMS m/z (ESI): 593.2 (M+1). $^1$HNMR (400MHz, $CD_3OD$): δ 8.62-8.54 (m, 1H), 8.49 (s, 1H), 8.19 (s, 1H), 7.96 (d, *J*=2.3Hz, 1H), 7.82 (dd, *J*=2.3, 8.6Hz, 1H), 7.71 (d, *J*=8.6Hz, 1H), 7.59 (s, 1H), 7.51 (s, 1H), 7.40 (d, *J*=3.9Hz, 1H), 6.32 (s, 1H), 4.36-4.19 (m, 1H), 2.38-2.24 (m, 1H), 1.97-1.72 (m, 3H), 1.50 (dt, *J*=8.2, 11.1Hz, 1H), 1.35-1.29 (m, 1H), 1.24 (d, *J*=6.8 Hz, 3H), 0.82-0.64 (m, 1H).

**Example** 7: **Compound** 7-1 **and Compound** 7-2

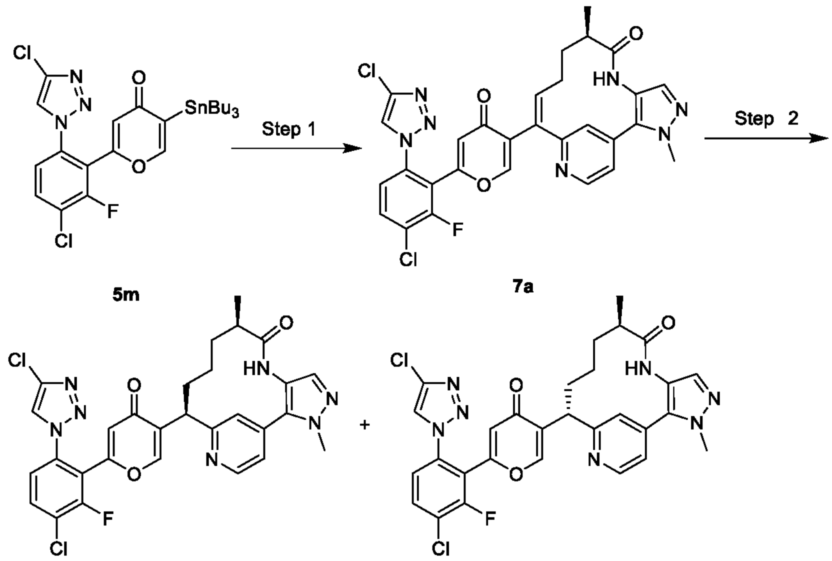


5m

7a

7-1 or 7-2

7-2 or 7-1

Step 1

Compound A5 (0.9 g, 1.55 mmol), compound 5m (953.85 mg, 1.55 mmol), $Pd(PPh_3)_4$ (89.59 mg, 77.53 μmol) and CuI (29.53 mg, 155.06 μmol) were dissolved in DMF (30 mL), after being replaced with nitrogen three times, and then the reaction was heated to 80°C and carried out under nitrogen protection for 3 hours. The reaction was quenched with water (100 mL) and ethyl acetate (100 mL), the organic phase was separated, and the aqueous phase was extracted with ethyl acetate (100 mL×2). The organic phases were combined, washed successively with saturated aqueous sodium carbonate solution (100 mL×2) and saturated brine (100 mL×3), the organic phase was separated, dried over anhydrous sodium sulfate, and filtered. The filtrate was concentrated under reduced pressure, and the residue was purified by column chromatography (EA) to obtain compound 7a. LCMS m/z (ESI): 606.2 (M+1).

Step 2

Compound 7a (0.5 g, 824.49 μmol) was dissolved in MeOH (50 mL), chloroform (98.43 mg, 824.49μmol, 66.51 μL) was added, and then Raney nickel (70.63 mg) was added under nitrogen protection. The reaction mixture was replaced with hydrogen several times, then the reaction was stirred and carried out under hydrogen (15 psi) at 20 °C for 5 h. The reaction mixture was filtered, the filtrate was concentrated under reduced pressure, and the residue was

separated by SFC (separation column: DAICEL CHIRALPAK IE (250 mm×30 mm, 10 μm); mobile phase: ethanol (0.1% ammonia water); ethanol %: 60%-60%, 16 min) to obtain the crude product of compound 7-1 and the crude product of compound 7-2.

The crude product of compound 7-1 was then purified by preparative HPLC (separation column: 3_Phenomenex Luna C18 75×30 mm×3 μm; mobile phase: [water (0.225% formic acid)-acetonitrile]; acetonitrile%: 28%-48%, 8min) to obtain compound 7-1. LCMS m/z (ESI): 608.2 (M+1). $^1$HNMR (400MHz, $CD_3OD$): δ 8.61 (d, *J*=4.8 Hz, 1H), 8.49 (s, 1H), 8.23 (s, 1H), 7.96 (t, *J*=8.2 Hz, 1H), 7.63-7.59 (m, 2H), 7.48 (s, 1H), 7.41 (br d, *J*=3.6 Hz, 1H), 6.44 (s, 1H), 4.30 (br d, *J*=10.8 Hz, 1H), 4.05 (s, 3H), 2.72 (br s, 1H), 2.10 (br s, 1H), 1.95-1.80 (m, 2H), 1.58 (br s, 1H), 1.36 (br s, 1H), 0.99 (br d, *J*=6.8 Hz, 3H), 0.50 (br s, 1H).

The crude product of compound 7-2 was then purified by preparative HPLC (separation column: 3_Phenomenex Luna C18 75×30 mm×3 μm; mobile phase: [water (0.225% formic acid)-acetonitrile]; acetonitrile %: 27%-47%, 8min) to obtain compound 7-2. LCMS m/z (ESI): 608.2 (M+1). $^1$HNMR (400MHz, $CD_3OD$): δ 8.58 (d, *J*=4.8 Hz, 1H), 8.50 (s, 1H), 8.27 (s, 1H), 7.96 (t, *J*=8.0 Hz, 1H), 7.62-7.59 (m, 2H), 7.50 (s, 1H), 7.40 (br d, *J*=4.8 Hz, 1H), 6.44 (s, 1H), 4.28 (br d, *J*=9.2 Hz, 1H), 4.05 (s, 3H), 2.33 (br s, 1H), 1.91-1.80 (m, 3H), 1.52 (br s, 1H), 1.31 (br s, 1H), 1.23 (br d, *J*=6.8 Hz, 3H), 0.74 (br s, 1H).

**Example 8: Compound 8-1 and Compound 8-2**

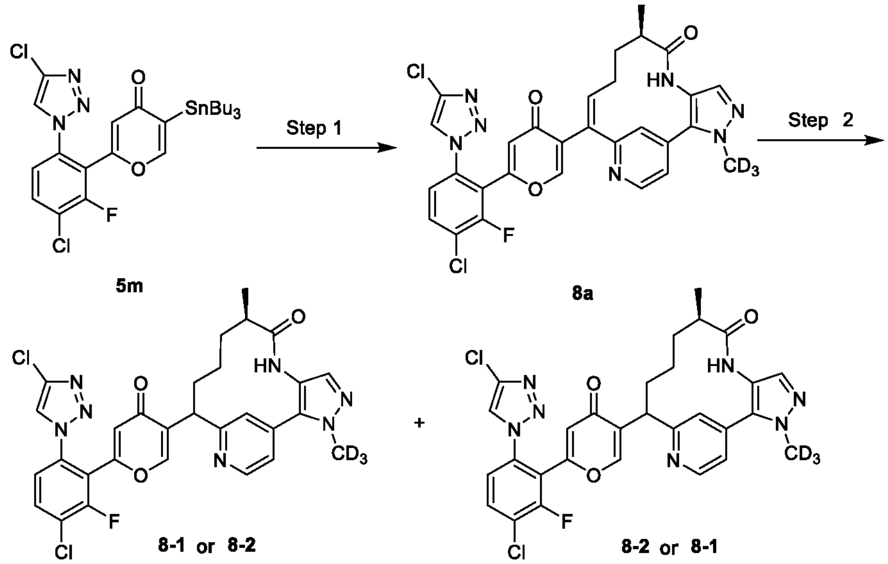


5m

8a

8-1 or 8-2

+

8-2 or 8-1

Step 1

Compound A4 (0.25 g, 428.49 μmol), compound 5m (263.59 mg, 428.49 μmol), $Pd(PPh_3)_4$ (24.76 mg, 21.42 μmol) and CuI (8.16 mg, 42.85 μmol) were dissolved in DMF (5 mL), the reaction was heated to 80°C and carried out for 3 hours under nitrogen protection. The reaction was quenched with water (10 mL) and extracted with ethyl acetate (5 mL×3). The organic phases were combined, washed with saturated brine (5 mL), the organic phase was separated, dried over anhydrous sodium sulfate, and filtered. The filtrate was concentrated under reduced pressure, and the residue was purified by thin layer chromatography (EA:MeOH=10:1) to obtain compound 8a. LCMS m/z (ESI): 609.2 (M+1).

Step 2

Compound 8a (0.1 g, 164.08 μmol) was dissolved in MeOH (25 mL), chloroform (19.59 mg, 164.08 μmol, 13.24 μL) was added, and then Raney nickel (28.54 mg) was added under nitrogen protection. The reaction mixture was replaced with hydrogen several times, then the reaction mixture was stirred and the reaction was carried out under hydrogen (15 psi) at 28 °C for 36 h. The reaction mixture was filtered, the filtrate was concentrated under reduced pressure, and the residue was separated by SFC (separation column: DAICEL CHIRALPAK IE (250 mm×30 mm, 10 μm); mobile phase: ethanol (0.1% ammonia water); ethanol %: 60%-60%, 18.6 min) to obtain the crude product of compound 8-1 ($t_R$ = 6.851 min) and the crude product of compound 8-2 ($t_R$ = 10.543 min).

The crude product of compound 8-1 was purified by thin-layer preparative chromatography (EA:MeOH = 10:1)

to obtain compound 8-1. LCMS m/z (ESI): 611.2 (M+1). $^1$HNMR (400MHz, $CD_3OD$): δ 8.61 (d, *J*=5.1 Hz, 1H), 8.49 (s, 1H), 8.23 (s, 1H), 7.96 (dd, *J*=7.8, 8.6 Hz, 1H), 7.64-7.58 (m, 2H), 7.48 (s, 1H), 7.41 (dd, *J*=1.6, 5.2 Hz, 1H), 6.44 (d, *J*=0.6 Hz, 1H), 4.31 (dd, *J*=4.3, 12.3 Hz, 1H), 2.71 (dt, *J*=3.2, 6.5 Hz, 1H), 2.18-2.05 (m, 1H), 1.96-1.89 (m, 1H), 1.87-1.78 (m, 1H), 1.63-1.48 (m, 1H), 1.42-1.30 (m, 1H), 0.99 (d, *J*=7.0 Hz, 3H), 0.61-0.38 (m, 1H);

The crude product of compound 8-2 was purified by thin layer chromatography (EA:MeOH=10:1) to obtain compound 8-2. LCMS m/z (ESI): 611.2 (M+1). $^1$HNMR (400MHz, $CD_3OD$): δ 8.64-8.57 (m, 1H), 8.49 (s, 1H), 8.27 (s, 1H), 7.96 (dd, *J*=7.8, 8.6 Hz, 1H), 7.65-7.54 (m, 2H), 7.51 (s, 1H), 7.40 (dd, *J*=1.6, 5.2 Hz, 1H), 6.47-6.42 (m, 1H), 4.39-4.16 (m, 1H), 2.39-2.25 (m, 1H), 1.98-1.78 (m, 3H), 1.50 (dt, *J*=8.6, 11.1 Hz, 1H), 1.38-1.29 (m, 1H), 1.24 (d, *J*=7.0 Hz, 3H), 0.83-0.60 (m, 1H).

**Activity test**

**1. Screening test of enzyme inhibitory activity on hFXIa (human** factor **XIa)**

Purpose of the experiment:
Detection of the inhibitory activity of the compounds of the present disclosure on human factor XIa

Experimental Materials:

1) Experimental buffer pH 7.4

100 mM Tris-HCl (Sigma, catalog number: T2663, batch number: SLBG2775 )

200 mM NaCl (Sigma, catalog number: 13423, batch number: SZBB2360V )

0.02% tween20 (Sigma-P1379)

2) Enzymes and Substrates

Enzyme, Human Factor XIa (Human Factor XIa, Abcam, Cat#ab62411): Total amount: 50μg. Dissolved with experimental buffer and packed separately.

Substrate S-2366 (DiaPharma, catalog number: S821090): 25mg

3) Instruments and Consumables

SpectraMax 340PC Multifunctional Microplate Reader (Molecular Devices)

384-well black transparent reaction plate (Corning Cata#3712)

Echo Liquid Workstation (Labcyte)

Echo 384-well polypropylene compound plate (Labcyte-P-05525) was used.

Echo 384 Shallow Pore Polypropylene Compound plate (Labcyte-LP-0200, 2.5-12 μL) was used.

Mutidrop automatic dispenser (Thermo Scientific)

Mutidrop Consumables (Thermo Scientific-24073290)

4) Compound
Compounds to be tested were dissolved in DMSO to form a 10 mM solution and stored in a nitrogen cabinet.

Experimental method:

1) Compound preparation
All test compounds were prepared using Echo, serially diluted and 100 nLof the compound solution was transferred to a 384-well reaction plate. Reference compounds and samples to be tested started at 200 μM (the final concentration of reaction started at 1 μM), 3-fold diluted, 10 points. High signal wells were DMSO and low signal wells were 100

μM reference compound. See Sample Distribution for sample and control distributions.

2) Human Factor XIa Enzyme Preparation
The human factor XIa enzyme was diluted to 0.5 μg/mL using experimental buffer.

3) Substrate S-2306 Preparation
A concentration of 1mM of S-2306 was prepared with experimental buffer solution.

4) 10 μL of 0.5 μg/mL human factor XIa enzyme was added to the reaction plate using Mutidrop automatic separator, and the final concentration was 5ng/well

5) 10 μL of 1mM S-2306 was added to the reaction plate using Mutidrop automatic separator, and the final concentration was 0.5 mM.

6) Centrifugation, 1000rpm, 10s.

7) The reaction plate was placed in SpectraMax 340PC and incubated at 37°C for 10 minutes, and the absorbance was detected at 405nm.

8) Data were analyzed using GraphPad Prism 5.0

$$\% \text{ Inhibition rate} = 100\% \times [1-(\text{sample reading}-\text{low signal average value})/(\text{high signal average value}-\text{low signal average value})]$$

ICso was analyzed using 4-factor linear regression:

Y=Bottom + (Top-Bottom)/(1+10^((LogIC50-X)*HillSlope))

Y was inhibition rate%, and X was the logarithm of sample concentration.

Experimental results:
The data of the $IC_{50}$ of the enzyme inhibitory activity of the compounds of the present disclosure on hFXIa were shown in Table 1 below:

**Table 1 $IC_{50}$ of inhibition of the compounds of the present disclosure on hFXIa**

| Compound | hFXIa $IC_{50}$(nM) |
|---|---|
| 1-1 | 3.34 |
| 2-1 | 5.21 |
| 3-1 | 9.2 |
| 4 | 2.28 |
| 5-1 | 9.56 |
| 5-2 | 3.94 |
| 6-1 | 6.1 |
| 7-1 | 2.9 |
| 8-1 | 3.5 |

Conclusion: The compounds of the present disclosure had good inhibitory activity on the enzyme of human factor XIa.

**2. Determination of aPTT (activated partial thromboplastin time) in human plasma in vitro**

Purpose of the experiment:
Detection of the anticoagulant effect of the compounds of the present disclosure on human plasma *in vitro*

Materials and reagents:

1) Coagulation quality control plasma: produced by TECO, Germany, batch number: 093B-J186A

2) aPTT test solution: produced by TECO, Germany, batch number: 20002745; CaCl2: produced by TECO, Germany, batch number: 031N-J073A; DMSO: Sangon Bioengineering (Shanghai) Co., Ltd., batch number: BB02BA0001

3) Instrument: Germany Meichuang MC-4000 blood coagulation instrument

Detection steps:

1) Sample solution preparation: 10 mM compound stock solution was prepared with DMSO, and then serially diluted with DMSO as follows: 1000, 2500, 625, 156.25, 39.06, 9.76, 2.44 μM; then series of concentrations were diluted with tris(hydroxymethylaminomethane) buffer (containing 5% Tween 80) 10 times for use, and the drug to plasma volume ratio was 1:9 during detection.

2) Positive control: Enoxaparin (GSK, batch number 4SH69) was diluted with pH7.4, 0.02 M trihydroxymethylaminomethane buffer (containing 5% Tween 80) at a gradient of 160, 80, 40, 20 and 10 μg/ml for later use.

3) Blank control: pH 7.4, 0.02 M trihydroxymethyl aminomethane buffer (containing 5% Tween 80 and 10% DMSO).

4) Test detection: According to the instructions of the kit, the sample solution to be tested or the reference solution were added into the 37 °C preheated colorimetric cup, preheated at 37 °C for 2 min, aPTT reagent was added, incubated at 37 °C for 3 min, 0.02 M $CaCl_2$ (preheated at 37 °C) was added, and the coagulation time was recorded.

**Table 2 Effect of compounds of the present disclosure on aPTT**

| Compound | $aPTT_{2x}$ (μM) |
|---|---|
| Enoxaparin | 4.61 |
| 6-1 | 2.69 |
| 8-1 | 4.94 |

Conclusion: The compounds of the present invention had an obvious anticoagulant effect on human plasma *in vitro.*

**3. *In vivo* pharmacokinetic evaluation in rats**

Purpose of the experiment:
Detection of pharmacokinetic parameters of the compounds of the present disclosure in rats.

Experimental scheme:

1) Experimental drug: compound 6-1;

2) Experimental animals: 4 male SD rats aged 7-9 weeks were randomly divided into 2 groups with 2 rats in each group;

3) Drug preparation: an appropriate amount of drug was weighed, and dissolved in a mixed solvent of DMAC: slolutol:water=10:10:80, and two kinds of solution 0.2mg/mL and 0.5mg/mL were prepared;

Experimental operation:

Animals in group 1 were given the drug at a single dose of 0.5 mg/kg at a concentration of 0.2 mg/mL by tail vein injection, and animals in group 2 were given compound at a dose of 3 mg/kg at a concentration of 0.5 mg/mL by gavage. Plasma samples were collected from animals at 0.0833 (tail vein injection group only), 0.25, 0.5, 1, 2, 4, 6, 8 and 24 hours after administration. The LC-MS/MS method was used to determine the drug concentration in the plasma samples, and the kinetic parameters of the tested drugs were obtained as shown in Table 3:

**Table 3**

| | | | | | | |
|---|---|---|---|---|---|---|
| **Compou nd 6-1** | **Tail vein injecti on group** | **Clearance rate** | **Initial concentrat ion** | **Distributi on volume** | **Half-life** | **Curv e area** |
| | | **Cl (mL/Kg/mi n)** | **$C_0$ (nM)** | **Vd (L/Kg)** | **$T_{1/2}$ (h)** | **AUC (nM· h)** |
| | | 1.84 | 16148 | 0.11 | 0.972 | 7929 |
| | **Gavag e group** | **Peak concentrat** ion | **Peak time** | **Curve area** | **Bioavailabi lity** | - |
| | | **$C_{max}$ (nM)** | **$T_{max}$(h)** | **AUC (nM·h)** | **F (%)** | -- |
| | | 3755 | 0.25 | 5105 | 10.8 | - |

Conclusion: The compounds of the present disclosure had good pharmacokinetic properties in rats.

**4. In vivo pharmacokinetic evaluation in cynomolgus monkeys**

Purpose of the experiment:
Detection of pharmacokinetic parameters of the compounds of the present disclosure in cynomolgus monkeys
Experimental scheme:

Experimental drug: compound 6-1 (hydrochloride);

Experimental animals: 4 male cynomolgus monkeys were randomly divided into 2 groups, 2 in each group;

Drug preparation: an appropriate amount of drug was weighed, and dissolved in a mixed solvent of DMAC: slolutol:water=10:10:80, and two kinds of solution 1 mg/mL and 2 mg/mL were prepared;

Experimental operation:
Animals in group 1 were given the drug at a single dose of 1 mg/kg at a concentration of 1 mg/mL by tail vein injection, and animals in group 2 were given compound at a dose of 10 mg/kg at a concentration of 2 mg/mL by gavage. Plasma samples were collected from animals at 0.0833 (tail vein injection group only), 0.25, 0.5, 1, 2, 4, 6, 8 and 24 hours after administration. The drug concentration in the plasma samples was determined by LC-MS/MS method, and the kinetic parameters of the tested drug were obtained as shown in Table 4:

**Table 4**

| | | | | | | |
|---|---|---|---|---|---|---|
| **Compou nd 6-1** | **Tail vein injecti on group** | **Clearance rate** | **Initial concentrat ion** | **Distributi on volume** | **Half-life** | **Curv e area** |
| | | **Cl (mL/Kg/mi n)** | **$C_0$ (nM)** | **Vd (L/Kg)** | **$T_{1/2}$ (h)** | **AUC (nM· h)** |
| | | 8.6 | 4530 | 0.619 | 1.05 | 3250 |
| | **Gavag e group** | **Peak concentrat ion** | **Peak time** | **Curve area** | **Bioavailabi lity** | -- |
| | | **$C_{max}$ (nM)** | **$T_{max}$(h)** | **AUC (nM·h)** | **F (%)** | -- |
| | | 2230 | 3.00 | 11953 | 36.8 | -- |

Conclusion: The compounds of the present disclosure had good pharmacokinetic properties in cynomolgus monkeys.

## Claims

1. A compound represented by formula (I), an isomer thereof or a pharmaceutically acceptable salt thereof,

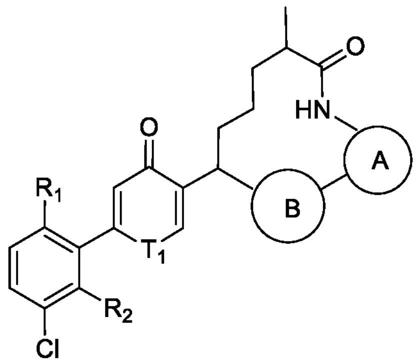

(I),

wherein, $R_1$ is triazolyl or tetrazolyl, wherein the triazolyl and tetrazolyl are optionally substituted by Ra;
Ra is F, Cl, Br, I, CN, $C_{1-3}$ alkyl, $C_{1-3}$ alkoxy or $C_{3-4}$ cycloalkyl;
$R_2$ is H or F;
$T_1$ is -O- or -N($R_b$)-;
$R_b$ is H, $-CH_3$, $-CH_2CH_3$, $-CH_2CH_2CH_3$ or $-CH(CH_3)_2$;
ring A is phenyl optionally substituted by 1, 2 or 3 $R_c$ or pyrazolyl optionally substituted by 1 or 2 $R_d$;
$R_c$ is H, F, Cl, Br, I, $C_{1-3}$ alkyl or $C_{1-3}$ alkoxy;
$R_d$ is H or $C_{1-3}$ alkyl, wherein the $C_{1-3}$ alkyl is optionally substituted by 1, 2 or 3 substituents independently selected from F, Cl, Br, I, D, $C_{1-3}$ alkoxy and $C_{3-4}$ cycloalkyl;
ring B is

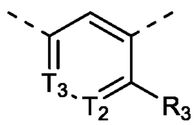

;

$T_2$ is N or $CR_4$;
$T_3$ is N or $CR_5$;
$R_3$ is H, F, Cl, Br, I, $C_{1-3}$ alkyl, $C_{1-3}$ haloalkyl or $C_{1-3}$ alkoxy;
$R_4$ and $R_5$ are each independently H, F, Cl, Br, I, $C_{1-3}$ alkyl, $C_{1-3}$ haloalkyl, or $C_{1-3}$ alkoxy.

2. The compound as defined in claim 1, the isomer thereof or the pharmaceutically acceptable salt thereof , wherein, the compound has the structure represented by formula (I-1):

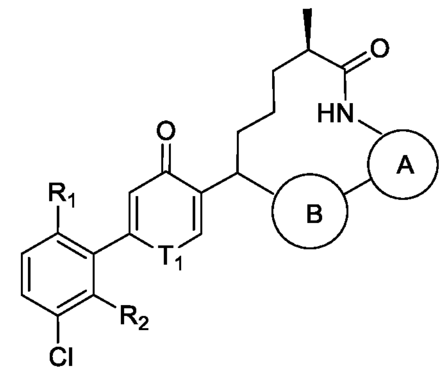

(I-1),

wherein, $R_1$, $R_2$, $T_1$, ring A and ring B are as defined in claim 1.

**3.** The compound as defined in claim 1 or 2, the isomer thereof or the pharmaceutically acceptable salt thereof, wherein, the ring B is

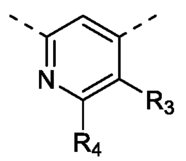
,
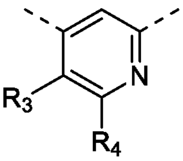
,
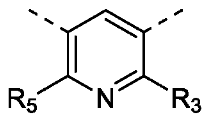
,
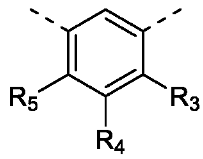
or
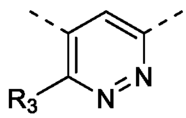
.

**4.** The compound as defined in claim 1 or 2 the isomer thereof or the pharmaceutically acceptable salt thereof, wherein, the ring B is

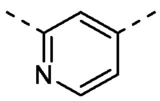
,
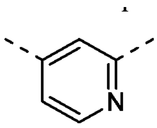
,
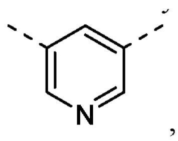
,
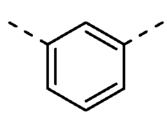
or
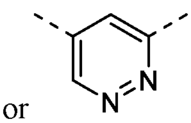
.

**5.** The compound as defined in claim 1, the isomer thereof or the pharmaceutically acceptable salt thereof, wherein, the compound has the structure represented by formula (1-2), (1-3), (1-4) or (1-5):

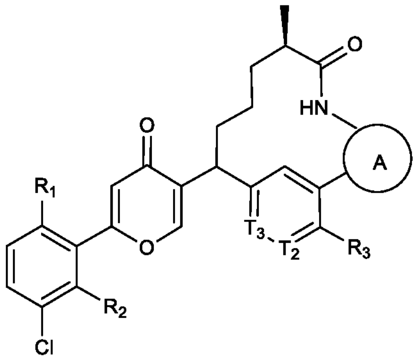

(I-2),

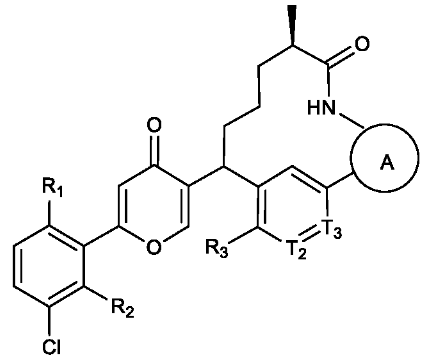

(I-3),

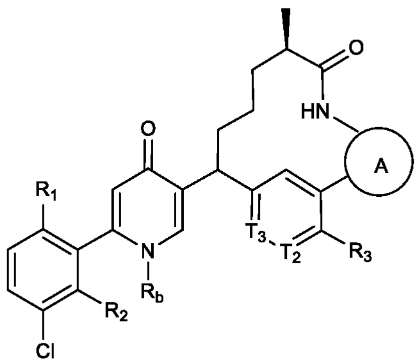

(I-4) or

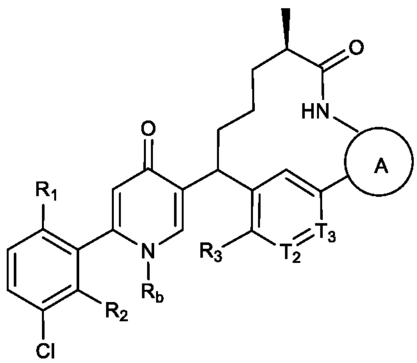

(I-5),

wherein, ring A, $R_1$, $R_2$, $R_3$, $T_2$, $T_3$ and $R_b$ are as defined in claim 1.

**6.** The compound as defined in claim 5, the isomer thereof or the pharmaceutically acceptable salt thereof, wherein, the compound has the structure represented by formula (1-6), (1-7), (1-8) or (1-9):

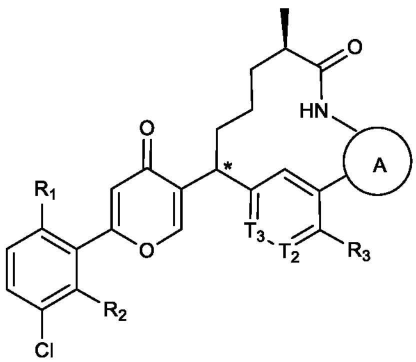

(I-6),

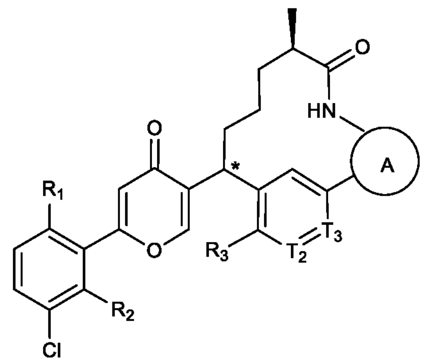

(I-7),

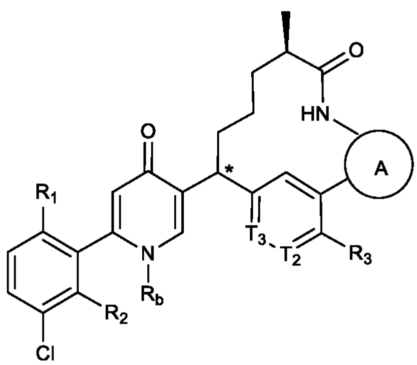

(I-8) or

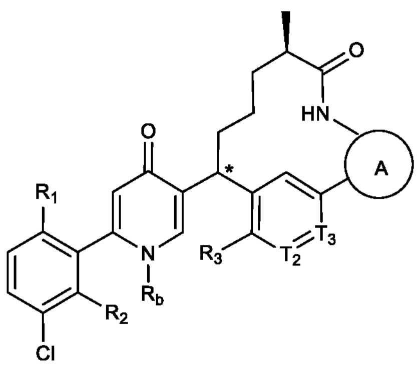

(I-9),

wherein, the carbon atom with "*" is a chiral carbon atom, existing in the form of (R) or (S) single enantiomer or

enriched in one enantiomer; ring A, $R_1$, $R_2$, $R_3$, $R_4$ and $R_5$ are as defined in claim 5.

**7.** The compound as defined in claim 6, the isomer thereof or the pharmaceutically acceptable salt thereof, wherein, the compound has the structure represented by formula (I-10), (1-11), (1-12) or (1-13):

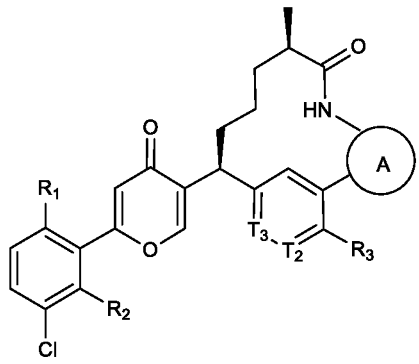

(I-10),

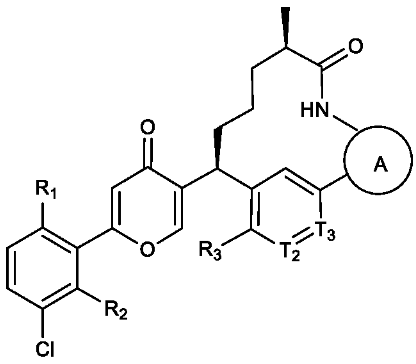

(I-11),

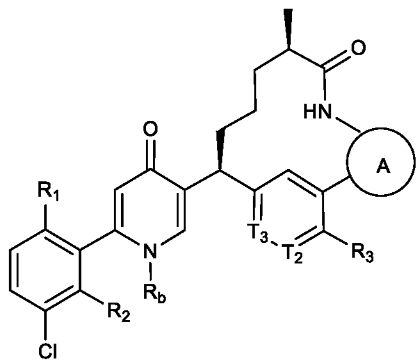

(I-12) or

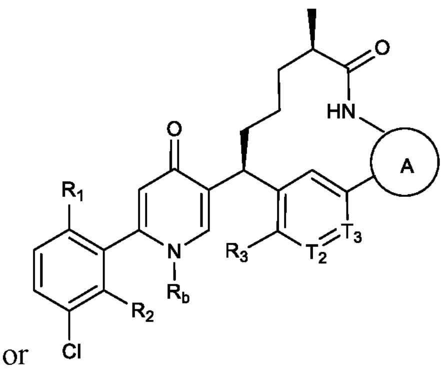

(I-13),

wherein, ring A, $R_1$, $R_2$, $R_3$, $T_2$ and $T_3$ are as defined in claim 6.

**8.** The compound as defined in claim 1, the isomer thereof or the pharmaceutically acceptable salt thereof, wherein, the $R_c$ is H, F, Cl or $-CH_3$.

**9.** The compound as defined in claim 1, the isomer thereof or the pharmaceutically acceptable salt thereof, wherein, the $R_d$ is

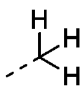, 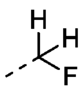, 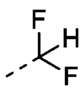, 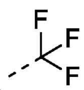 or 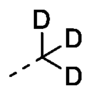.

**10.** The compound as defined in any one of claims 1, 2 or 5-9, the isomer thereof or the pharmaceutically acceptable salt thereof, wherein, the ring A is

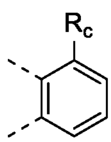, 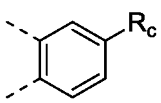,

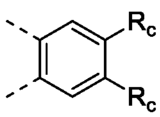 or 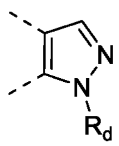.

**11.** The compound as defined in claim 10, the isomer thereof or the pharmaceutically acceptable salt thereof, wherein, the ring A is

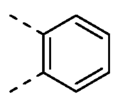 , 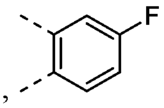 , 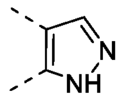 ,

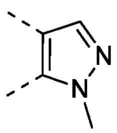 , 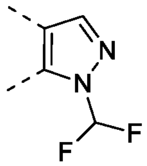 or 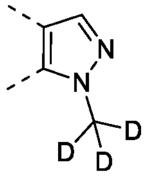 .

**12.** The compound as defined in claim 1, the isomer thereof or the pharmaceutically acceptable salt thereof, wherein, the compound has the structure represented by any one of formulas (I-14)-(I-21):

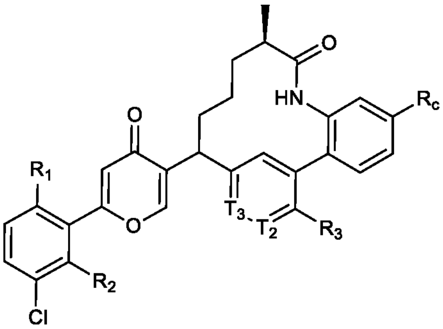 (I-14), 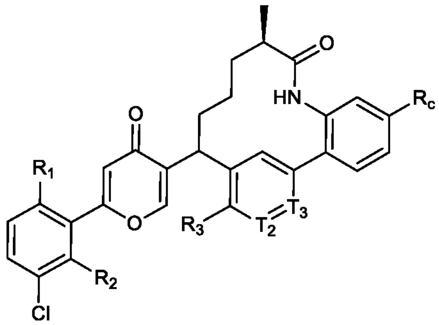 (I-15),

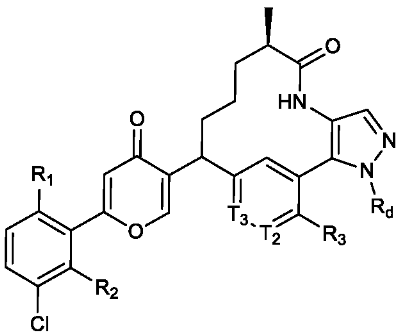 (I-16), 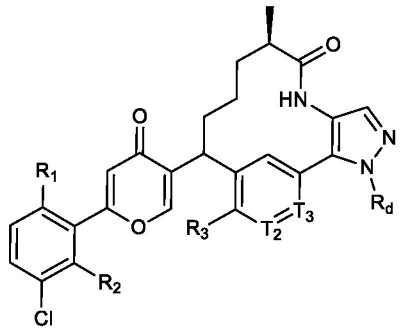 (I-17),

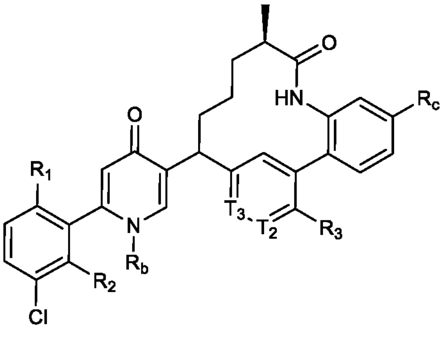 (I-18), 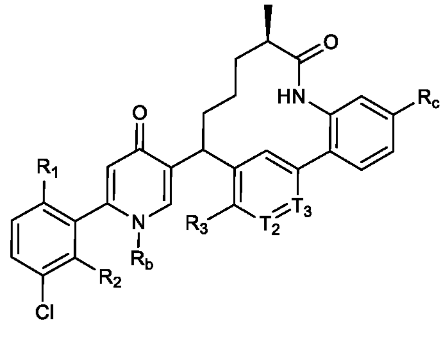 (I-19),

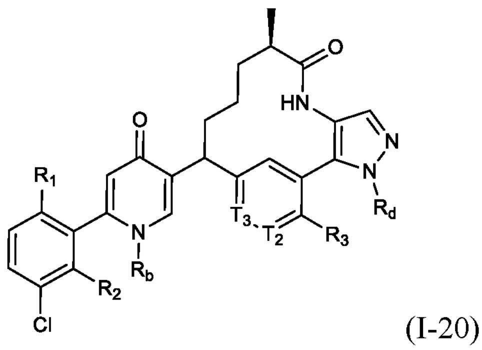

(I-20) or

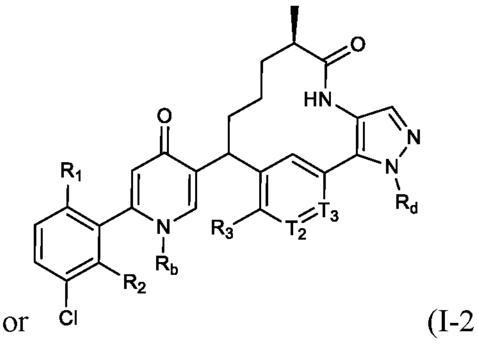

(I-21),

wherein, $R_1$, $R_2$, $R_3$, $T_2$, $T_3$, $R_b$, $R_c$ and $R_d$ are as defined in claim 1.

**13.** The compound as defined in claim 12, the isomer thereof or the pharmaceutically acceptable salt thereof, wherein, the compound has the structure represented by any one of formulas (I-22)-(I-29):

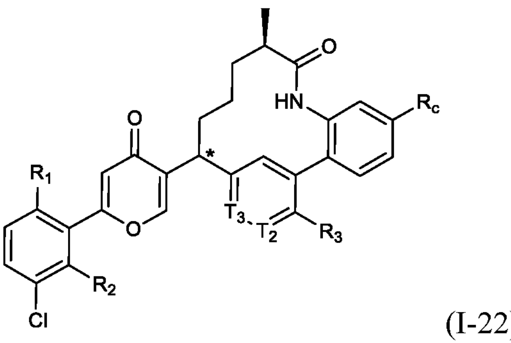

(I-22),

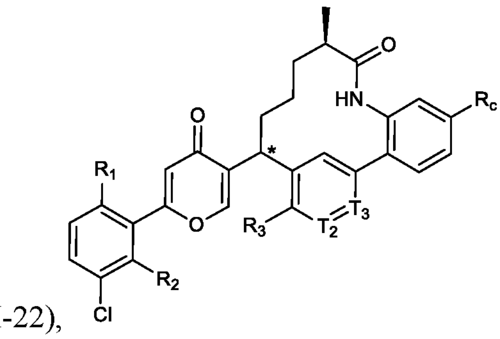

(I-23),

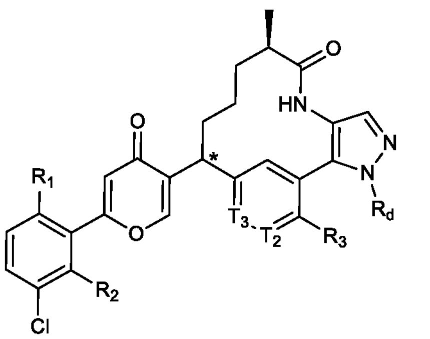

(I-24),

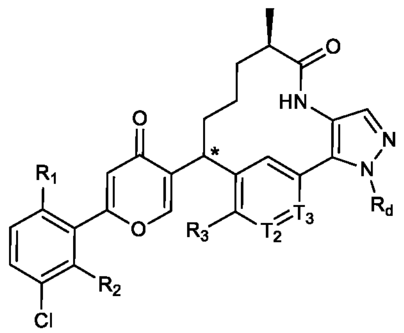

(I-25),

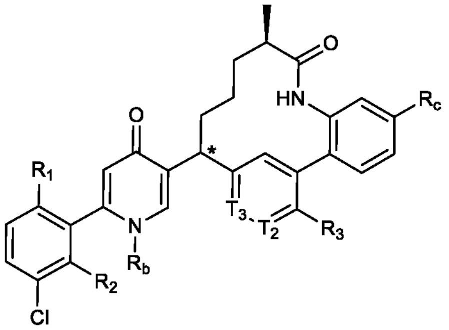

(I-26),

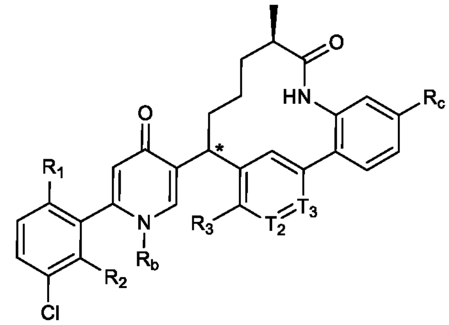

(I-27),

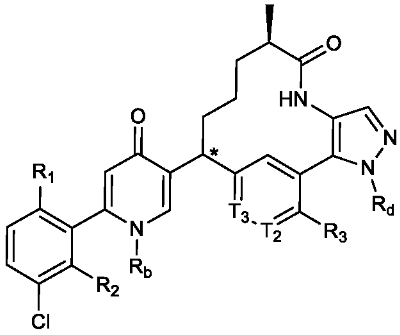 (I-28) or 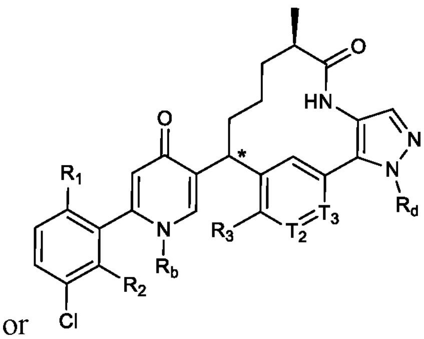 (I-29),

wherein, the carbon atom with "*" is a chiral carbon atom, existing in the form of (R) or (S) single enantiomer or enriched in one enantiomer; $R_1$, $R_2$, $R_3$, $T_2$, $T_3$, $R_b$, $R_c$ and $R_d$ are as defined in claim 12.

**14.** The compound as defined in claim 13, the isomer thereof or the pharmaceutically acceptable salt thereof, wherein, the compound has the structure represented by any one of formulas (I-30)-(I-37):

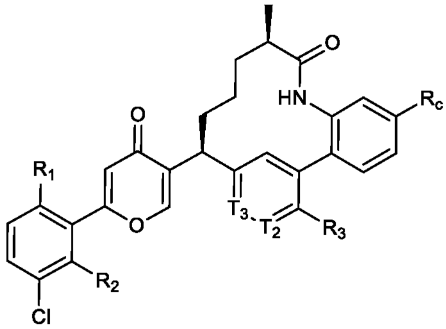 (I-30), 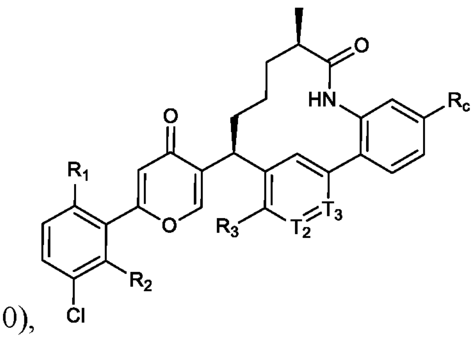 (I-31),

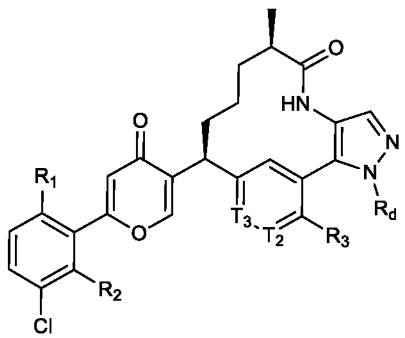 (I-32), 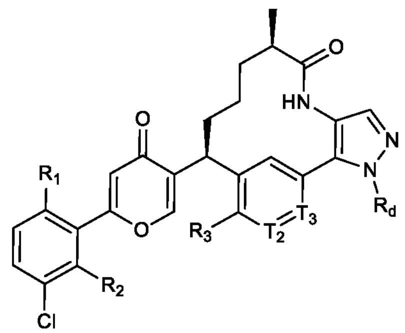 (I-33),

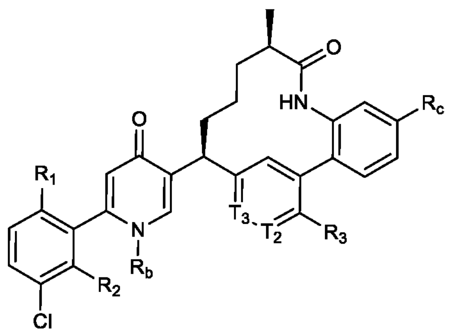 (I-34), 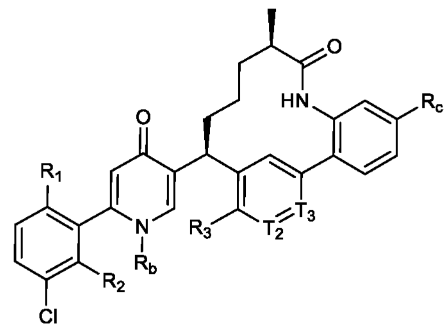 (I-35),

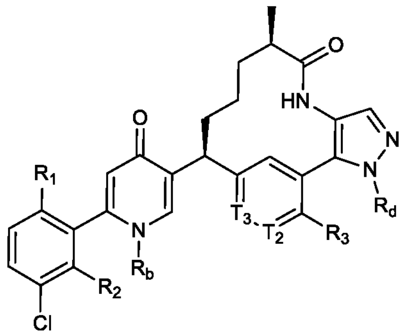 (I-36) or 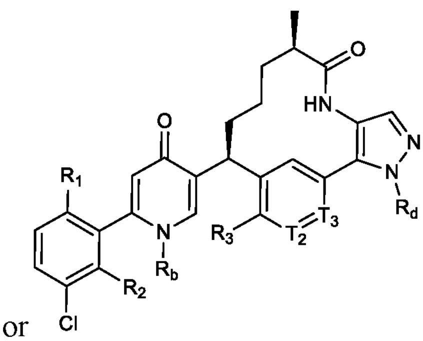 (I-37),

wherein, $R_1$, $R_2$, $R_3$, $T_2$, $T_3$, $R_b$, $R_c$ and $R_d$ are as defined in claim 13.

**15.** The compound as defined in claim 14, the isomer thereof or the pharmaceutically acceptable salt thereof, wherein, the compound has the structure represented by formula (I-38) or (1-39):

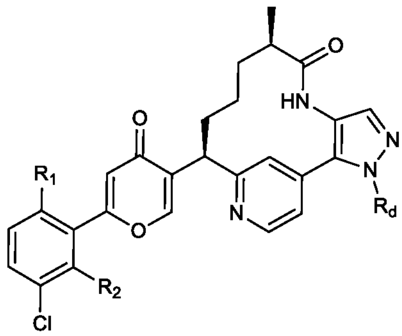 (I-38) or 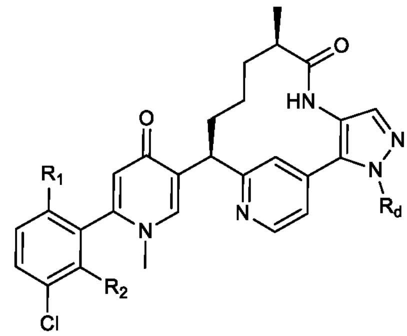 (I-39),

wherein, $R_1$, $R_2$ and $R_d$ are as defined in claim 14.

**16.** The compound as defined in claim 1, the isomer thereof or the pharmaceutically acceptable salt thereof, wherein, the Ra is F, Cl, CN, $-CH_3$ or

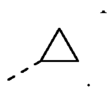.

**17.** The compound as defined in any one of claims 1, 2, 5-7 or 12-16, the isomer thereof or the pharmaceutically acceptable salt thereof, wherein, the $R_1$ is

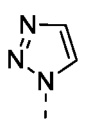, 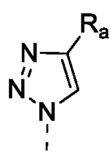,

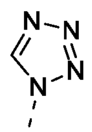 or 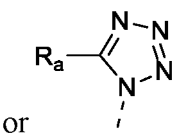.

**18.** The compound as defined in claim 17, the isomer thereof or the pharmaceutically acceptable salt thereof, wherein, the $R_1$ is

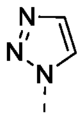, 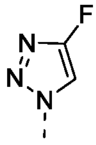, 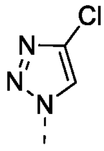, 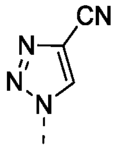 or 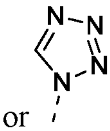.

**19.** The compound as defined in any one of claims 1, 2, 5-7 or 12-16, the isomer thereof or the pharmaceutically acceptable salt thereof, wherein, the $R_3$ is H, F, Cl, Br,

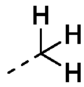,

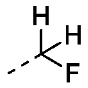, 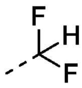, 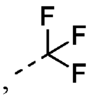 or 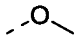.

**20.** The compound as defined in claim 1, the isomer thereof or the pharmaceutically acceptable salt thereof, wherein, the $R_4$ and $R_5$ are each independently H, F, Cl, Br,

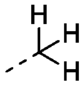, 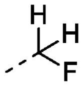, 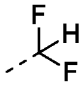, 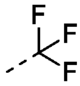 or 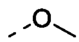.

**21.** The compound as defined in any one of claims 1, 2, 5-7, 12-16 or 20, the isomer thereof or the pharmaceutically acceptable salt thereof, wherein, the $T_2$ is N, CH or CF.

**22.** The compound as defined in any one of claims 1, 2 5-7, 12-16 or 20, the isomer thereof or the pharmaceutically acceptable salt thereof, wherein, the $T_3$ is N, CH or CF.

**23.** A compound of the following formula, an isomer thereof or a pharmaceutically acceptable salt thereof,

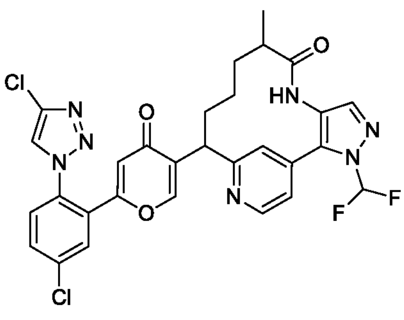,

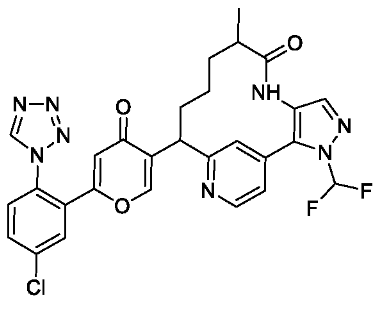,

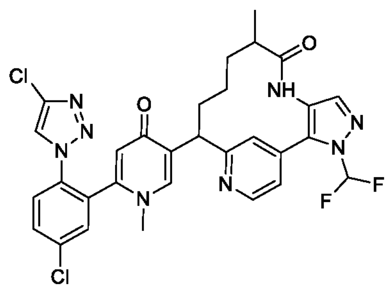 , 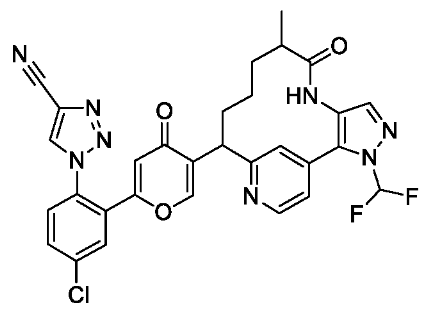 ,

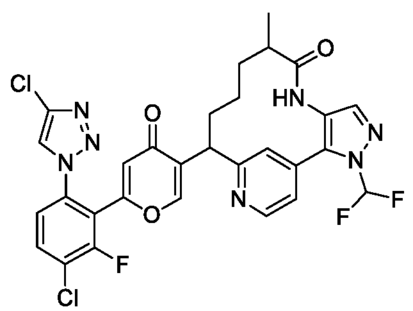 , 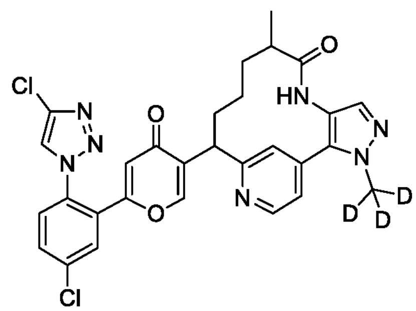 ,

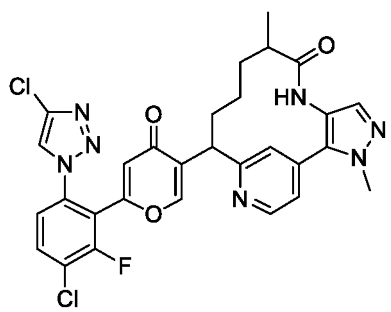 or 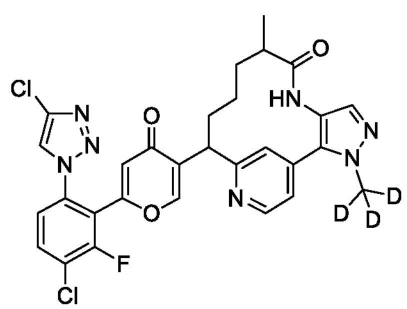 .

**24.** A compound of the following formula, an isomer thereof or a pharmaceutically acceptable salt thereof,

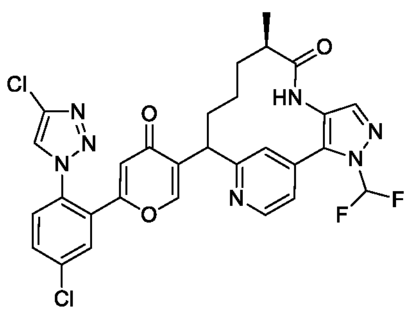 , 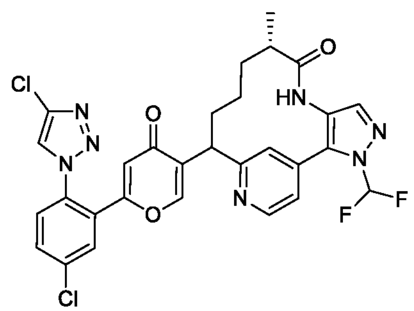 ,

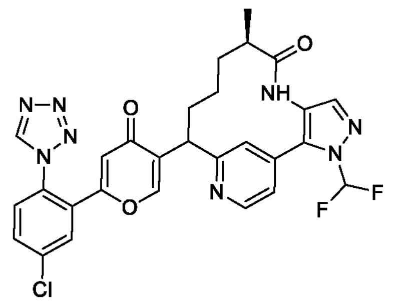

,

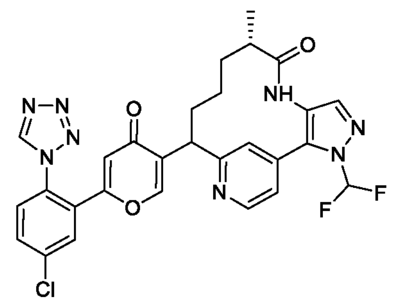

,

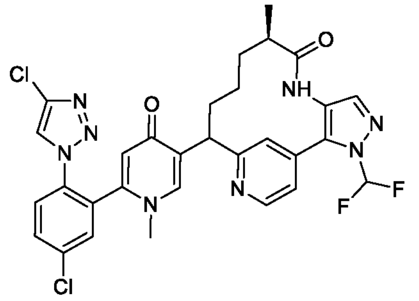

,

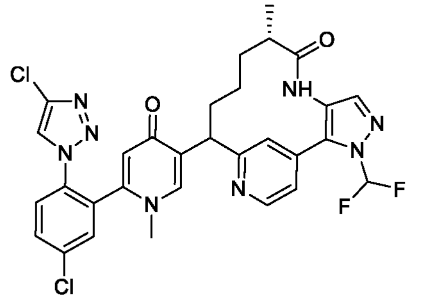

,

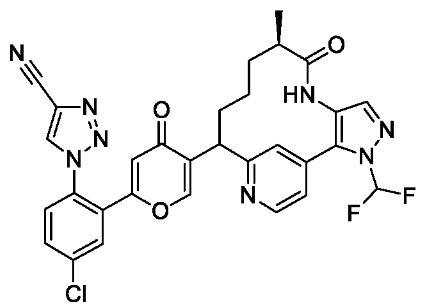

,

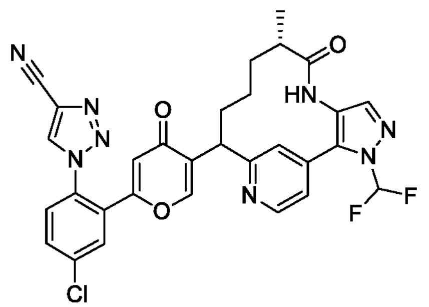

,

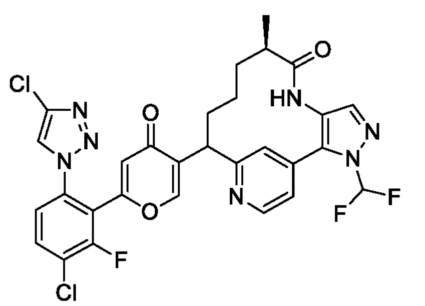

,

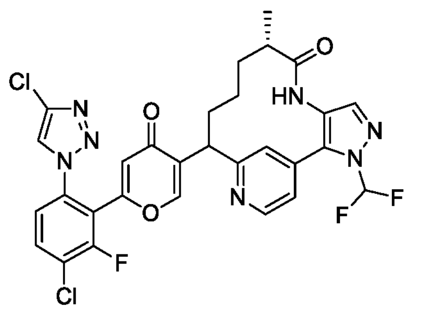

,

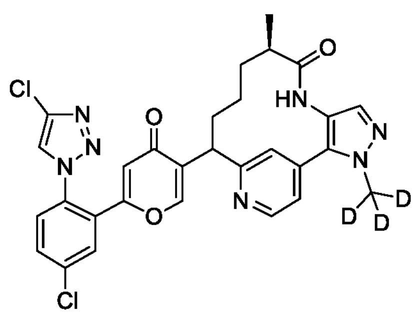

,

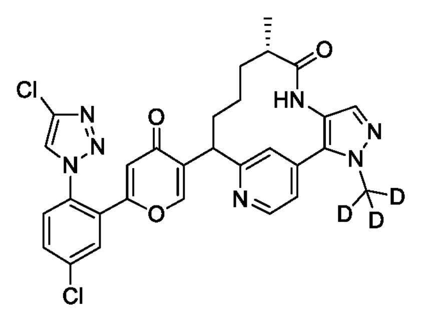

,

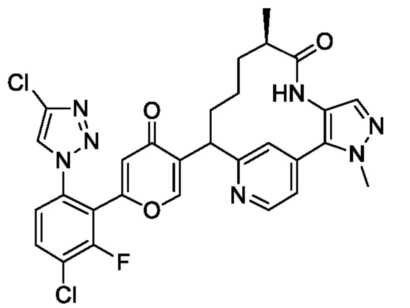

,

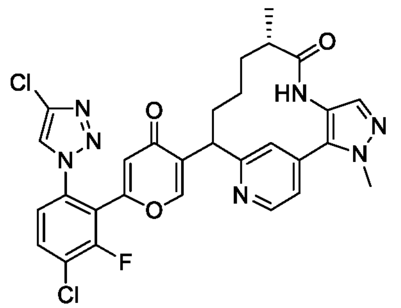

,

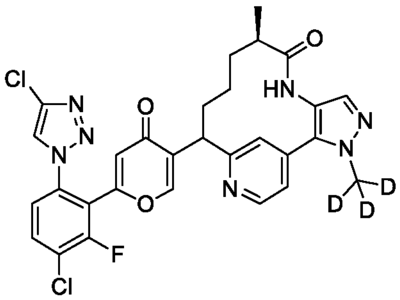

or

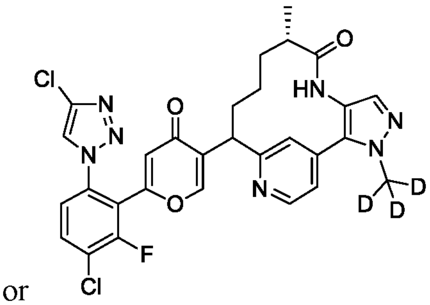

.

**25.** A compound of the following formula, an isomer thereof or a pharmaceutically acceptable salt thereof,

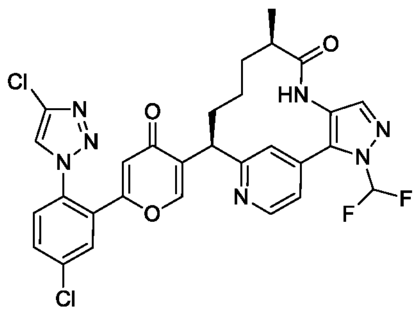

,

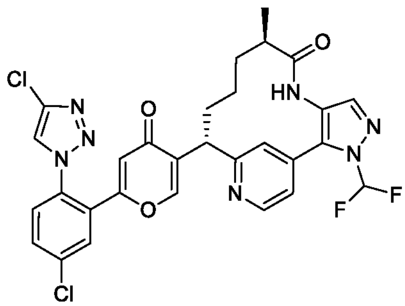

,

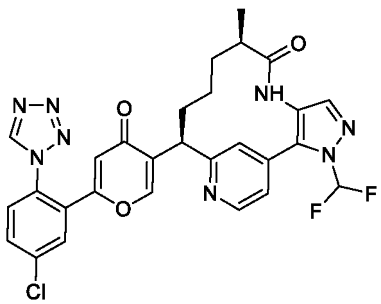

,

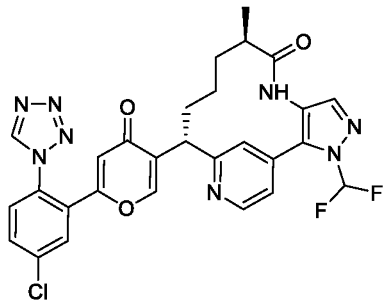

,

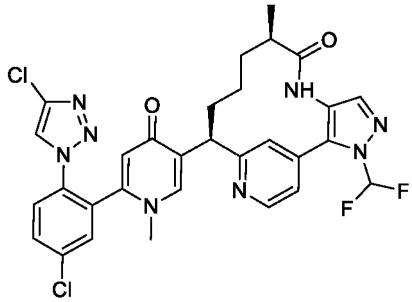

,

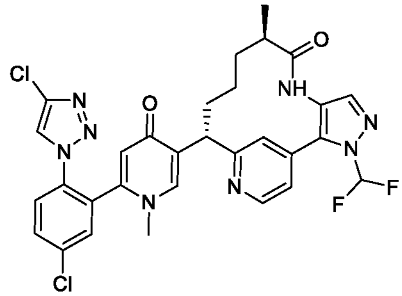

,

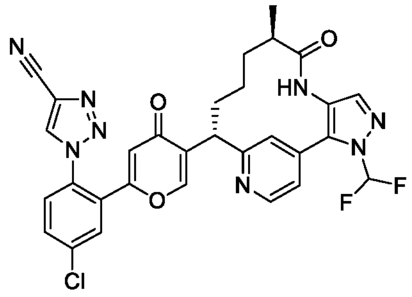
,

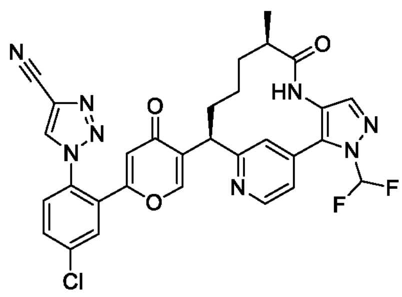
,

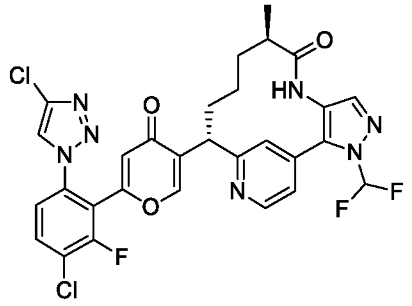
,

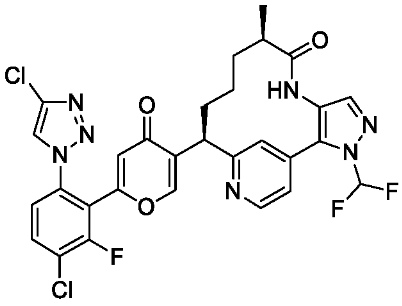
,

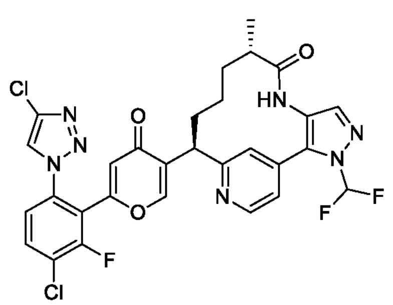
,

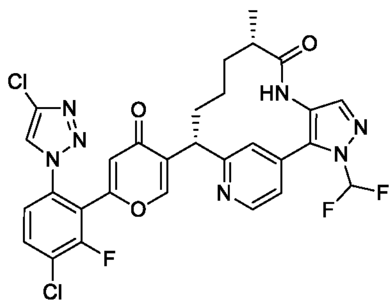
,

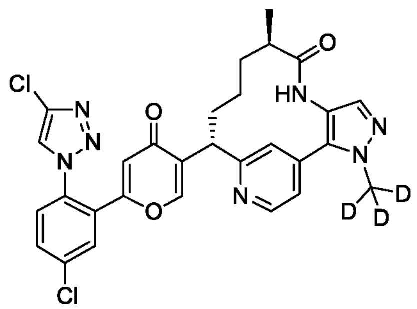
,

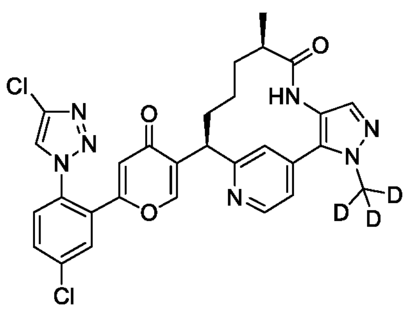
,

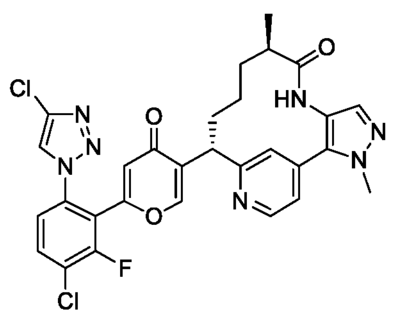
,

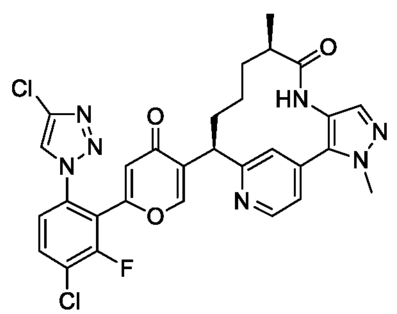
,

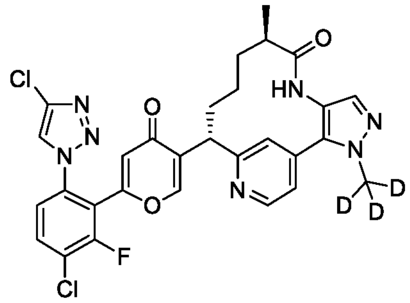

or

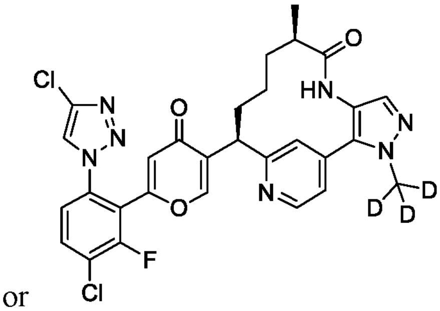

.

**26.** A pharmaceutical composition comprising a therapeutically effective amount of the compound in any one claims 1-25, the isomer thereof or the pharmaceutically acceptable salt thereof as defined, and a pharmaceutically acceptable carrier.

**27.** A use of the compound as defined in any one of claims 1-25, the isomer thereof or the pharmaceutically acceptable salt thereof and the pharmaceutical composition as defined in claim 26 in the manufacture of a medicament of factor XIa inhibitor.

**INTERNATIONAL SEARCH REPORT**

International application No.
**PCT/CN2020/103692**

**A. CLASSIFICATION OF SUBJECT MATTER**

C07D 471/18(2006.01)i; C07D 487/18(2006.01)i; A61K 31/439(2006.01)i; A61P 9/10(2006.01)i; A61P 7/02(2006.01)i

According to International Patent Classification (IPC) or to both national classification and IPC

**B. FIELDS SEARCHED**

Minimum documentation searched (classification system followed by classification symbols)

C07D, A61K, A61P

Documentation searched other than minimum documentation to the extent that such documents are included in the fields searched

Electronic data base consulted during the international search (name of data base and, where practicable, search terms used)

VEN; DWPI; SIPOABS; CNABS; CNTXT; STNext; Web of Science; CNKI; 百度学术, Baidu Xueshu: 大环, 环状氨基甲酸酯, 凝血因子XIa, 连接, 吡喃酮, 吡啶酮, 血栓, 栓塞, 明德新药, macrocycle, macrocyclic, cyclic carbamate, linker, pyrone, pyridone, blood coagulation factor XIa, plasma kallilrein, thrombembolic, MEDS-N, MEDSHINE DISCOVERY, 基于通式（I）的结构检索, structural search based on general formula (I)

**C. DOCUMENTS CONSIDERED TO BE RELEVANT**

| Category* | Citation of document, with indication, where appropriate, of the relevant passages | Relevant to claim No. |
|---|---|---|
| X | WO 2018133793 A1 (SUNSHINE LAKE PHARMA. CO., LTD.) 26 July 2018 (2018-07-26) claims 1, 2 and 19-32 | 1-27 |
| X | CN 108430471 A (MERCK SHARP & DOHME CORP.) 21 August 2018 (2018-08-21) claims 1-16 | 1-27 |
| X | CN 106795161 A (BRISTOL-MYERS SQUIBB COMPANY) 31 May 2017 (2017-05-31) claims 1-15 | 1-27 |
| X | CN 106459051 A (BRISTOL-MYERS SQUIBB COMPANY) 22 February 2017 (2017-02-22) claims 1-10 | 1-27 |
| X | CN 104507924 A (BRISTOL-MYERS SQUIBB COMPANY) 08 April 2015 (2015-04-08) claims 1-17 | 1-27 |
| A | CN 103702999 A (BRISTOL-MYERS SQUIBB COMPANY) 02 April 2014 (2014-04-02) claims 1-14 | 1-27 |
| A | CN 104520289 A (BRISTOL-MYERS SQUIBB COMPANY) 15 April 2015 (2015-04-15) claims 1-12 | 1-27 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 September 2020** | **29 October 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2020/103692**

| Patent document cited in search report | Publication date (day/month/year) | Patent family member(s) | Publication date (day/month/year) |
|---|---|---|---|
| WO 2018133793 A1 | 26 July 2018 | CN 110062757 A | 26 July 2019 |
| CN 108430471 A | 21 August 2018 | WO 2017074832 A1 | 04 May 2017 |
| | | RU 2018119015 A | 29 November 2019 |
| | | EP 3368036 A1 | 05 September 2018 |
| | | JP 2018531954 A | 01 November 2018 |
| | | MX 2018005045 A | 01 August 2018 |
| | | AU 2016344476 A1 | 08 March 2018 |
| | | EP 3368036 A4 | 17 April 2019 |
| | | BR 112018008506 A2 | 23 October 2018 |
| | | CA 2998902 A1 | 04 May 2017 |
| | | US 2018339977 A1 | 29 November 2018 |
| | | US 10214512 B2 | 26 February 2019 |
| | | RU 2018119015 A3 | 18 February 2020 |
| | | KR 20180073602 A | 02 July 2018 |
| CN 106795161 A | 31 May 2017 | AU 2020200376 A1 | 06 February 2020 |
| | | BR 112017006702 A2 | 26 December 2017 |
| | | HU E038061 T2 | 28 September 2018 |
| | | PE 20170939 A1 | 13 July 2017 |
| | | KR 20170057431 A | 24 May 2017 |
| | | NO 2721243 T3 | 20 October 2018 |
| | | AU 2015324530 A1 | 18 May 2017 |
| | | SG 10201911652T A | 27 February 2020 |
| | | IL 251434 D0 | 29 May 2017 |
| | | TW 201613923 A | 16 April 2016 |
| | | LT 3089979 T | 27 December 2017 |
| | | MX 2017003695 A | 30 May 2017 |
| | | PH 12017500580 B1 | 30 August 2017 |
| | | AU 2015324530 B2 | 24 October 2019 |
| | | DK 3089979 T3 | 15 January 2018 |
| | | EP 3089979 B1 | 18 October 2017 |
| | | PT 3089979 T | 16 January 2018 |
| | | JP 2017530157 A | 12 October 2017 |
| | | CN 110734435 A | 31 January 2020 |
| | | EA 201790595 A1 | 31 July 2017 |
| | | HR P20171950 T1 | 23 February 2018 |
| | | SG 11201702576Q A | 27 April 2017 |
| | | KR 20180126612 A | 27 November 2018 |
| | | JP 6462865 B2 | 30 January 2019 |
| | | CL 2017000712 A1 | 03 November 2017 |
| | | WO 2016053455 A1 | 07 April 2016 |
| | | CA 2963395 A1 | 07 April 2016 |
| | | PL 3089979 T3 | 28 February 2018 |
| | | TN 2017000112 A1 | 04 July 2018 |
| | | SI 3089979 T1 | 29 December 2017 |
| | | PH 12017500580 A1 | 30 August 2017 |
| | | AR 101367 A1 | 14 December 2016 |
| | | UY 36244 A | 29 January 2016 |
| | | EP 3089979 A1 | 09 November 2016 |
| | | CY 1119678 T1 | 04 April 2018 |
| | | RS 56786 B1 | 30 April 2018 |

Form PCT/ISA/210 (patent family annex) (January 2015)

**INTERNATIONAL SEARCH REPORT**
**Information on patent family members**

International application No.
**PCT/CN2020/103692**

| Patent document cited in search report | Publication date (day/month/year) | Patent family member(s) | Publication date (day/month/year) |
| --- | --- | --- | --- |
| | | MA 40123 A1 | 29 September 2017 |
| | | JP 2019069989 A | 09 May 2019 |
| | | ZA 201702478 B | 26 June 2019 |
| | | CN 106795161 B | 15 October 2019 |
| | | KR 101921436 B1 | 22 November 2018 |
| | | EA 031590 B1 | 31 January 2019 |
| | | ES 2655884 T3 | 22 February 2018 |
| | | EP 3293186 A1 | 14 March 2018 |
| CN 106459051 A | 22 February 2017 | US 2017057961 A1 | 02 March 2017 |
| | | PL 3099688 T3 | 30 March 2018 |
| | | CA 2937738 A1 | 06 August 2015 |
| | | PT 3099688 T | 15 January 2018 |
| | | TW 201615640 A | 01 May 2016 |
| | | EP 3099688 A1 | 07 December 2016 |
| | | US 2018222907 A1 | 09 August 2018 |
| | | US 2017342071 A1 | 30 November 2017 |
| | | AR 099228 A1 | 06 July 2016 |
| | | WO 2015116882 A1 | 06 August 2015 |
| | | CN 106459051 B | 16 October 2018 |
| | | NO 2760821 T3 | 10 March 2018 |
| | | EA 201691561 A1 | 30 January 2017 |
| | | EA 032650 B1 | 28 June 2019 |
| | | LT 3099688 T | 27 December 2017 |
| | | HR P20172002 T1 | 09 February 2018 |
| | | EP 3099688 B1 | 18 October 2017 |
| | | MX 2016009385 A | 16 September 2016 |
| | | SI 3099688 T1 | 29 December 2017 |
| | | US 10273236 B2 | 30 April 2019 |
| | | RS 56785 B1 | 30 April 2018 |
| | | CY 1120534 T1 | 10 July 2019 |
| | | DK 3099688 T3 | 15 January 2018 |
| | | ES 2655540 T3 | 20 February 2018 |
| | | JP 2017504640 A | 09 February 2017 |
| | | JP 6419836 B2 | 07 November 2018 |
| | | HU E038060 T2 | 28 September 2018 |
| | | UY 35972 A | 31 July 2015 |
| CN 104507924 A | 08 April 2015 | CA 2880866 A1 | 06 February 2014 |
| | | JP 2015528022 A | 24 September 2015 |
| | | US 2015203492 A1 | 23 July 2015 |
| | | ES 2623175 T3 | 10 July 2017 |
| | | PT 2880026 T | 03 May 2017 |
| | | BR 112015002081 A2 | 04 July 2017 |
| | | HR P20170506 T1 | 02 June 2017 |
| | | DK 2880026 T3 | 06 June 2017 |
| | | EP 2880026 A1 | 10 June 2015 |
| | | AR 091981 A1 | 11 March 2015 |
| | | SI 2880026 T1 | 26 April 2017 |
| | | TW 201410667 A | 16 March 2014 |
| | | IL 237011 D0 | 31 March 2015 |
| | | CN 108250199 A | 06 July 2018 |

Form PCT/ISA/210 (patent family annex) (January 2015)

**INTERNATIONAL SEARCH REPORT**
**Information on patent family members**

International application No.
**PCT/CN2020/103692**

| Patent document cited in search report | Publication date (day/month/year) | Patent family member(s) | Publication date (day/month/year) |
|---|---|---|---|
| | | JP 6082463 B2 | 15 February 2017 |
| | | US 9951071 B2 | 24 April 2018 |
| | | CN 104507924 B | 23 January 2018 |
| | | EA 028581 B1 | 29 December 2017 |
| | | EA 201590277 A1 | 29 May 2015 |
| | | KR 20150038372 A | 08 April 2015 |
| | | AU 2013296259 A1 | 19 March 2015 |
| | | US 2016340360 A1 | 24 November 2016 |
| | | MX 361759 B | 17 December 2018 |
| | | UY 34960 A | 31 January 2014 |
| | | MX 2015000892 A | 17 April 2015 |
| | | RS 55975 B1 | 29 September 2017 |
| | | CY 1119004 T1 | 10 January 2018 |
| | | HU E032622 T2 | 30 October 2017 |
| | | SG 11201500271U A | 30 March 2015 |
| | | LT 2880026 T | 25 April 2017 |
| | | EP 2880026 B1 | 22 February 2017 |
| | | SI EP2880026 T1 | 26 April 2017 |
| | | WO 2014022767 A1 | 06 February 2014 |
| | | PL 2880026 T3 | 31 August 2017 |
| | | US 9409908 B2 | 09 August 2016 |
| CN 103702999 A | 02 April 2014 | US 9108981 B2 | 18 August 2015 |
| | | CN 103702999 B | 25 November 2015 |
| | | EP 2739625 B1 | 20 January 2016 |
| | | ES 2563445 T3 | 15 March 2016 |
| | | EP 3070087 A1 | 21 September 2016 |
| | | US 2014163002 A1 | 12 June 2014 |
| | | WO 2013022814 A1 | 14 February 2013 |
| | | US 8901115 B2 | 02 December 2014 |
| | | JP 6113727 B2 | 12 April 2017 |
| | | AR 088423 A1 | 11 June 2014 |
| | | CN 105294701 A | 03 February 2016 |
| | | JP 2014521700 A | 28 August 2014 |
| | | US 2015057262 A1 | 26 February 2015 |
| | | CN 105294701 B | 24 October 2017 |
| | | EP 2739625 A1 | 11 June 2014 |
| | | ES 2733096 T3 | 27 November 2019 |
| | | EP 3070087 B1 | 10 April 2019 |
| | | TW 201319068 A | 16 May 2013 |
| CN 104520289 A | 15 April 2015 | LT 2882734 T | 12 December 2016 |
| | | EP 2882734 B1 | 12 October 2016 |
| | | DK 2882734 T3 | 30 January 2017 |
| | | WO 2014022766 A1 | 06 February 2014 |
| | | CA 2880898 A1 | 06 February 2014 |
| | | BR 112015002293 A2 | 04 July 2017 |
| | | SI 2882734 T1 | 31 January 2017 |
| | | MX 361370 B | 05 December 2018 |
| | | US 2015166550 A1 | 18 June 2015 |
| | | JP 6082462 B2 | 15 February 2017 |
| | | AR 093759 A1 | 24 June 2015 |

Form PCT/ISA/210 (patent family annex) (January 2015)

**INTERNATIONAL SEARCH REPORT**
**Information on patent family members**

International application No.
**PCT/CN2020/103692**

| Patent document cited in search report | Publication date (day/month/year) | Patent family member(s) | | | Publication date (day/month/year) |
|---|---|---|---|---|---|
| | | IL | 237012 | D0 | 31 March 2015 |
| | | HR | P20161378 | T1 | 02 December 2016 |
| | | PT | 2882734 | T | 09 December 2016 |
| | | US | 9376444 | B2 | 28 June 2016 |
| | | CY | 1118450 | T1 | 28 June 2017 |
| | | KR | 20150038369 | A | 08 April 2015 |
| | | EP | 2882734 | A1 | 17 June 2015 |
| | | US | 2014038969 | A1 | 06 February 2014 |
| | | AU | 2013296258 | A1 | 19 March 2015 |
| | | MX | 2015000919 | A | 21 September 2015 |
| | | HU | E031582 | T2 | 28 July 2017 |
| | | EA | 025392 | B1 | 30 December 2016 |
| | | SG | 11201500270R | A | 30 March 2015 |
| | | RS | 55581 | B1 | 30 June 2017 |
| | | SI | EP2882734 | T1 | 31 January 2017 |
| | | PL | 2882734 | T3 | 31 May 2017 |
| | | UY | 34959 | A | 31 January 2014 |
| | | EA | 201590284 | A1 | 29 May 2015 |
| | | JP | 2015524443 | A | 24 August 2015 |
| | | TW | 201410666 | A | 16 March 2014 |
| | | ES | 2605824 | T3 | 16 March 2017 |
| | | SM | T201700003 | B | 08 March 2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201910668575
- WO 2011100401 A
- WO 2011100402 A
- WO 2013022814 A
- WO 2013022818 A
- WO 2014022766 A
- WO 2014022767 A
- WO 2015116882 A
- WO 2015116885 A
- WO 2015116886 A
- WO 2016053455 A
- WO 2017074832 A
- WO 2017074833 A
- WO 2018133793 A